# EUROPEAN PATENT SPECIFICATION

(11) **EP 2 991 684 B1**
(45) Date of publication and mention of the grant of the patent: **11.07.2018**
(21) Application number: 14727380.9
(22) Date of filing: 27.04.2014
(51) Int. Cl.: A61K 47/61, A61P 7/04, A61P 37/06

(54) **COMPOSITIONS FOR INDUCING IMMUNE TOLERANCE TO COAGULATION FACTOR PROTEINS**
ZUSAMMENSETZUNGEN ZUR INDUKTION EINER IMMUNTOLERANZ GEGEN BLUTGERINNUNGSFAKTORPROTEINE
COMPOSITIONS POUR L'INDUCTION D'UNE IMMUNOTOLÉRANCE VIS-À-VIS DE PROTÉINES DE TYPE FACTEUR DE COAGULATION

(30) Priority: 28.04.2013 US 201361816790 P
(43) Date of publication of application: 09.03.2016
(73) Proprietor: Bayer HealthCare LLC, Whippany, NJ 07981 (US)
(72) Inventor: ASWAD, Fred, Jullien, Oakland, CA 94611 (US)
(74) Representative: BIP Patents
(86) International application number: PCT/US2014/035590
(87) International publication number: WO 2014/179184

(56) References cited:
- WO-A1-2011/014890
- US-A1- 2006 040 856
- US-A1- 2006 115 876
- B. H. DUONG ET AL: "Decoration of T-independent antigen with ligands for CD22 and Siglec-G can suppress immunity and induce B cell tolerance in vivo", JOURNAL OF EXPERIMENTAL MEDICINE, vol. 207, no. 1, 18 January 2010 (2010-01-18), pages 173-187, XP055032317, ISSN: 0022-1007, DOI: 10.1084/jem.20091873

## Description

### CROSS REFERENCE TO RELATED APPLICATIONS

This application claims the benefit of U.S. provisional application No. 61/816,790, filed April 28, 2013.

### INCORPORATION-BY-REFERENCE OF MATERIAL SUBMITTED ELECTRONICALLY

Incorporated by reference in its entirety herein is a computer-readable sequence listing submitted concurrently herewith and identified as follows: One (90,252 Byte ASCII (Text)) file named "Sequence_listing_ST25.txt," created on March 25, 2014.

### BACKGROUND

The development of coagulation factor replacement therapy has transformed the lives of many individuals with blood clotting disorders, such as hemophilia. Hemophilia is a group of hereditary genetic disorders that impair the body's ability to control blood clotting or coagulation. Patients with hemophilia do not produce adequate amounts of Factor VIII (FVIII) or Factor IX (FIX) proteins, which are necessary for effective blood clotting. In severe hemophiliacs even a minor injury can result in blood loss that continues for days or weeks, and complete healing may not occur, leading to the potential for debilitating permanent damage to joints and other organs, and premature death. Hemophilia A is the most common hereditary coagulation disorder, with an estimated incidence of 1 per 5000 males. It is caused by deficiency or structural defects in FVIII, a critical component of the intrinsic pathway of blood coagulation. The current treatment for hemophilia A involves intravenous injection of human FVIII. Human FVIII has been produced recombinantly as a single-chain molecule of approximately 300 kD. It consists of the structural domains A1-A2-B-A3-C1-C2 (Thompson, Semin. Hematol. 29:11-22 (2003)). The precursor product is processed into two polypeptide chains of 200 kD (heavy) and 80 kD (light) in the Golgi Apparatus, with the two chains held together by metal ions (Kaufman et al., J. Biol. Chem. 263:6352 (1988); Andersson et al., Proc. Natl. Acad. Sci. 83: 2979 (1986)). The B-domain of FVIII seems to be dispensable as B-domain deleted FVIII (BDD, 90 kD A1-A2 heavy chain plus 80 kD light chain) has also been shown to be effective as a replacement therapy for hemophilia A. The B-domain deleted FVIII sequence contains a deletion of all but 14 amino acids of the B-domain.

Hemophilia A patients are currently treated by intravenous administration of FVIII on demand or as a prophylactic therapy administered several times a week. For prophylactic treatment 15-25 IU/kg bodyweight is given of FVIII three times a week. It is constantly required in the patient. Because of its short half-life in man, FVIII must be administered frequently. Despite its large size of greater than 300 kD for the full-length protein, FVIII has a half-life in humans of only about 11 hours. (Ewenstein et al., Semin. Hematol. 41:1-16 (2004)).

A serious limitation of therapy is the possibility that the patient's immune system will develop antibodies to the exogenously administered FVIII (Saenko et al., Haemophilia 8:1-11 (2002)). The major epitopes of inhibitory antibodies are located within the A2 domain at residues 484-508, the A3 domain at residues 1811-1818, and the C2 domain. Unfortunately, antibody development prevents the use of FVIII as a replacement therapy in many patients.

Thus, in order for replacement therapy to be effective, it is crucial to prevent any undesired immune responses. There are many shortcomings in methodologies for preventing or eliminating undesired immune responses, particularly against biotherapeutics. Current treatment of undesirable immune responses often involves broad immunosuppresion, such as chemical inhibitors or B cell depletion therapy (REFS), which may increase susceptibility to infection. US2006/040856 teaches factor IX - PEG -sialic acid conjugates (claim 1, examples 2-8). Sialic acid is a B cell Siglec ligand. The coagulation factor IX includes a mutation that adds one or more N or O glycosylation sites to the peptide sequence (par. 259). The polymer is bound to the peptide at the glycosylated site (par. 235). Thus the polymer is covalently attached to the mutated site. The conjugate is used for stimulating blood coagulation (claim 39, 40). Accordingly, there remains a need in the art for compositions and methods which can prevent antibody responses and induce tolerance to coagulation factor biotherapeutics in patients.

### SUMMARY

The present embodiments provide compositions and methods for preventing or reducing undesired antibody immune responses and inducing immune tolerance of blood coagulation factors, such as FVIII. as defined in the claims. The invention is defined by the claims. Any subject-matter falling outside the scope of the claims is provided for information purposes only. In one aspect, there is provided a conjugate for inducing tolerance of a coagulation factor protein, wherein the conjugate comprises a coagulation factor protein or an antigenic fragment or variant thereof and a Siglec ligand. as defined in the claims. In some embodiments, the Siglec ligand is a ligand for an inhibitory Siglec. In some embodiments, the Siglec ligand binds to a Siglec selected from Siglec-1 (CD169), Siglec-2 (CD22), Siglec-3 (CD33), Siglec-4 (MAG), Siglec-5, Siglec-6, Siglec-7, Siglec-8, Siglec-9, Siglec-G/10, Siglec-11, and Siglec-12. In some embodiments, the Siglec is expressed on the surface of a B lymphocyte. In some embodiments, the Siglec ligand is a B cell Siglec-2 (CD22) ligand. In some embodiments, the Siglec ligand is a Siglec-G/10 ligand. In some embodiments, the coagulation factor protein is conjugated to the ligand, such as a Siglec-2 ligand, directly or indirectly, in a covalent or non-covalent manner.

In another aspect, there is provided pharmaceutical compositions comprising effective amounts of the conjugate for inducing tolerance in a subject.

In another aspect, there is provided a method of inducing tolerance to a coagulation factor protein in a subject, comprising administering to the subject an effective amount of a conjugate comprising a coagulation factor protein or an antigenic fragment or variant thereof and a Siglec ligand.

In some embodiments, the conjugate further comprises a small particle, such as a liposome, and the coagulation factor protein or an antigenic fragment or variant thereof and a Siglec ligand are displayed on the surface of the liposome. In some embodiments, the coagulation factor protein or an antigenic fragment or variant thereof and the Siglec ligand are linked via the small particle. The Siglec ligand is a glycan selected from the group consisting of 9-N-biphenylcarboxyl-NeuAca2-6 Gal∼1-4GlcNAc (6'-BPCNeuAc), NeuAca2-6Gal∼1-4GlcNAc and NeuAca2-6Gal∼1-4(6-sulfo)GlcNAc and combinations thereof.

In some embodiments, the coagulation factor protein is selected from the group consisting of Factor VII, Factor VIII, Factor IX, Factor X, and Factor XI and combinations thereof.

It is to be understood that both the foregoing general description of the embodiments and the following detailed description are exemplary.

### BRIEF DESCRIPTION OF THE DRAWINGS

The skilled artisan will understand that the drawings, described below, are for illustration purposes only.

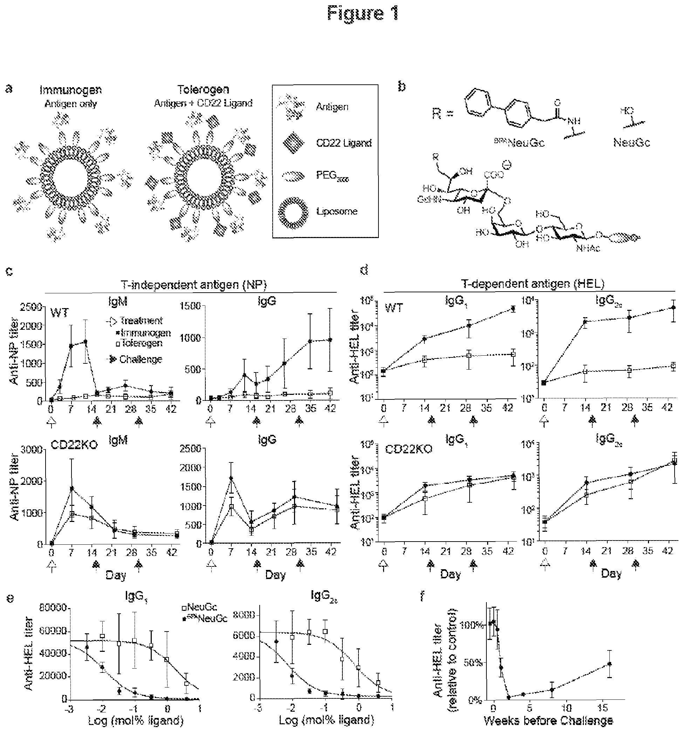

FIG. 1. Induction of tolerance with liposomes displaying antigen and CD22 ligands. a, Schematic of immunogenic and tolerogenic liposomes. b, Chemical structures of CD22 ligands used in this study. c and d, CD22-dependent induction of tolerance to a T-independent (NP; panel c) and a T-dependent antigen (HEL; panel d). WT or CD22KO mice were treated on day 0 (open arrow) as shown and challenged with the immunogenic liposomes on days 15 and 30 (closed arrow). Data represents mean +/- s.e.m. (n=8-10). e, Titration of ^{BPA}NeuGc and NeuGc on toleragenic liposomes. Titers were determined after two challenges with immunogenic liposomes (n=4). f, Mice were tolerized to HEL at different times relative to the challenge and titers were determined two weeks after challenge with immunogenic liposomes and are relative to immunization of naive mice (n=4). Data represents mean +/- s.e.m. (n=4).

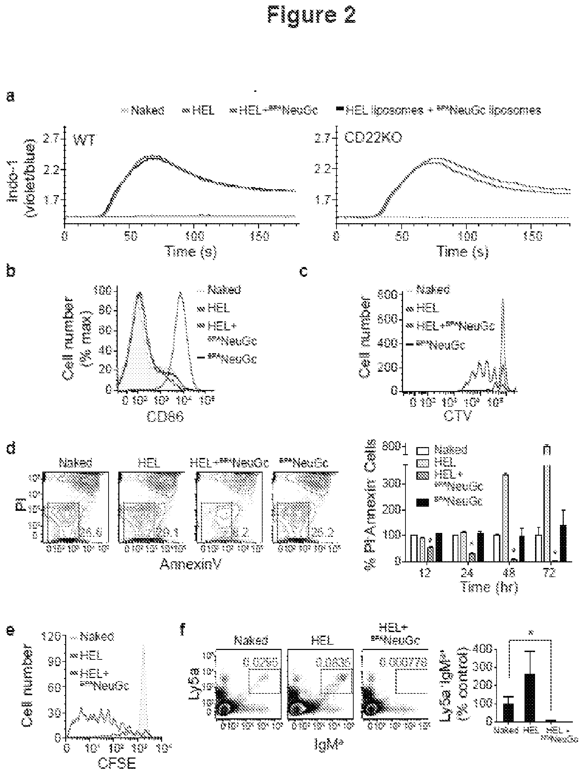

FIG. 2. Toleragenic liposomes strongly inhibit BCR signaling and cause apoptosis. a, Calcium flux in IgM^{HEL} B cells stimulated with the indicated liposomes. b, CD86 upregulation of IgM^{HEL} B cells 24 hr after stimulation with the indicated liposomes. b, In vitro proliferation of CTV-labeled IgM^{HEL} B cells three days after simulation with the indicated liposomes. d, AnnexinV versus PI staining of IgM^{HEL} B cells treated for 24 hr with the indicated liposomes. Data represents mean +/- s.e.m. (n=3). e, In vivo proliferation of adoptively-transferred CFSE-labeled IgM^{HEL} B cells four days after immunization with the indicated liposomes. f, Analysis of the number of adoptively-transferred Ly5a⁺IgM^{HEL} B cells remaining in the spleen of host mice 12 days after immunization with the indicated liposomes. Quantitation represents mean +/- s.e.m (n=4).

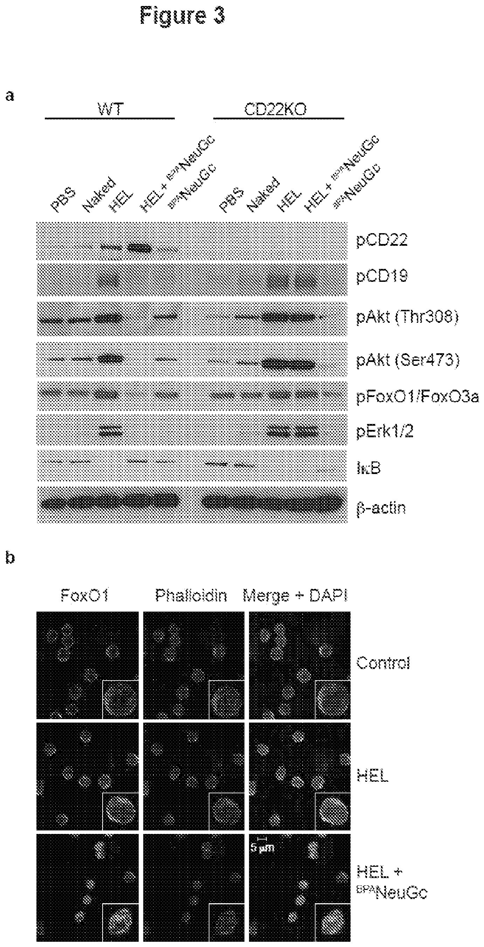

FIG. 3. A CD22-dependent tolerogenic circuit inhibits the Akt survival pathway and drive nuclear import of FoxO1. a, Western blot analysis of BCR signaling components in WT and CD22KO IgM^{HEL} B cells 30 minutes after stimulation of cells with the indicated liposomes or PBS as a control. Tolerogenic liposomes inhibit phosphorylation of signaling components of all major BCR signaling pathways, and induce hypophosphorylation of Akt and FoxO1 in WT B cells, but not CD22 deficient IgM^{HEL} B cells. b, Confocal microscopy of IgM^{HEL} B cells stimulated for 2hr with the indicated liposomes. Cells were stained with anti-FoxO1, phalloidin, and DAPI. Inserts are a representative cell at three-times the magnification.

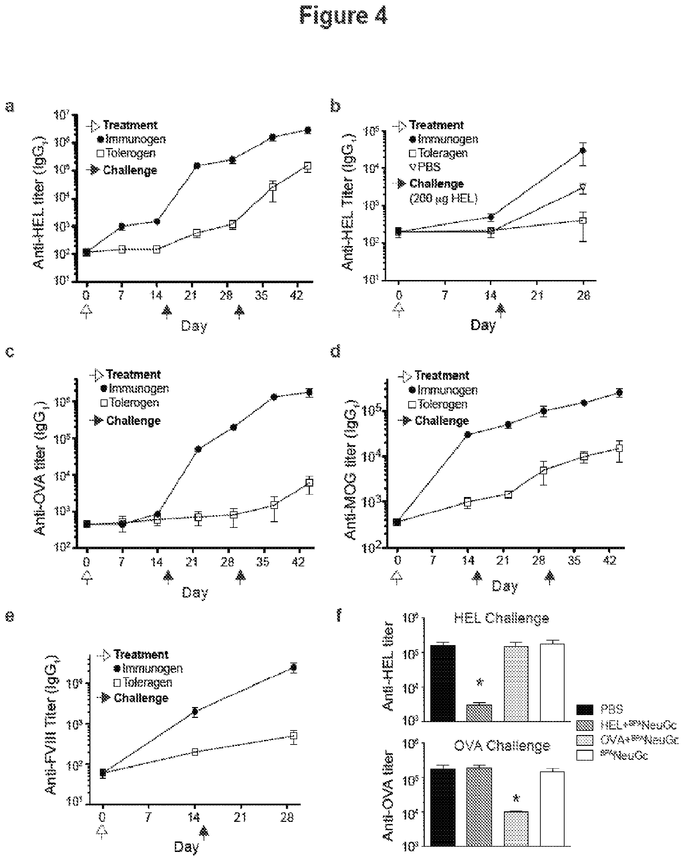

FIG. 4. Antigen-specific tolerization of mice to strong T-dependent antigens, a-b, Tolerization of HEL in Balb/c mice to a liposomal (panel a) or soluble (panel b) challenge, c, tolerization of OVA in C57BL/6J mice. d, Tolerization of MOG in Balb/c mice. e, Tolerization of FVIII in Balb/c. f, Tolerization is antigen-specific. Balb/c mice tolerized to HEL or OVA have normal responses to other antigen. Mice were immunized on day 0 with the indicated conditions, challenged on day 15 with immunogenic liposomes, and titers determined two weeks later on day 29. All data represents mean +/- s.e.m. (n=4).

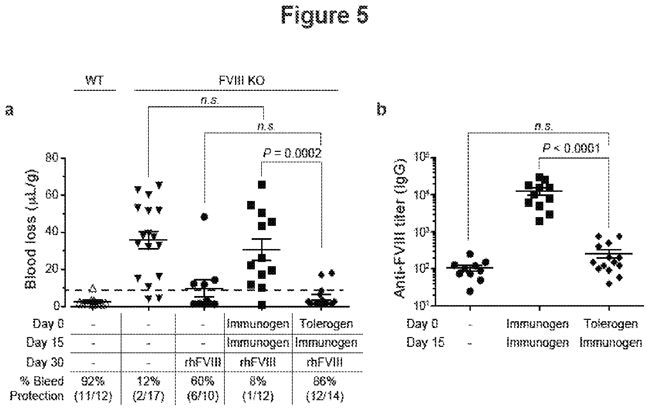

FIG. 5. Immune tolerization to FVIII prevents bleeding in FVIII-deficient mice. a, WT or FVIII-deficient mice were dosed as described on day 0 and 15. On day 30, mice were reconsituted with recombinant human FVIII (rhFVIII) at 50 U/kg or saline. FVIII-deficient mice treated with tolerogenic liposomes had significantly less blood loss over 20 minutes following a tail clip than mice initially treated with immunogenic liposomes. Percent bleeding protection (dashed line) represents blood loss < 9.9 µl/g as defined by mean plus 3 SDs in WT Balb/c mice. b, FVIII-titers in the three reconstituted groups demonstrates that bleeding prevention is accompanied by a significant reduction in anti-FVIII antibodies. Data represents mean +/- s.e.m. A two-tailed Student's t-test was used to establish the level of significance; no statistical difference (n.s.) is defined by a P value greater than 0.05.

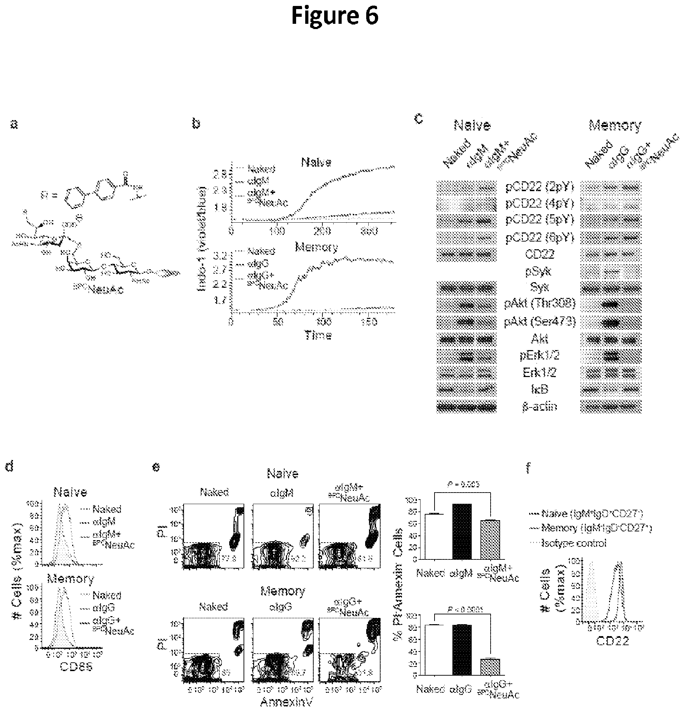

FIG. 6. A CD22-mediated tolerogenic circuit is operative in both naive and memory human B cells. a, Staining of naive (CD19⁺IgM⁺IgD⁺CD27⁻; red) and memory (CD19⁺IgM⁻IgD⁻CD27⁻; blue) human B cells with anti-CD22 or isotype control (grey) antibodies. b, Structure of the high affinity human CD22 ligand ^{BPC}NeuAc. c-e, Activation of naive and memory human B cells is inhibited by co-presentation of ^{BPC}NeuAc with cognate antigen (anti-IgM or anti-IgG, respectively) on liposomes, as judged by calcium flux (panel c), Western blot analysis of BCR signaling components (panel d), and CD86 upregulation (panel e). f, Liposomes displaying cognate antigen and CD22 ligands decrease viability of both naive and memory human B cells. Data represents mean +/- s.e.m (n=3). A two-tailed Student's t-test was used to establish the level of significance.

### DESCRIPTION OF VARIOUS EMBODIMENTS

Successful treatments utilizing biotherapeutics, particularly polypeptide drugs, require that the subject's immune system does not interfere or inhibit the activity of the biotherapeutic drug. Anti-drug antibodies (ADA) are recognized as a serious issue with biotherapeutics and can remain a problem even after steps have been taken to minimize immunogenicity of the drugs themselves. This problem can be particularly threatening for biotherapeutic coagulation factors provided to patients with blood clotting disorders, where the biotherapeutic is critical to stop blood loss following an injury. Described herein are compositions and methods for inducing antigen-specific tolerance to coagulation factor biotherapeutics. The compositions comprise one or more coagulation factor proteins or antigenic fragments or variants thereof conjugated to one or more Siglec ligands. Without being bound by theory as to how the embodiments work, a tolerogenic circuit is induced in B cells when the Siglec and the B cell receptor are juxtaposed in an immunological synapse with the conjugate comprising the coagulation factor protein and the Siglec ligand.

It is shown herein that tolerance to coagulation Factor VIII (FVIII) was induced in a hemophilia mouse model, preventing formation of inhibitory antibodies, allowing administration of FVIII to prevent bleeding on subsequent challenge. It is also shown herein that enforced ligation of the B cell receptor and CD22 shuts down B cell receptor signaling and induces apoptosis in both mouse and human B cells. It is also shown that the tolerogenic circuit is operative in human primary B cells within both the naive and memory compartments, indicating that the approach of engaging CD22 and the B cell receptor to induce antigen-specific tolerance to polypeptide T-dependent antigens is applicable to not only preventing but also eliminating pre-existing conditions in humans.

For the purpose of interpreting this specification, the following definitions will apply and whenever appropriate, terms used in the singular will also include the plural and vice versa. In the event that any definition set forth below conflicts with the usage of that word in any other document, including any document incorporated herein by reference, the definition set forth below shall always control for purposes of interpreting this specification and its associated claims unless a contrary meaning is clearly intended (for example in the document where the term is originally used). The use of "or" means "and/or" unless stated otherwise. The use of "a" herein means "one or more" unless stated otherwise or where the use of "one or more" is clearly inappropriate. The use of "comprise," "comprises," "comprising," "include," "includes," and "including" are interchangeable and not intended to be limiting. Furthermore, where the description of one or more embodiments uses the term "comprising," those skilled in the art would understand that, in some specific instances, the embodiment or embodiments can be alternatively described using the language "consisting essentially of' and/or "consisting of."

Unless defined otherwise, all technical and scientific terms used herein have the same meaning as commonly understood by those of ordinary skill in the art to which these embodients pertain. The following references provide one of skill with a general definition of many of the terms used: Academic Press Dictionary of Science and Technology, Morris (Ed.), Academic Press (1st ed., 1992); Oxford Dictionary of Biochemistry and Molecular Biology, Smith et al. (Eds.), Oxford University Press (revised ed., 2000); Encyclopaedic Dictionary of Chemistry, Kumar (Ed.), Anmol Publications Pvt. Ltd. (2002); Dictionary of Microbiology and Molecular Biology, Singleton et al. (Eds.), John Wiley & Sons (3rd ed., 2002); Dictionary of Chemistry, Hunt (Ed.), Routledge (1st ed., 1999); Dictionary of Pharmaceutical Medicine, Nahler (Ed.), Springer-Verlag Telos (1994); Dictionary of Organic Chemistry, Kumar and Anandand (Eds.), Anmol Publications Pvt. Ltd. (2002); and A Dictionary of Biology (Oxford Paperback Reference), Martin and Hine (Eds.), Oxford University Press (4th ed., 2000). Further clarifications of some of these terms as they apply specifically to the embodiments are provided herein.

The present embodiments provide compositions and methods for preventing or reducing undesired antibody immune responses and inducing immune tolerance of blood coagulation factor proteins, such as FVIII.

In some embodiments, provided is a conjugate for inducing tolerance of a coagulation factor, wherein the conjugate comprises a coagulation factor protein or an antigenic fragment or variant thereof and a Siglec ligand. In some embodiments, provided are pharmaceutical compositions comprising effective amounts of the conjugate for inducing tolerance in a subject. In some embodiments, the subject has a blood clotting disorder and is administered coagulation factor replacement therapy.

In some embodiments, further provided are methods of inducing tolerance to a coagulation factor protein in a subject, comprising administering to the subject an effective amount of a conjugate comprising a coagulation factor protein or an antigenic fragment or variant thereof and a Siglec ligand.

In some embodiments, the subject has a bleeding disorder. In some embodiments, the subject is undergoing coagulation factor replacement therapy. In some embodiments, the bleeding disorder is selected from the group consisting of hemophilia A, hemophilia B, Factor X deficiency, and Rosenthal syndrome (also known as hemophilia C).

In some embodiments, the distance separating the coagulation factor moiety and the Siglec ligand moiety of the conjugate enables efficient presentation to a B cell resulting in enforced ligation and juxtaposition of the Siglec and B cell receptor in an immunological synapse.

As used herein, immune tolerance (or simply "tolerance") is the process by which the immune system does not attack an antigen. It occurs in three forms: central tolerance, peripheral tolerance and acquired tolerance. Tolerance can be either "natural" or "self tolerance," where the body does not mount an immune response to self antigens, or "induced tolerance", where tolerance to antigens can be created by manipulating the immune system. When tolerance is induced, the body cannot produce an immune response to the antigen. Mechanisms of tolerance and tolerance induction are complex and poorly understood. As is well known in the art (*see, e.g.,* Basten et al., 30 Curr. Opinion Immunol. 22:566-574, 2010), known variables in the generation of tolerance include the differentiation stage of the B cell when antigen is presented, the type of antigen, and the involvement of T cells and other leukocytes in production of cytokines and co factors. Thus, suppression of B cell activation cannot be equated with immune tolerance. For example, while B cell activation can be inhibited by crosslinking CD22 to the BCR, the selective silencing of B cells does not indicate induction of tolerance. *See, e.g.,* Nikolova et al., Autoimmunity Rev. 9:775-779 (2010); Mihaylova et al., Mol. Immunol. 47:123-130 (2009); and Courtney et al., Proc. Natl. Acad. Sci. 106:2500-2505 (2009).

### Conjugates

The term "conjugate" as used herein refers to a complex in which one or more Siglec ligands is coupled to one or more coagulation factor proteins or an antigenic fragment or variant thereof. The coagulation factor protein and the Siglec ligand may be coupled either directly or indirectly, by covalent or non-covalent interactions. In some embodiments, the Siglec ligand is coupled directly to the coagulation factor via an appropriate linking chemistry.
Conjugation of the Siglec ligand and coagulation factor protein can be performed in accordance with methods well known in the art. *See, e.g.,* Chemistry of protein conjugation and cross-linking, Shan Wong, CRC Press (Boca Raton, FL, 1991); and Bioconjugate techniques, 2nd ed., Greg T. Hermanson, Academic Press (London, UK, 2008).In some embodiments, the Siglec ligand is conjugated directly to the coagulation factor protein or antigenic fragment or variant thereof. In some embodiments, the coagulation factor protein or antigenic fragment or variant thereof is conjugated to a Siglec ligand directly, by conjugation to one or more pre-existing carbohydrates on the coagulation factor protein or antigenic fragment or variant.
In some embodiments, one or more sialic acid residues are removed from the coagulation factor protein or antigenic fragment or variant thereof before the Siglec ligand is conjugated. In some embodiments, the Siglec ligand can be conjugated to the coagulation factor polypeptide or antigenic fragment or variant thereof in equal molar ratios. In some embodiments, the ratio of Siglec ligand to coagulation factor protein or antigenic fragment or variant thereof is 1:1, 2:1, 5:1, 10:1, 15:1, 25:1, 35:1, 50:1, 75:1, 100:1. 200:1, 250:1, 500:1 or 1000:1. In one embodiment, the ratio of Siglec ligand to coagulation factor protein or antigenic fragment or variant thereof is from 50:1 to 100:1.

In some embodiments, the Siglec ligand is conjugated directly to any available or engineered cysteines on any domain of the coagulation factor protein or antigenic fragment or variant, e.g., FVIII.

In some embodiments, the coagulation factor protein or antigenic fragment or variant thereof and Siglec ligand are linked by a physiologically acceptable linker molecule. A physiologically acceptable linker molecule can include, *e.g*., polymers which are soluble in an aqueous solution or suspension and have no negative impact, such as side effects, to mammals upon administration of the Siglec ligand-coagulation factor protein conjugate in a pharmaceutically effective amount. There is no particular limitation to the physiologically acceptable linker used according to the present embodiments. In some embodiments, the linkers are typically characterized as having from 1 to about 500 repeating units. Examples of such polymers include, but are not limited to, poly(alkylene glycols) such as polyethylene glycol (PEG), poly(propylene glycol) (PPG), copolymers of ethylene glycol and propylene glycol and the like, poly(oxyethylated polyol), poly(olefinic alcohol), poly(vinylpyrrolidone), poly(hydroxyalkylmethacrylamide), poly(hydroxyalkylmethacrylate), poly(saccharides), poly(a-hydroxy acid), poly(vinyl alcohol), polyphosphazene, polyoxazoline, poly(N-acryloylmorpholine), and combinations of any of the foregoing.

The physiologically acceptable linker is not limited to a particular structure and can be linear (*e.g*. alkoxy PEG or bifunctional PEG), branched or multi-armed (e.g. forked PEG or PEG attached to a polyol core), dendritic, or with degradable linkages. Moreover, the internal structure of the linker can be organized in any number of different patterns and can be selected from the group consisting of homopolymer, alternating copolymer, random copolymer, block copolymer, alternating tripolymer, random tripolymer, and block tripolymer. These linkers can also include poly(alkylene oxide) polymers, poly(maleic acid), poly(DL-alanine), such as carboxymethylcellulose, dextran, hyaluronic acid and chitin, and poly(meth)acrylates.

In some embodiments, the Siglec ligand is conjugated to a physiologically acceptable linker, e.g., PEG and/or branched PEG, and the physiologically acceptable linker is itself conjugated directly to the coagulation factor protein or antigenic fragment or variant, e.g., FVIII. In some embodiments, the physiologically acceptable linker can be conjugated to the coagulation factor protein or antigenic fragment or variant directly to one or more pre-existing carbohydrates on any domain. In some embodiments, the physiologically acceptable linker can be conjugated to the coagulation factor protein or antigenic fragment or variant directly to any available or engineered cysteines on any domain. In some embodiments, the physiologically acceptable linker can be conjugated to the coagulation factor protein or antigenic fragment or variant directly to any amino acid on any domain.

In one embodiment, the physiologically acceptable linker is PEG and derivatives thereof. The PEG side chain can be linear, branched, forked or can consist of multiple arms. There is no specific limitation of the PEG used according to the present embodiments. In some embodiments, the PEG has a molecular weight in the range of 1,000-20,000. In some embodiments, useful PEG molecules are disclosed in WO 03/040211; U.S. Pat. No. 6,566,506; U.S. Pat. No. 6,864,350; and U.S. Pat. No. 6,455,639, for example. In another embodiment, the physiologically acceptable linker is polysialic acid (PSA) and/or derivatives thereof. PSA can be bound to the coagulation factor protein using known methods and techniques (*see, e.g.,* U.S. Pat. No. 4,356,170,). In one embodiment, the physiologically acceptable linker is a naturally occurring polysaccharide, a derivative of a naturally occurring polysaccharide, or a naturally occurring polysaccharide derivative. In some embodiments, the polysaccharide portion of the compound has more than 5, typically at least 10, and in another embodiment at least 20 to 50 sialic acid residues in the polymer chain. In some embodiments, the polysaccharide compounds may have up to 500 saccharide residues in total. In some embodiments, all of the saccharide residues in the compound are sialic acid residues. The saccharide unit may contain other functional groups, such as, amine, hydroxyl or sulphate groups, or combinations thereof. These groups may be present on naturally occurring saccharide compounds, or introduced into derivative polysaccharide compounds.

The coagulation factor protein or antigenic fragment or variant thereof can be covalently linked to the polysaccharide compounds by any of various techniques known to those of skill in the art. Examples include linkage through the peptide bond between a carboxyl group on one of either the coagulation factor protein or polysaccharide and an amine group of the other, or an ester linkage between a carboxyl group of one and a hydroxyl group of the other. Alternatively a Schiff base can be formed between an amino group of one and an aldehyde group of the other. Other mechanisms of linkage are within the ordinary skill of the art. Various examples are identified in U.S. Pat. No. 5,846,951,

As used herein, reference to coagulation factor protein or antigenic fragment or variant thereof being bound to one or more physiologically acceptable linker molecules includes any suitable chemical binding, such as, covalently bound or non-covalently bound such as ionic, hydrophobic, affinity, bioaffinity interactions. The linker can also be coupled to the protein by use of bifunctional reagents and via a spacer arm. In addition the linker molecule can be coupled to the coagulation factor protein by affinity interaction. For example, the coagulation factor protein can be biotinylated and avidin or strepavidin conjugated polymers can be bound to the coagulation factor protein.

Linkers can be bound to the coagulation factor protein or antigenic fragment or variant thereof also by enzymatical methods such as, for example, the transfer of saccharides with polyglycosyltransferase as taught in U.S. Pat. No. 6,379,933 or glycopegylation as taught in US Patent Application Pub. No. 20040132640 A1.

According to one embodiment, the physiologically acceptable linker is PEG or a PEG derivative, which is covalently linked to the coagulation factor protein by any strategy and method known in the art. In some embodiments, the modification strategies are the binding of at least one linker molecule via amino groups of lysine residues, the binding of at least one linker molecule via carbohydrate side chains, the binding of at least one linker molecule via sulfhydryl groups, the binding of at least one linker molecule via carboxyl groups of aspartic acids and glutamic acids as well as the binding of at least one linker molecule of hydroxyl groups and the binding of at least one linker molecule of the N-terminus.

In another embodiment, the coagulation factor protein or antigenic fragment or variant thereof can also bound to at least one linker molecule via its carbohydrate residues. In some embodiments, this can be carried out by e.g. mild oxidation of the carbohydrate chains, such as with NaIO₄, forming an aldehyde function and subsequent coupling to a PEG, such as PEG-hydrazide.

Another embodiment provides the binding of at least one linker molecule to the coagulation factor protein or antigenic fragment or variant thereof via sulfhydryl groups. The free SH-groups can be modified, for example, by PEG maleimide forming a stable sulfide. PEGylation of cysteine residues may also be carried out using, for instance, PEG-vinylsulfone, PEG-iodoacetamide, or PEG-orthopyridyl disulfide.

In some embodiments, the conjugation of a cysteine (including cysteine mutants) of the coagulation factor protein or antigenic fragment or variant thereof to a physiologically acceptable linker, e.g., PEG, or a Siglec ligand can be carried out as follows. For example, a FVIII molecule can have a cysteine introduced at specific locations (e.g., at residue 1804), and this FVIII is reduced with TCEP by adding 120 ul of TCEP stock solution (25 mM) which is freshly prepared in 20 mM MOPS/10 mM CaCl₂/100 ppm Tween 80, pH 7.0 into 12 mL factor VIII (0.15 mg/mL) to give a final concentration of 0.25 mM. The sample is incubated for 1 h at RT without mixing, and TCEP is removed using cation-exchange chromatography. Before conjugation, the FVIII sample is incubated at 4 °C overnight to allow reformation of protein disulfide bonds that may have been reduced by TCEP. A maleimide activated form of the ligand is mixed with the FVIII and incubated 4 °C on a rocker for 5 h (mixing slowly). The conjugated FVIII is purified from unreacted ligand. For example, using cation-exchange chromatography where the conjugate can be eluted with a 30 min gradient to 40% Buffer E (20 mM MOPS/10 mM CaCl₂/100 ppm Tween80, pH 7.0) over 60% Buffer F (Buffer E plus 600 mM NaCl) at a flow rate of 0.5 mL/min. Sucrose crystals are dissolved in the elution pool to give a final concentration of 1%, and the protein can be stored at -80 °C.

In some embodiments, enzymatic glyco-conjugation of a linker (such as PEG) or Siglec ligand to the coagulation factor protein or antigenic fragment or variant thereof (such as FVIII) can be carried out as follows. Enzymatic conjugation of a sialic-acid-ligand molecule to native N-glycans on a glycoprotein such as FVIII can be carried out in a three-step process. First, the glycoprotein is desialylated by incubation with sialidase in 10 mM His, 50 mM NaCl, 3 mM CaCl₂, pH 6.0 buffer. Then, CMP-sialic acid-Gly-ligand, at a sutable ratio for reaction (e.g., 1-20 fold molar excess), is added together with ST3GalIII to catalyze the transfer of sialic-acid-ligand. Following incubation at room temperature for 18-24 hrs remaining galactoses are capped with sialic acid by addition of a molar excess of CMP-sialic acid. The glyco-conjugate can be subsequently purified from unreacted reactants or fractionated according to the level of conjugation (e.g., by anion exchange chromatography or affinity chromatography or size exclusion chromatography). Fractions containing suitably active conjugates can be pooled, buffer exchanged into 20 mM MOPS/10 mM CaCl₂/100 ppm Tween80, 1% sucrose, pH 7.0, and stored at -80 °C.

In some embodiments, the conjugate comprises a small particle, such as a metal-based nanoparticle, polymeric nanoparticle, lipid-based nanoparticle, liposome or solid lipid nanoparticle. In some embodiments, the small particle serves to couple the Siglec ligand and the coagulation factor protein indirectly, and facilitates their juxtaposition and binding of Siglec and the B cell receptor in an immunological synapse on B lymphocyte cells. In some embodiments, the Siglec ligand present on the small particle is a glycan ligand that specifically recognizes a Siglec expressed on the surface of B cells. In some embodiments, the Siglec expressed on the surface of B cells is CD22 and/or Siglec G/10. The conjugation to a small particle, such as a liposome, can be either direct or indirect, and can be covalent or non-covalent in nature. In some embodiments, the Siglec ligand and coagulation factor protein are conjugated to the small particle so that they are displayed on the outer surface of the small particle. In some embodiments, the Siglec ligand and coagulation factor protein are attached to the same molecule of a liposome. In another embodiment, the Siglec ligand and coagulation factor protein are attached to different molecules on a liposome.

In some embodiments, the small particle has an average particle size of between 1 to 600 nm. In some embodiments, the small particle has an average particle size of between 1 to 500 nm, between 1 and 400 nm, between 1 and 300 nm, between 1 and 200 nm, between 1 and 150 nm or between 10 to 100 nm. In some embodiments, about 90% of the small particles have a particle size that falls within the above mentioned ranges. In some embodiments, about 50%, about 60%, about 70%, about 80%, about 85%, about 90%, about 95% or about 99% of the small particles have a particle size that falls within the above mentioned ranges. As used herein, "about" means ± 10%.

In some embodiments, the liposome is typically a vesicular structure of a water soluble particle obtained by aggregating amphipathic molecules including a hydrophilic region and a hydrophobic region. While the liposome component is a closed micelle formed by any amphipathic molecules, in some embodiments it includes lipids and forms a bilayer structure. In some embodiments, the liposomal composition is a semi-solid, ultra fine vesicle sized between about 10 and about 200 nanometers. The structure of the liposome is not particularly limited, and may be any liposome such as unilamella and multilamella. As a solution encapsulated inside the liposome, it is possible to use buffer and saline and others in addition to water.

In some embodiments, the liposomes comprise phospholipids such as distearoyl phosphatidylcholine (DSPC) and polyethyleneglycol-distearoyl phosphoethanolamine (PEG-DSPE). Other phospholipids can also be used in preparing the liposomes of the embodiments, including dipalmitoylphosphatidylcholine (DPPC), dioleylphosphatidylcholine (DOPC) and dioleylphosphatidyl ethanolamine (DOPE), sphingoglycolipid and glyceroglycolipid. These phospholipids can be used in making the liposome, alone or in combination of two or more or in combination with a lipid derivative where a non-polar substance such as cholesterol or a water soluble polymer such as polyethylene glycol has been bound to the lipid.

The liposomes can be prepared in accordance with methods well known in the art. For example, incorporation of a Siglec ligand and a coagulation factor on the surface of a liposome can be achieved by any of the routinely practiced procedures. Detailed procedures for producing a liposome nanoparticle bearing a Siglec ligand and a coagulant factor protein are also exemplified in the Examples herein. In some embodiments, the conjugate comprises a liposome and an incorporated glycan ligand (e.g., ^{BPA}NeuGc) and a specific coagulant factor protein such as Factor VIII. In addition to the methods and procedures exemplified herein, various methods routinely used by the skilled artisans for preparing liposomes can also be employed in the present embodiments. For example, the methods described in Chen et al., Blood 115:4778-86, 2010; and Liposome Technology, vol. 1, 2nd edition (by Gregory Gregoriadis (CRC Press, Boca Raton, Ann Arbor, London, Tokyo), Chapter 4, pp 67-80, Chapter 10, pp 167-184 and Chapter 17, pp 261-276 (1993)) can be used. More specifically, suitable methods include, but are not limited to, a sonication method, an ethanol injection method, a French press method, an ether injection method, a cholic acid method, a calcium fusion method, a lyophilization method and a reverse phase evaporation method.

### Coagulation factors proteins

As used herein, "coagulation factor protein" refers to a protein that is involved in the coagulation cascade and has predominantly procoagulant activity. Coagulation factors are well known in the art and include without limitation coagulation factors I, II, V, VI, VII, VIII, IX, X, XI, XII, and XIII. In some embodiments, the coagulation factors can be concentrated from plasma or can be recombinantly produced. In some embodiments, the coagulation factors have an amino acid structure that varies from the natural structure. In some embodiments, the coagulation factor has sufficient procoagulant activity such that it would be therapeutically useful if administered for replacement therapy. In one embodiment, the coagulation factor is a functional FVIII polypeptide, such as without limitation a FVIII concentrate from plasma or recombinantly produced FVIII, or Factor IX (FIX).

The term "polypeptide" as used herein refers to any peptide or protein comprising two or more amino acids joined to each other in a linear chain by peptide bonds. The term refers to both short chains, which also commonly are referred to in the art as peptides, oligopeptides and oligomers, for example, and to longer chains, which generally are referred to in the art as proteins, of which there are many types. Proteins may comprise one or more polypeptide chains. It will be appreciated that polypeptides may contain amino acids other than the 20 amino acids commonly referred to as the 20 naturally occurring amino acids, and that many amino acids, including the terminal amino acids, can be modified in a given polypeptide, either by natural processes, such as processing and other post-translational modifications, but also by chemical modification techniques which are well known to the art. Modifications can include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. Such modifications are well known to those of skill in the art. Several particularly common modifications, glycosylation, lipid attachment, sulfation, gamma-carboxylation of glutamic acid residues, hydroxylation and ADP-ribosylation, for instance, are described in most basic texts, such as, for example PROTEINS--STRUCTURE AND MOLECULAR PROPERTIES, 2nd Ed., T. E. Creighton, W. H. Freeman and Company, New York (1993). Modifications can occur anywhere in a polypeptide, including the peptide backbone, the amino acid side-chains and the amino or carboxyl termini. In fact, blockage of the amino or carboxyl group in a polypeptide, or both, by a covalent modification, is common in naturally occurring and synthetic polypeptides and such modifications may be present in polypeptides of the present embodiments, as well. During post-translational modification of the peptide, a methionine residue at the NH₂-terminus may be deleted. Accordingly, these embodiments contemplate the use of both the methionine-containing and the methionineless amino terminal variants of the protein of the embodiments. The modifications that occur in a polypeptide often will be a function of how it is made. For polypeptides made by expressing a cloned gene in a host, for instance, the nature and extent of the modifications in large part will be determined by the host cell posttranslational modification capacity and the modification signals present in the polypeptide amino acid sequence. For instance, as is well known, glycosylation often does not occur in bacterial hosts such as, for example, *E. coli.* Accordingly, when glycosylation is desired, a polypeptide should be expressed in a glycosylating host, generally a eukaryotic cell. It will be appreciated that the same type of modification may be present in the same or varying degree at several sites in a given polypeptide. Also, a given polypeptide may contain many types of modifications. In general, as used herein, the term polypeptide encompasses all such modifications, particularly those that are present in polypeptides synthesized by expressing a polynucleotide in a host cell.

In some embodiments, the coagulation factor protein may be a recombinant protein, a natural protein or a synthetic protein. In certain embodiments it is a recombinant protein. In some embodiments, the subject is administered a conjugate comprising a coagulation factor protein, variant or antigenic fragment which has the same amino acid sequence as the coagulation factor protein used in replacement therapy in the subject.

In some embodiments, the coagulation factors are mammalian in origin. In some embodiments, the coagulation factor proteins have an origin selected from the group consisting of human, non-human primate, mouse, rat, pig, cat, dog, cow, horse, rabbit and monkey. In one embodiment, the coagulation factor protein is a human protein.

In one embodiment, the coagulation factor protein is recombinant human FVIII or an antigenic fragment or variant thereof.

In another embodiment, the coagulation factor protein is full length recombinant FVIII, based on the amino acid sequence of the product KOGENATE. In some embodiments, the coagulation factor protein is selected from the group consisting of SEQ ID NO:1, SEQ ID NO:2 and a combination thereof.

In another embodiment, the coagulation factor protein is a B-domain deleted recombinant FVIII. In some embodiments, the B-domain deleted recombinant FVIII is selected from the group consisting of SEQ ID NO:5, SEQ ID NO: 6 and a combination thereof.

In another embodiment, the coagulation factor protein is full length recombinant FVIII, based on any human FVIII amino acid sequence found in nature.

In another embodiment, the coagulation factor protein is any FVIII product used in replacement therapy.

In another embodiment, the coagulation factor protein is a B-domain deleted recombinant FVIII, based on any human FVIII amino acid sequence where the B-domain is deleted completely or in part.The conjugates may also comprise variants of a coagulation factor protein. The term "variant" as applied to proteins as used herein, is a protein that differs from a reference protein. Examples of variants in this sense are described below and elsewhere in the present disclosure in greater detail. With reference to proteins generally, differences can be limited so that the sequences of the reference and the variant are closely similar overall and, in many regions, identical. A variant and reference protein can differ in amino acid sequence by one or more substitutions, additions, deletions, fusions and truncations, which may be present in any combination. Variants can also encompass proteins that have the same amino acid sequence as a reference sequence, but exhibit differences with respect to one or more post-translational modifications, such as glycosylation or pegylation.

In some embodiments, the coagulation factor protein is a variant that has been modified by attachment with one or more biocompatible polymers to improve, e.g., half-life or stability. Suitable biocompatible polymers include polyalkylene oxides such as, without limitation, polyethylene glycol (PEG), dextrans, colominic acids or other carbohydrate based polymers, polymers of amino acids, biotin derivatives, polyvinyl alcohol (PVA), polycarboxylates, polyvinylpyrrolidone, polyethylene-co-maleic acid anhydride, polystyrene-co-malic acid anhydride, polyoxazoline, polyacryloylmorpholine, heparin, albumin, celluloses, hydrolysates of chitosan, starches such as hydroxyethyl-starches and hydroxy propyl-starches, glycogen, agaroses and derivatives thereof, guar gum, pullulan, inulin, xanthan gum, carrageenan, pectin, alginic acid hydrolysates, other bio-polymers and any equivalents thereof. In one embodiment, the polymer is polyethylene glycol (PEG). In another embodiment, the polymer is methoxypolyethylene glycol (mPEG). Other useful polyalkylene glycol compounds are polypropylene glycols (PPG), polybutylene glycols (PBG), PEG-glycidyl ethers (Epox-PEG), PEG-oxycarbonylimidazole (CDI-PEG), branched polyethylene glycols, linear polyethylene glycols, forked polyethylene glycols and multi-armed or "super branched" polyethylene glycols (star-PEG).

"PEG" and "polyethylene glycol" as used herein are interchangeable and include any water-soluble poly(ethylene oxide). Typically, PEGs for use in accordance with the embodiments comprise the following structure "-(OCH₂CH₂)ₙ--" where (n) is 2 to 4000. As used herein, PEG also includes "-CH₂CH₂--O(CH₂CH₂O)ₙ--CH₂CH₂--" and "--(OCH₂CH₂)ₙO--," depending upon whether or not the terminal oxygens have been displaced. Throughout the specification and claims, it should be remembered that the term "PEG" includes structures having various terminal or "end capping" groups, such as, without limitation, a hydroxyl or a C₁₋₂₀ alkoxy group. The term "PEG" also means a polymer that contains a majority, that is to say, greater than 50%, of --OCH₂CH₂-repeating subunits. With respect to specific forms, the PEG can take any number of a variety of molecular weights, as well as structures or geometries such as branched, linear, forked, and multifunctional. PEGylation is a process whereby a polyethylene glycol (PEG) is covalently attached to a molecule such as a protein. In some embodiments, PEGylation can enhance the half-life of the protein after administration. In some embodiments, the coagulation factor is conjugated to PEG. In one embodiment, the coagulation factor is FVIII and is conjugated to PEG 1) directly to 1 or more pre-existing carbohydrates on any domain of FVIII; 2) directly to any available or engineered cysteines on any domain of FVIII; 3) to any other amino acid on FVIII; or 4) any combination thereof.

In some embodiments, the coagulation factor protein or variant or antigenic fragment thereof may be mutated at a predetermined site and then covalently attached at that site to a biocompatible polymer. Methods of attaching biocompatible polymers to coagulation factors can be found, *e.g.,* in U.S. Application Pub. No.: 2006/0115876. The biocompatible polymer that can be used in the conjugates of the embodiments may be any of the polymers discussed above. The biocompatible polymer can be selected to provide the desired improvement in pharmacokinetics. For example, in some embodiments, the identity, size and structure of the polymer is selected so as to improve the circulation half-life of the polypeptide or decrease the antigenicity of the polypeptide without an unacceptable decrease in activity. In some embodiments, the polymer comprises PEG, and in some embodiments has at least 50% of its molecular weight as PEG. In one embodiment, the polymer is a polyethylene glycol terminally capped with an end-capping moiety such as hydroxyl, alkoxy, substituted alkoxy, alkenoxy, substituted alkenoxy, alkynoxy, substituted alkynoxy, aryloxy and substituted aryloxy. In one embodiment the polymer comprises methoxypolyethylene glycol. In other embodiments, the polymers comprise methoxypolyethylene glycol having a size range from 3 kD to 100 kD, from 5 kD to 64 kD or from 5 kD to 43 kD.

In some embodiments, the biocompatible polymer has a reactive moiety. For example, in one embodiment, the polymer has a sulfhydryl reactive moiety that can react with a free cysteine on a polypeptide to form a covalent linkage. Such sulfhydryl reactive moieties include thiol, triflate, tresylate, aziridine, oxirane, S-pyridyl or maleimide moieties. In some embodiments the reactive moiety is a maleimide moiety. In one embodiment, the polymer is linear and has a "cap" at one terminus that is not strongly reactive towards sulfhydryls (such as methoxy) and a sulfhydryl reactive moiety at the other terminus. In one embodiment, the conjugate comprises PEG-maleimide and has a size range from 5 kD to 64 kD.

Site-directed mutation of a nucleotide sequence encoding a coagulation factor polypeptide or antigenic fragment or variant thereof may occur by any method known in the art. Some methods include mutagenesis to introduce a cysteine codon at the site chosen for covalent attachment of the polymer. This may be accomplished using a commercially available site-directed mutagenesis kit such as the Stratagene cQuickChange™. II site-directed mutagenesis kit, the Clontech Transformer site-directed mutagenesis kit no. K1600-1, the Invitrogen GenTaylor site-directed mutagenesis system no. 12397014, the Promega Altered Sites II in vitro mutagenesis system kit no. Q6210, or the Takara Mirus Bio LA PCR mutagenesis kit no. TAK RR016.

In some embodiments, the variants comprising a biocompatible polymer may be prepared by first replacing the codon for one or more amino acids on the surface of the polypeptide with a codon for cysteine, producing the cysteine variant in a recombinant expression system, reacting the variant with a cysteine-specific polymer reagent, and purifying the variant. In this system, the addition of a polymer at the cysteine site can be accomplished through a maleimide active functionality on the polymer. The amount of sulfhydryl reactive polymer used can be at least equimolar to the molar amount of cysteines to be derivatized and in some embodiments is present in excess. In some embodiments, at least a 5-fold molar excess of sulfhydryl reactive polymer is used, or at least a ten-fold excess of such polymer is used. Other conditions useful for covalent attachment are within the skill of those in the art.

In some embodiments, the variant comprises a protein in which one or more of the amino acid residues are substituted with a conserved or non-conserved amino acid residue and such substituted amino acid residue may or may not be one encoded by the genetic code. Conservative substitutions are those that substitute a given amino acid in a protein by another amino acid of like characteristics. In some embodiments, the variant is a conservative variant that has at least about 80% identity to the original antigen and the substitutions between the sequence of the antigenic variant and the original antigen are conservative amino acid substitutions. The following substitutions are considered conservative amino acid substitutions: valine, isoleucine, or leucine are substituted for alanine; lysine, glutamine, or asparagine are substituted for arginine; glutamine, histidine, lysine, or arginine are substituted for asparagine; glutamic acid is substituted for aspartic acid; serine is substituted for cysteine; asparagine is substituted for glutamine; aspartic acid is substituted for glutamic acid; proline or alanine is substituted for glycine; asparagine, glutamine, lysine or arginine is substituted for histidine; leucine, valine, methionine, alanine, phenylalanine, or norleucine is substituted for isoleucine; norleucine, isoleucine, valine, methionine, alanine, or phenylalanine is substituted for leucine; arginine, glutamine, or asparagine is substituted for lysine; leucine, phenylalanine, or isoleucine is substituted for methionine; leucine, valine, isoleucine, alanine, or tyrosine is substituted for phenylalanine; alanine is substituted for proline; threonine is substituted for serine; serine is substituted for threonine; tyrosine or phenylalanine is substituted for tryptophan; tryptophan, phenylalanine, threonine, or serine is substituted for tyrosine; tryptophan, phenylalanine, threonine, or serine is substituted for tyrosine; isoleucine, leucine, methionine, phenylalanine, alanine, or norleucine is substituted for valine. In some embodiments, the variant is a convervative variant that has at least about 90% identity to the original antigen.

In some embodiments, the variant comprises a protein in which one or more of the amino acid residues includes a substituent group. In some embodiments, the variant comprises a protein that is fused with one or more other compounds. In some embodiments, the variant comprises a protein in which additional amino acids are fused to the mature protein, such as a leader or secretory sequence or a sequence which is employed for purification of the mature protein or a proprotein sequence. Such variants are deemed to be obtained by those of ordinary skill in the art, from the teachings herein.

In some embodiments, the variant has at least 100% of the activity of the native protein. In some embodiments, the variant has at least 50% of the activity of the native coagulation factor protein. In some embodiments, the variant has at least 70%, 80%, 85%, 90%, 95%, 96%, 97%, 98% or 99% or more of the activity of the native coagulation factor protein.

In some embodiments, 1, 2, 3, 4, 5, 6, 7, 8, 9, 10 or no amino acid residues are substituted, deleted or added, in any combination. In some embodiments, the coagulation factor protein comprises silent substitutions, additions and deletions, which do not alter the properties and activities of the coagulatant factor protein.

In some embodiments, variants include portions of a reference sequence which generally contain at least 30 contiguous amino acids or at least 50-100 contiguous amino acids which are identical to the reference sequence.

In some embodiments, the proteins are provided in an isolated form, and in some embodiments are purified to substantial homogeneity using known methods and techniques of protein isolation and purification.

The conjugates of the embodiments may also comprise an antigenic fragment of the coagulation factor proteins. In this regard an antigenic fragment is a polypeptide having an amino acid sequence that entirely is the same as part but not all of the amino acid sequence of the aforementioned reference polypeptides and variants thereof and which is capable of generating an antibody response. An antigenic fragment of a coagulation factor protein comprises at least one epitope from the protein.

The antigenic fragment may be of any length, but is most typically at least about 6 amino acids, at least about 9 amino acids, at least about 12 amino acids, at least about 20 amino acids, at least about 30 amino acids, at least about 50 amino acids, or at least about 100 amino acids. Larger antigenic fragments are also contemplated.

Such antigenic fragments may be "free-standing," i.e., not part of or fused to other amino acids or polypeptides, or they may be comprised within a larger polypeptide of which they form a part or region. When comprised within a larger polypeptide, the presently discussed fragments in some embodiments form a single continuous region. However, several fragments may be comprised within a single larger polypeptide. For instance, in some embodiments, an antigenic fragment of a polypeptide of the present embodiments can be comprised within a precursor polypeptide designed for expression in a host and having heterologous pre and/or pro-polypeptide regions fused to the amino terminus of the antigenic fragment and/or an additional region fused to the carboxyl terminus of the fragment. Therefore, fragments in one aspect of the meaning intended herein, refers to the portion or portions of a fusion polypeptide or fusion protein derived from a coagulation factor protein.

Representative examples of antigenic polypeptide fragments of the embodiments, include, for example, those which have from about 5-15, 10-20, 15-40, 30-55, 41-75, 41-80, 41-90, 50-100, 75-100, 90-115, 100-125, and 110-140 amino acids in length.

In some embodiments, the polypeptide is part of a fusion protein encoded by a recombinant nucleic acid molecule, expression cassette, or expression vector and is heterologous to the signal peptide of the fusion protein.

In some embodiments, a polynucleotide encoding the polypeptide is codon-optimized for expression in the host cell. In some embodiments, the amino acid sequence of an antigenic fragment has at least about 30%, at least about 40%, at least about 50%, at least about 60%, at least about 70%, at least about 80%, at least about 90%, at least about 95%, or at least about 98% identity to the original protein.

### Factor VII (FVII)

FVII is a vitamin K-dependent plasma protein synthesized in the liver and secreted into the blood as a single-chain glycoprotein with a molecular weight of 53 kDa (Broze & Majerus, J. Biol. Chem 1980; 255:1242-1247 (1980)). The FVII zymogen is converted into an activated form (FVIIa) by proteolytic cleavage at a single site, R152-I153, resulting in two chains linked by a single disulfide bridge. FVIIa in complex with tissue factor (FVIIa complex) is able to convert both factor IX and factor X into their activated forms, followed by reactions leading to rapid thrombin production and fibrin formation (Osterud & Rapaport, Proc Natl Acad Sci USA 1977; 74:5260-5264 (1977)).

The gene coding for human FVII (hFVII) has been mapped to chromosome 13 at q34-qter 9 (de Grouchy et al., Hum Genet 66:230-233 (1984)). It contains nine exons and spans 12.8 Kb (O'Hara et al., Proc Natl Acad Sci USA 84:5158-5162 (1987)). The gene organization and protein structure of FVII are similar to those of other vitamin K-dependent procoagulant proteins, with exons 1a and 1b encoding for signal sequence; exon 2 the propeptide and Gla domain; exon 3 a short hydrophobic region; exons 4 and 5 the epidermal growth factor-like domains; and exon 6 through 8 the serine protease catalytic domain (Yoshitake et al., Biochemistry 1985; 24: 3736-3750). Commercial preparations of human recombinant FVIIa are sold as NOVOSEVEN. NOVOSEVEN is indicated for the treatment of bleeding episodes in hemophilia A or B patients.

The FVII molecules useful for the present embodiments include the full length protein, precursors of the protein, subunits or fragments of the protein, and variants and antigenic fragments thereof. Reference to FVII is meant to include all potential forms of such proteins.

In some embodiments, the FVII polypeptide comprises SEQ ID NO:9, although allelic variants are possible. Factor VII, variants, fragments, and/or methods of making the same also useful in the embodiments are described in, *e.g.,* the following U.S. Patent Appl. Publications and U.S. Patents: 20130084274; 20130017184; 20120321607; 20120263701; 20120208860; 20120178693; 20120171765; 20120115204; 20120087908; 20120064075; 20120004176; 20120003206; 20110250702; 20110097754; 20110064719; 20110059894; 20110059510; 20110046061; 20110045535; 20110040073; 20110003363; 20100330059; 20100303786; 20100294677; 20100260741; 20100197597; 20100166730; 20100166729; 20100158891; 20100145009; 20100124547; 20100120093; 20100113743; 20100056453; 20100015684; 20100009396; 20090311239; 20090305967; 20090291890; 20090281022; 20090264511; 20090263866; 20090239788; 20090227504; 20090221484; 20090181895; 20090162871; 20090130085; 20090104661; 20090098103; 20090093616; 20090093410; 20090087864; 20090075895; 20090055942; 20090047723; 20090043080; 20090042784; 20090041747; 20090023635; 20090017007; 20090011992; 20080318276; 20080312161; 20080286259; 20080274534; 20080268521; 20080227715; 20080206227; 20080206225; 20080175878; 20080145914; 20080102064; 20080076702; 20080075711; 20080075709; 20080069810; 20080058266; 20080058255; 20080057059; 20080039373; 20080010693; 20070243588; 20070219135; 20070207960; 20070207956; 20070190574; 20070142625; 20070142280; 20070129298; 20070122884; 20070099229; 20070049523; 20070037966; 20070027077; 20070021338; 20060293241; 20060276398; 20060276377; 20060270002; 20060270001; 20060270000; 20060258585; 20060252690; 20060252689; 20060252129; 20060252127; 20060252039; 20060240525; 20060240524; 20060234935; 20060228782; 20060211621; 20060205648; 20060205036; 20060183683; 20060166915; 20060166882; 20060111282; 20060063714; 20060052286; 20060045879; 20060030531; 20060025336; 20060019336; 20060013812; 20050267014; 20050266006; 20050204411; 20050204406; 20050202002; 20050113565; 20050075289; 20050032690; 20050032109; 20040258690; 20040248793; 20040197370; 20040192602; 20040186277; 20040117862; 20040087498; 20040063187; 20040043933; 20040037893; 20040009918; 20040009543; 20040006020; 20030215447; 20030203845; 20030170863; 20030152567; 20030130191; 20030125256; 20030124622; 20030124118; 20030119743; 20030119741; 20030119723; 20030118582; 20030118580; 20030118574; 20030109446; 20030104978; 20030100740; 20030100075; 20030096338; 20030077271; 20030044908; 20030040480; 20030003096; 20020151471; 20020142316; 20020137673; 20020110552; 20010007901; 8,334,273; 8,318,904; 8,299,029; 8,084,591; 8,053,410; 8,026,214; 8,022,031; 8,008,252; 7,951,910; 7,943,333; 7,892,842; 7,879,803; 7,871,985; 7,863,009; 7,829,095; 7,803,569; 7,790,852; 7,786,070; 7,754,682; 7,732,405; 7,700,733; 7,622,558; 7,598,056; 7,517,974; 7,511,024; 7,442,524; 7,442,514; 7,427,592; 7,419,803; 7,416,861; 7,416,860; 7,414,022; 7,371,543; 7,291,587; 7,235,638; 7,202,065; 7,176,288; 7,153,679; 7,125,846; 7,078,479; 7,052,868; 7,026,524; 6,960,657; 6,919,311; 6,911,334; 6,911,323; 6,905,683; 6,903,069; 6,835,817; 6,831,167; 6,806,063; 6,777,390; 6,677,440; 6,573,056; 6,528,299; 6,479,245; 6,329,176; 6,268,163; 6,183,743; 6,168,789; 6,039,944; 5,997,864; 5,968,759; 5,962,418; 5,948,759; 5,874,408; 5,861,374; 5,859,010; 5,833,982; 5,824,639; 5,817,788; 5,788,965; 5,750,358; 5,741,658; 5,700,914; 5,472,850; 5,344,918; 5,288,629; 5,190,919; 4,784,950; 4,456,591; and 3,962,427,

### Factor VIII (FVIII)

Blood clotting FVIII is a glycoprotein synthesized and released into the bloodstream by the liver. As a secreted protein, FVIII contains a signal sequence that is proteolytically cleaved during the translation process. Following removal of the 19 amino acid signal sequence, the first amino acid of the secreted FVIII product is an alanine. In the circulating blood, it is bound to von Willebrand factor (vWF, also known as Factor VIII-related antigen) to form a stable complex. Upon activation by thrombin, it dissociates from the complex to interact with other clotting factors in the coagulation cascade, which eventually leads to the formation of a thrombus.

FVIII itself does not cause coagulation, but plays an essential role in the coagulation cascade. The role of FVIII in coagulation is to be activated to FVIIIa, which is a catalytic cofactor for intrinsic FX activation (Thompson, Semin. Thromb. Hemost. 29 :11-22 (2003)). FVIII is proteolytically activated by thrombin or FXa, which dissociates it from von Willebrand factor (vWf) and activates its procoagulant function in the cascade. In its active form, FVIIIa functions as a cofactor for the FX activation enzyme complex in the intrinsic pathway of blood coagulation, and it is decreased or nonfunctional in patients with hemophilia A.

In some embodiments, the FVIII useful with the present embodiments includes those forms, which are biologically active including the full length FVIII and any derivative capability of acting as a cofactor in the activation of coagulation FIX and the capability of forming a complex with VWF. In some embodiments, the FVIII used according to the present embodiments may be a plasma-derived FVIII (pdFVIII) or a recombinant FVIII (rFVIII) or biologically active derivatives thereof. The pdFVIII and the rFVIII may be produced by any method known in the art. PdFVIII may be purified by any suitable means. One useful method is described in U.S. Pat. No. 5,470,954. rFVIII proteins may be prepared by any suitable means. Examples of such rFVIII include Recombinate™ and Advate®, both manufactured and sold by Baxter Healthcare Corporation; ReFacto®, a B-domain deleted form of FVIII manufactured and sold by Wyeth Corporation; and KOGENATE, manufactured and sold by Bayer Corporation. Methods and examples of rFVIII are described in U.S. Pat. Nos. 4,757,006; 4,965,199; and 5,618,788. Other commercial preparations of FVIII which can be used to induce tolerance include Alphanate®, Bioclate®, Helixate® FS, Hemofil® M, Humate-P®, Hyate C®, Koate®-DVI, Kogenate® FS, Monarc-M™, Monarc-M™, Monarc-M® and Monoclate-P®.

In some embodiments, the FVIII polypeptides include allelic variations, glycosylated versions, modifications and fragments resulting in derivatives of FVIII so long as they contain the functional segment of human FVIII and the essential, characteristic human FVIII functional activity.

In some embodiments, the FVIII molecules include the full length protein, precursors of the protein, subunits or fragments of the protein, and variants and antigenic fragments thereof. Reference to FVIII is meant to include all potential forms of such proteins.

In some embodiments, the FVIII polypeptides comprise full-length human FVIII. In some embodiments, the full length FVIII comprises an amino acid sequence selected from the group consisting of SEQ ID NO: 1, SEQ ID NO: 2 and a combination thereof, although allelic variants are possible. As a secreted protein, FVIII contains a signal sequence that is proteolytically cleaved during the translation process. Following removal of the 19 amino acid signal sequence, the first amino acid of the secreted FVIII product is an alanine.

In some embodiments, the human FVIII is B-domain deleted FVIII (BDD). As used herein, BDD is characterized by having the amino acid sequence which contains a deletion of all but 14 amino acids of the B-domain of FVIII. The first 4 amino acids of the B-domain (SEQ ID NO:3) are linked to the 10 last residues of the B-domain (NPPVLKRHQR, SEQ ID NO:4). In some embodiments, the BDD FVIII comprises an amino acid sequence selected from the group consisting of SEQ ID NO: 5 and SEQ ID NO: 6 and a combination thereof.

Factor VIII, variants fragments, and/or methods of making the same also useful in the embodiments are described in, *e.g.,* the following U.S. Patent Appl. Publications and U.S. Patents: 20130085110; 20130072434; 20130040889; 20130040888; 20130017997; 20130012442; 20130005656; 20130004462; 20120322737; 20120308641; 20120270266; 20120245289; 20120244597; 20120232252; 20120225819; 20120190623; 20120178692; 20120178691; 20120142594; 20120142593; 20120093840; 20120083446; 20120065136; 20120045819; 20120028900; 20110286988; 20110262424; 20110206651; 20110183907; 20110178019; 20110160435; 20110124565; 20110118188; 20110112028; 20110112027; 20110112026; 20110112025; 20110112024; 20110112023; 20110112022; 20110077203; 20110039302; 20100305305; 20100292440; 20100284971; 20100261872; 20100256062; 20100233119; 20100204452; 20100197578; 20100183556; 20100173831; 20100173830; 20100172891; 20100168391; 20100168018; 20100167392; 20100130427; 20100125049; 20100120689; 20100120094; 20100113365; 20100113364 20100112641 20100099113; 20100003254; 20090325881; 20090305349; 20090297503; 20090297498; 20090275141; 20090271163; 20090263380; 20090247459; 20090215070; 20090215025; 20090208512; 20090203077; 20090130094; 20090118185; 20090118184; 20090076237; 20090041714; 20080312143; 20080300174 20080234193 20080219983 20080206254; 20080176791 20080160015; 20080076702; 20080070275; 20080070251; 20080058504 20080044430; 20070275880; 20070265199; 20070244301; 20070232789; 20070232788; 20070215475; 20070135342; 20070065425; 20060293505; 20060293238 20060276398; 20060239998; 20060233786; 20060205661; 20060193829; 20060160994; 20060099685; 20060051367; 20060014683;20050276787; 20050256304; 20050256038; 20050229261; 20050165221;20050118684; 20050100990; 20050079584; 20050074836; 20050060775; 20050009148; 20040249134; 20040248785; 20040235734; 20040197875; 20040197390; 20040166150; 20040147436; 20040126774; 20040120951; 20040116345; 20040092442; 20040087776; 20040062752; 20040038396; 20030199444; 20030166536; 20030165822; 20030148953; 20030147900; 20030134778; 20030129174; 20030106798; 20030099618; 20030083257; 20030077752; 20030068785; 20020182684; 20020182670; 20020159977; 20020146729; 20020132306; 20020115832; 20020115152; 20020102730; 20020068303; 20010010815; 8,399,620; 8,372,800; 8,349,800; 8,338,571; 8,329,871; 8,309,086; 8,293,234; 8,282,923; 8,252,287; 8,247,536; 8,236,518; 8,198,421; 8,188,246; 8,183,345; 8,183,344; 8,173,597; 8,143,378; 8,133,977; 8,133,865; 8,110,190; 8,076,292; 8,071,728; 8,071,727; 8,071,726; 8,071,725; 8,071,724; 8,071,094; 8,067,543; 8,058,226; 8,058,017; 8,053,561; 8,038,993; 8,003,760; 7,985,839; 7,985,838; 7,982,010; 7,981,865; 7,960,182; 7,932,355; 7,884,075; 7,867,974; 7,863,421; 7,858,749; 7,855,274; 7,829,085; 7,820,796; 7,790,680; 7,785,594; 7,691,565; 7,683,158; 7,678,761; 7,645,860; 7,635,763; 7,615,622; 7,582,296; 7,560,107; 7,544,660; 7,507,540; 7,459,534; 7,459,525; 7,351,577; 7,247,707; 7,214,785; 7,211,559; 7,199,223; 7,157,277; 7,144,487; 7,122,634; 7,112,438; 7,087,723; 7,041,635; 7,033,791; 7,012,132; 6,967,239; 6,930,087; 6,887,852; 6,866,848; 6,838,437; 6,800,461; 6,780,614; 6,770,744; 6,759,216; 6,683,159; 6,599,724; 6,593,294; 6,586,573; 6,518,482; 6,517,830; 6,492,105; 6,458,563; 6,376,463; 6,358,703; 6,355,422; 6,346,513; 6,316,226; 6,307,032; 6,284,871; 6,271,025; 6,255,554; 6,251,632; 6,221,349; 6,200,560; 6,197,526; 6,191,256; 6,180,371; 6,171,825; 6,143,179; 6,057,164; 6,037,452; 6,005,082; 5,998,589; 5,994,310; 5,972,885; 5,962,650; 5,952,198; 5,925,739; 5,919,908; 5,919,766; 5,888,974; 5,880,327; 5,859,204; 5,831,026; 5,824,780; 5,804,420; 5,763,401; 5,747,337; 5,744,446; 5,733,873; 5,714,590; 5,707,832; 5,693,499; 5,681,746; 5,679,776; 5,679,549; 5,668,108; 5,663,060; 5,661,008; 5,659,017; 5,633,150; 5,618,789; 5,618,788; 5,610,278; 5,605,884; 5,597,711; 5,583,209; 5,576,291; 5,565,427; 5,543,502; 5,543,145; 5,506,112; 5,470,954; 5,424,401; 5,422,250; 5,410,022; 5,399,670; 5,371,195; 5,364,771; 5,362,854; 5,356,878; 5,328,694; 5,288,853; 5,260,274; 5,259,951; 5,214,033; 5,177,191; 5,171,844; 5,112,950; 5,110,907; 5,101,016; 5,091,363; 5,043,429; 5,043,428; 4,981,951; 4,970,300; 4,965,199; 4,886,876; 4,857,635; 4,845,074; 4,822,872; 4,814,435; 4,789,733; 4,769,336; 4,675,385; 4,657,894; 4,650,858; 4,649,132; 4,578,218; 4,556,558; RE32,011; 4,522,751; 4,456,590; 4,446,134; 4,406,886; 4,404,131; 4,387,092; 4,383,989; 4,370,264; 4,361,509; 4,359,463; 4,348,384; 4,348,315; 4,302,445; 4,289,691; 4,250,008; 4,235,881; 4,221,780; 4,203,891; 4,188,318; 4,093,608; 4,085,095; 4,069,216; and 4,027,013.

In some embodiments, FVIII can be modified with a biocompatible polymer, such as PEG. Pegylated forms of Factor VIII are disclosed in WO 2006/053299 and U.S. Patent Application Pub. No. 20060115876.

In the examples of FVIII that follow, the FVIII muteins are named in a manner conventional in the art. As used herein, a "mutein" is a genetically engineered protein arising as a result of a laboratory induced mutation to a protein or polypeptide The convention for naming mutants is based on the amino acid sequence for the mature, full length Factor VIII as provided in SEQ ID NO:2.

As is conventional and used herein, when referring to mutated amino acids in BDD FVIII, the mutated amino acid is designated by its position in the sequence of full-length FVIII. For example, the PEG6 mutein discussed below is designated K1808C because it changes the lysine (K) at the position analogous to 1808 in the full-length sequence to cysteine (C). In some embodiments, for the mutants discussed below, a cysteine replaces the natural amino acid at the designated location of the full length FVIII or the B-domain deleted FVIII, and a biocompatible polymer, such as PEG, is attached to the cysteine residue.

The predefined site for covalent binding of a biocompatible polymer, such as PEG, is best selected from sites exposed on the surface of the polypeptide that are not involved in FVIII activity or involved in other mechanisms that stabilize FVIII in vivo, such as binding to vWF. Such sites are also best selected from those sites known to be involved in mechanisms by which FVIII is deactivated or cleared from circulation. Selection of these sites is discussed in detail below. In some embodiments, sites include an amino acid residue in or near a binding site for (a) low density lipoprotein receptor related protein, (b) a heparin sulphate proteoglycan, (c) low density lipoprotein receptor and/or (d) factor VIII inhibitory antibodies. By "in or near a binding site" means a residue that is sufficiently close to a binding site such that covalent attachment of a biocompatible polymer to the site would result in steric hindrance of the binding site. Such a site is expected to be within 20 Angstroms of a binding site, for example.

In one embodiment, the biocompatible polymer is covalently attached to the functional factor VIII polypeptide at an amino acid residue in or near (a) a factor VIII clearance receptor as defined supra, (b) a binding site for a protease capable of degradation of factor VIII and/or (c) a binding site for factor VIII inhibitory antibodies. The protease may be activated protein C (APC). In another embodiment, the biocompatible polymer is covalently attached at the predefined site on the functional factor VIII polypeptide such that binding of low-density lipoprotein receptor related protein to the polypeptide is less than to the polypeptide when it is not conjugated, and in some embodiments more than twofold less. In one embodiment, the biocompatible polymer is covalently attached at the predefined site on the functional factor VIII polypeptide such that binding of heparin sulphate proteoglycans to the polypeptide is less than to the polypeptide when it is not conjugated, and in some embodiments is more than twofold less. In a further embodiment, the biocompatible polymer is covalently attached at the predefined site on the functional factor VIII polypeptide such that binding of factor VIII inhibitory antibodies to the polypeptide is less than to the polypeptide when it is not conjugated, in some embodiments more than twofold less than the binding to the polypeptide when it is not conjugated. In another embodiment, the biocompatible polymer is covalently attached at the predefined site on the functional factor VIII polypeptide such that binding of low density lipoprotein receptor to the polypeptide is less than to the polypeptide when it is not conjugated, in some embodiments more than twofold less. In another embodiment, the biocompatible polymer is covalently attached at the predefined site on the functional factor VIII polypeptide such that a plasma protease degrades the polypeptide less than when the polypeptide is not conjugated. In a further embodiment, the degradation of the polypeptide by the plasma protease is more than twofold less than the degradation of the polypeptide when it is not conjugated as measured under the same conditions over the same time period.

LRP, LDL receptor, or HSPG binding affinity for FVIII can be determined using surface plasmon resonance technology (Biacore). For example, FVIII can be coated directly or indirectly through a FVIII antibody to a Biacore chip, and varying concentrations of LRP can be passed over the chip to measure both on-rate and off-rate of the interaction (Bovenschen N. et al., 2003, J. Biol. Chem. 278(11), pp. 9370-7). The ratio of the two rates gives a measure of affinity. In some embodiments, a two-fold, five-fold, ten-fold, or 30-fold decrease in affinity upon PEGylation would be desired.

Degradation of a FVIII by the protease APC can be measured by any of the methods known to those of skill in the art.

In one embodiment, the biocompatible polymer is covalently attached to the polypeptide at one or more of the FVIII (SEQ ID NO:2) amino acid positions 81, 129, 377, 378, 468, 487, 491, 504, 556, 570, 711, 1648, 1795, 1796, 1803, 1804, 1808, 1810, 1864, 1903, 1911, 2091, 2118 and 2284. In another embodiment, the biocompatible polymer is covalently attached to the polypeptide at one or more of factor VIII (SEQ ID NO:2) amino acid positions 377, 378, 468, 491, 504, 556, 1795, 1796, 1803, 1804, 1808, 1810, 1864, 1903, 1911 and 2284 and (1) the binding of the conjugate to low-density lipoprotein receptor related protein is less than the binding of the unconjugated polypeptide to the low-density lipoprotein receptor related protein; (2) the binding of the conjugate to low-density lipoprotein receptor is less than the binding of the unconjugated polypeptide to the low-density lipoprotein receptor; or (3) the binding of the conjugate to both low-density lipoprotein receptor related protein and low-density lipoprotein receptor is less than the binding of the unconjugated polypeptide to the low-density lipoprotein receptor related protein and the low-density lipoprotein receptor. In one embodiment, residue 1804 in a B-domain deleted FVIII is mutated to cysteine and conjugated to PEG.

In a further embodiment, the biocompatible polymer is covalently attached to the polypeptide at one or more of FVIII (SEQ ID NO:2) amino acid positions 377, 378, 468, 491, 504, 556 and 711 and the binding of the conjugate to heparin sulphate proteoglycan is less than the binding of the unconjugated polypeptide to heparin sulphate proteoglycan. In a further embodiment, the biocompatible polymer is covalently attached to the polypeptide at one or more of the factor VIII (SEQ ID NO:2) amino acid positions 81, 129, 377, 378, 468, 487, 491, 504, 556, 570, 711, 1648, 1795, 1796, 1803, 1804, 1808, 1810, 1864, 1903, 1911, 2091, 2118 and 2284 and the conjugate has less binding to factor VIII inhibitory antibodies than the unconjugated polypeptide. In a further embodiment, the biocompatible polymer is covalently attached to the polypeptide at one or more of the factor VIII (SEQ ID NO:2) amino acid positions 81, 129, 377, 378, 468, 487, 491, 504, 556, 570, 711, 1648, 1795, 1796, 1803, 1804, 1808, 1810, 1864, 1903, 1911, 2091, 2118 and 2284, and in some embodiments at one or more of positions 377, 378, 468, 491, 504, 556, and 711 and the conjugate has less degradation from a plasma protease capable of factor VIII degradation than does the unconjugated polypeptide. In some embodiments, the plasma protease is activated protein C.

In a further embodiment, the biocompatible polymer is covalently attached to B-domain deleted factor VIII at amino acid position 129, 491, 1804, and/or 1808, and in some embodiments at 491 or 1808. In a further embodiment, the biocompatible polymer is attached to the polypeptide at factor VIII amino acid position 1804 and comprises polyethylene glycol. In some embodiments, the one or more predefined sites for biocompatible polymer attachment are controlled by site specific cysteine mutation.

One or more sites, in some embodiments one or two, on the functional factor VIII polypeptide may be the predefined sites for polymer attachment. In particular embodiments, the polypeptide is mono-PEGylated or diPEGylated.

The embodiments also relate to a method for the preparation of the conjugate comprising mutating a nucleotide sequence that encodes for the functional factor VIII polypeptide to substitute a coding sequence for a cysteine residue at a pre-defined site; expressing the mutated nucleotide sequence to produce a cysteine enhanced mutein; purifying the mutein; reacting the mutein with the biocompatible polymer that has been activated to react with polypeptides at substantially only reduced cysteine residues such that the conjugate is formed; and purifying the conjugate. Another embodiment provides a method for site-directed PEGylation of a factor VIII mutein comprising: (a) expressing a site-directed factor VIII mutein wherein the mutein has a cysteine replacement for an amino acid residue on the exposed surface of the factor VIII mutein and that cysteine is capped; (b) contacting the cysteine mutein with a reductant under conditions to mildly reduce the cysteine mutein and to release the cap; (c) removing the cap and the reductant from the cysteine mutein; and (d) at least about 5 minutes, and in some embodiments at least 15 minutes, in some embodiments at least 30 minutes after the removal of the reductant, treating the cysteine mutein with PEG comprising a sulfhydryl coupling moiety under conditions such that PEGylated factor VIII mutein is produced. The sulfhydryl coupling moiety of the PEG is selected from the group consisting of thiol, triflate, tresylate, aziridine, oxirane, S-pyridyl and maleimide moieties, in some embodiments maleimide.

In one embodiment the inventive method involves replacing one or more surface BDD amino acids with a cysteine, producing the cysteine mutein in a mammalian expression system, reducing a cysteine which has been capped during expression by cysteine from growth media, removing the reductant to allow BDD disulfides to reform, and reacting with a cysteine-specific biocompatible polymer reagent, such as such as PEG-maleimide. Examples of such reagents are PEG-maleimide with PEG sizes such as 5, 22, or 43 kD available from Nektar Therapeutics of San Carlos, Calif. under Nektar catalog numbers 2D2M0H01 mPEG-MAL MW 5,000 Da, 2D2M0P01 mPEG-MAL MW 20 kD, 2D3X0P01 mPEG2-MAL MW 40 kD, respectively, or 12 or 33 kD available from NOF Corporation, Tokyo, Japan under NOF catalog number Sunbright ME-120MA and Sunbright ME-300MA, respectively. The PEGylated product is purified using ionexchange chromatography to remove unreacted PEG and using size-exclusion chromatography to remove unreacted BDD. This method can be used to identify and selectively shield any unfavorable interactions with FVIII such as receptor-mediated clearance, inhibitory antibody binding, and degradation by proteolytic enzymes. It was noted that the PEG reagent supplied by Nektar or NOF as 5 kD tested as 6 kD in our laboratory, and similarly the PEG reagent supplied as linear 20 kD tested as 22 kD, that supplied as 40 kD tested as 43 kD and that supplied as 60 kD tested as 64 kD in our laboratory. To avoid confusion, I use the molecular weight as tested in our laboratory in the discussion herein, except for the 5 kD PEG, which I report as 5 kD as the manufacturer identified it.

In addition to cysteine mutations at positions 491 and 1808 of BDD (disclosed above), positions 487, 496, 504, 468, 1810, 1812, 1813, 1815, 1795, 1796, 1803, and 1804 were mutated to cysteine to potentially allow blockage of LRP binding upon PEGylation. Also, positions 377, 378, and 556 were mutated to cysteine to allow blockage of both LRP and HSPG binding upon PEGylation. Positions 81, 129, 422, 523, 570, 1864, 1911, 2091, and 2284 were selected to be equally spaced on BDD so that site-directed PEGylation with large PEGs (>40 kD) at these positions together with PEGylation at the native glycosylation sites (41, 239, and 2118) and LRP binding sites should completely cover the surface of BDD and identify novel clearance mechanism for BDD.

In one embodiment, the cell culture medium contains cysteines that "cap" the cysteine residues on the mutein by forming disulfide bonds. In the preparation of the conjugate, the cysteine mutein produced in the recombinant system is capped with a cysteine from the medium and this cap is removed by mild reduction that releases the cap before adding the cysteine-specific polymer reagent. Other methods known in the art for site-specific mutation of FVIII may also be used, as would be apparent to one of skill in the art.

### Factor IX (FIX)

Factor IX is essential in the blood coagulation cascade. A deficiency of Factor IX in the body characterizes a type of hemophilia (type B). Treatment of this disease is usually limited to intravenous transfusion of human plasma protein concentrates of Factor IX. The commercially available recombinant product is marketed under the trade name Benefix™.

The FIX molecules useful include the full length protein, precursors of the protein, subunits or fragments of the protein, and variants and antigenic fragments thereof. Reference to FIX is meant to include all potential forms of such proteins.

In some embodiments, the sequence of human FIX comprises SEQ ID NO:8, although allelic variants are possible. Factor IX, variants, fragments and/or methods of making thereof also useful are described in, *e.g.,* the following U.S. Patent Application Publications and U.S. Patents: 20130095555; 20120308540; 20120263703; 2012/0270300; 20120177625; 20120164130; 20110244550; 20110217284; 20110183906; 20110154516; 20110137011; 20110046060; 20100330060; 20100316625; 20100284971; 20100249033; 20100137511; 20100130684; 20100130428; 20100120982; 20100081791; 20100081712; 20090280550; 20090239797; 20090221492; 20090176708; 2009008188; 20080318850; 20080305991; 20080255026; 20080207897; 20080188414; 20080176287; 20080167219; 20080153156; 20080102115; 20080075711; 20070244036; 20060287228; 20060211621; 20060052302; 20060040856; 20050100982; 20040254106; 20040133930; 20040110675; 20040106779; 20030203845; 20020166130; 20020031799; 20010031721; 8,404,809; 8,399,632; 8,383,388; 8,198,421; 8,168,425; 8,030,065; 7,888,321; 7,888,067; 7,700,734; 7,579,444; 7,575,897; 7,419,948; 7,375,084; 7,179,617; 7,125,841; 6,670,176; 6,627,737; 6,531,298; 6,372,716; 6,344,596; 6,284,871; 6,280,729; 6,063,909; 6,046,380; 6,043,215; 6,037,452; 6,034,222; 5,969,040; 5,919,909; 5,919,908; 5,770,700; 5,714,583; 5,639,857; 5,621,039; 5,614,500; 5,521,070; 5,457,181; 5,409,990; 5,286,849; 5,281,661; 5,171,569; 5,061,789; 5,055,557; 4,786,726; 4,770,999; and 4,081,432.

### Factor X (FX)

Factor X (FX) is a vitamin K-dependent two-chain glycoprotein which plays a central role in blood coagulation. Factor X deficiency is a rare bleeding disorder which affects between 1 in 500,000 and 1 in 1,000,000 of the population. It is characterized by a tendency to excessive bleeding, similar to that caused by factor VIII and factor IX deficiencies in hemophilia A and B respectively.

The FX molecules useful include the full length protein, precursors of the protein, subunits or fragments of the protein, and variants and antigenic fragments thereof. Reference to FX is meant to include all potential forms of such proteins.

In some embodiments, the FX polypeptide comprises SEQ ID NO: 10, although allelic variants are possible. Factor X, variants, fragments and/or methods of making thereof also useful are described in, *e.g.,* the following U.S. Patent Application Publications and U.S. Patents: 20120231523; 20120039863; 20110275666; 20100285568; 20100233149; 20090175828; 20090053185; 20070207953; 20070032424; 20060148038; 20050153882; 20030207796; 20030181381; 20030138914; 8,293,874; 8,173,777; 8,168,753; 7,772,371; 7,220,569; 7,179,890; 6,958,322; 6,905,846; 6,783,953; 6,573,071; 6,562,598; 6,117,836; 5,798,332; and 4,501,731.

### Factor XI (FXI)

Human Factor XI is a two-chain glycoprotein with a molecular weight of approximately 160,000 daltons. The two chains are identical disulfide bonded polypeptides with molecular weights of approximately 80,000 daltons. Factor XI is activated to factor XIa by Factor XIIa. The amino acid sequence of human factor XI has been determined (see, e.g., Fujikawa et al., Biochemistry 25:2417-2424 (1986)) and is provided as SEQ ID NO:7. In humans, the gene for FXI is located at the distal end of chromosome 4 (4q35.2) and contains 15 exons spread over .about.25 kb of genomic DNA (Asaki et al., Biochemistry 26:7221-7228 (1987); Kato et al. Cytogenet. Cell Genet. 52:77 (1989)). In some embodiments, the sequence of human FXI is SEQ ID NO:7 (GenBank Accession No. P03951).

During activation of factor XI, an internal peptide bond is cleaved by factor XIIa in each of the two chains, resulting in activated factor XIa, a serine protease composed of two heavy and two light chains held together by disulfide bonds. Activated Factor XI triggers the middle phase of the intrinsic pathway of blood coagulation by activating factor IX. Defects in this factor lead to Rosenthal syndrome (also known as hemophilia C), a blood coagulation abnormality. The Factor XI protein is encoded by the F11 gene. FXI is also known as coagulation factor XI or plasma thromboplastin antecedent.

The cleavage site for the activation of factor XI by factor XIIa is an internal peptide bond between Arg-369 and Ile-370 in each polypeptide chain (Fujikawa et al. Biochemistry 25:2417-2424 (1986)). Each heavy chain of factor XIa (369 amino acids) contains four tandem repeats of 90-91 amino acids called apple domains (designated A1-A4) plus a short connecting peptide (Fujikawa et al. Biochemistry 25:2417-2424 (1986); Sun et al., J. Biol. Chem. 274:36373-36378 (1999)). The light chains of factor XIa (each 238 amino acids) contain the catalytic portion of the enzyme with sequences that are typical of the trypsin family of serine proteases (Fujikawa et al. Biochemistry 25:2417-2424 (1986)). XIa proteolytically cleaves its substrate, factor IX, in an interaction requiring the factor XI A3 domain (Sun, Y., and Gailani, D. J. Biol. Chem. 271, 29023-29028 (1996)).

The FXI molecules useful include the full length protein, precursors of the protein, subunits or fragments of the protein, and variants and antigenic fragments thereof. Reference to FXI is meant to include all potential forms of such proteins.

In some embodiments, the FXI polypeptide comprises SEQ ID NO:7, although allelic variants are possible. Factor XI, variants, fragments and/or methods of making thereof also useful are described in, *e.g.,* the following U.S. Patent Application Publications and U.S. Patents: 20120083522; 20110159006; 20110020349; 20100144620; 20100062512; 20080058266; 20070027077; 20050181978; 20030040480; and 5,252,217.

### Siglec ligands

In accordance with the embodiments, the conjugate comprises a Siglec ligand, wherein the Siglec ligand is a glycan selected from 9-N-biphenylcarboxyl-NeuAca2-6Gal-1-4GlcNAc (6'-BPCNeuAc), NeuAca2-6Gal-1-4GlcNAc and NeuAca2-6Gal-1-4(6-sulfo)GIcNAc and combinations thereof. Siglecs, short for sialic acid binding Ig-like lectins, are cell surface receptors and members of the immunoglobulin superfamily (lgSF) that recognize sugars. Their ability to recognize carbohydrates using an immunoglobulin domain places them in the group of I-type (Ig-type) lectins. They are transmembrane proteins that contain an N-terminal V-like immunoglobulin (IgV) domain that binds sialic acid and a variable number of C2-type Ig (IgC2) domains. The first described Siglec is sialoadhesin (Siglec-l/CD169) that is a lectin-like adhesion molecule on macrophages. Other Siglecs were later added to this family, including CD22 (Siglec-2) and Siglec-G/10 (i.e., human Siglec-10 and mouse Siglec-G), which is expressed on B cells and has an important role in regulating their adhesion and activation, CD33 (Siglec-3) and myelin-associated glycoprotein (MAG/Siglec-4). Several additional Siglecs (Siglecs 5-12) have been identified in humans that are highly similar in structure to CD33 so are collectively referred to as 'CD33-related Siglecs'. These Siglecs are expressed on human NK cells, B cells, and/or monocytes. CD33-related Siglecs all have two conserved immunoreceptor tyrosine-based inhibitory motif (ITIM)-like motifs in their cytoplasmic tails suggesting their involvement in cellular activation. Detailed descriptions of Siglecs is provided in the literature, *e.g.,* Crocker et al., Nat. Rev. Immunol. 7:255-66, 2007; Crocker et al., Immunol. 103:137-45, 2001; Angata et al., Mol. Diversity 10:555-566, 2006; and Hoffman et al., Nat. Immunol. 8:695-704, 2007.

Glycan ligands of Siglecs refer to compounds which specifically recognize one or more Siglecs and which comprise homo- or heteropolymers of monosaccharide residues. In addition to glycan sequences, the Siglec glycan ligands can also contain pegylated lipid moiety connected to the glycan via a linker. Examples of various Siglec glycan ligands are reported in the literature, *e.g.,* U.S. Pat. No. 8,357,671; and Blixt et al., J. Am. Chem. Soc. 130:6680-1 (2008).

In some embodiments, the Siglec ligand is a ligand for an inhibitory Siglec. In some embodiments, the Siglec ligand binds to a Siglec selected from Siglec-1 (CD169), Siglec-2 (CD22), Siglec-3 (CD33), Siglec-4 (MAG), Siglec-5, Siglec-6, Siglec-7, Siglec-8, Siglec-9, Siglec-G/10, Siglec-11, and Siglec-12. In some embodiments, the Siglec is expressed on the surface of a B lymphocyte. In some embodiments, the Siglec ligand is a Siglec-G/10 ligand.

In some embodiments, the Siglec ligands suitable include ligands for Siglec-2 (CD22), found on B lymphocyte cells. In some embodiments the ligand is a glycan ligand. The ligands can be natural or synthetic ligands that recognize CD22 (Siglec-2). CD22 from a number of species are known in the art. For example, amino acid sequences for human CD22 are disclosed in the National Center for Biotechnology Information (NCBI) database (http://www.ncbi.nlm.nih.gov/) at accession number NP 001762 (gi: 4502651) and also available in WO 2007/056525. Mouse CD22 is also characterized in the art, *e.g.,* Torres et al., J. Immunol. 149:2641-9, 1992; and Law et al., J Immunol. 155:3368-76, 1995. Other than CD22, Siglec-G/10 is another Siglec expressed on the surface of B cells. Human Siglec-10 and its mouse ortholog Siglec-G are both well known and characterized in the art. See, *e.g.,* Munday et al., Biochem. J. 355:489-497, 2001; Whitney et al., Eur. J. Biochem. 268:6083-96, 2001; Hoffman et al., Nat. Immunol. 8:695-704, 2007; and Liu et al., Trends Immunol. 30:557-61, 2009.

Various ligands of Siglecs are known. *See, e.g.,* U.S. Pat. No. 8,357,671; Chen et al., Blood 115:4778-86 (2010); Blixt et al., J. Am. Chern. Soc. 130:6680-1 (2008); Kumari et al., Virol. J. 4:42 (2007); and Kimura et al., J. Biol. Chem. 282:32200-7 (2007),

For example, natural ligands of human CD22 such as NeuAcα2-6Ga1β1-4GlcNAc, or NeuAcα2-6Galβ1-4(6-sulfb)GlcNAc can be used for targeting a coagulation factor protein to human B cells. In addition, a number of synthetic CD22 ligands with improved activities are also available, *e.g*., 9-N-biphenylcarboxyl-NeuAcα2-6Galβ1-4GlcNAc (6'-BPCNeuAc) and 9-N-biphenylcarboxyl-NeuAcα2-3Galβ1-4GlcNAc (3'-BPCNeuAc). More specific glycan ligands for human CD22 are described in the art, *e.g.,* Blixt et al., J. Am. Chern. Soc. 130:6680-1, 2008; and Paulson et al., WO 2007/056525. Similarly, many glycan ligands for mouse CD22 have been reported in the literature. Examples include NeuGcα2-6Galβ1-4GlcNAc (NeuGc), 9-N-biphenylacetyl-NeuGcα2-6Galβ1-4GlcNAc (^{BPA}NeuGc), and NeuGcα2-3Galβ1-4GlcNAc. Some of these CD22 ligands are also known to be able to bind to Siglec-G/10. Other than the natural and synthetic Siglec ligands exemplified herein, one can also employ derivative or analog compounds of any of these exemplified glycan ligands in the practice of the embodiments.

The term "analog" or "derivative" is used herein to refer to a molecule that resembles a known Siglec ligand in structure but which has been modified in a targeted and controlled manner, by replacing a specific substituent of the reference molecule with an alternate substituent. Compared to the reference molecule, an analog would be expected, by one skilled in the art, to exhibit the same, similar, or improved utility. Synthesis and screening of analogs to identify variants of known compounds having improved traits (such as higher binding affinity for a target 30 molecule) is an approach that is well known in pharmaceutical chemistry.

### Methods

The embodiments provide methods and therapeutic uses for suppressing undesired immune responses and/or inducing immune tolerance to coagulation factor proteins. The conjugates described herein can be used for treating or preventing various disorders which are associated with or mediated by an undesired immune response or immune activation. By targeting coagulation factor protein to B cells in a subject in need of treatment, the conjugates are suitable for inducing tolerance.

In one embodiment there is provided a method of inducing tolerance to a coagulation factor protein in a subject, comprising administering to the subject an effective amount of a conjugate comprising a coagulation factor protein or an antigenic fragment or variant thereof and a B cell Siglec ligand.

In some embodiments, a combination of conjugates can be administered to a subject, wherein the conjugates comprise a cocktail of coagulation factor proteins, antigenic fragments or variants thereof.

In one embodiment, the combination comprises a plurality of FVIII proteins, including, for example, a plurality of different commercially available FVIII products.

In another embodiment, the combination comprises a one or more different commercially available FVIII products and one or more BDD FVIII.

In another embodiment, the conjugates comprise one or more of FVII, FVIII, FIX, FX and FXI.

The route of administration of the conjugates does not exhibit particular limitations, and in one embodiment the conjugate can be administered by injection, such as intravenous, intramuscular, or intraperitoneal injection.

The methods of inducing tolerance against the coagulation factor proteins as described herein can be practiced before, during and/or after methods of treating bleeding disorders wherein an effective amount of the coagulation factor protein is administered as a biotherapeutic to treat the bleeding disorder. In some embodiments, the subject to be treated has a shortened *in vivo* half-life of FVIII, altered binding properties of FVIII, genetic defects of FVIII, and/or a reduced expression of FVIII. In one embodiment, the bleeding disorder is hemophilia.

The embodiments also provide methods of treating a bleeding disorder, such as hemophilia, comprising administering to a subject in need of treatment 1) an effective amount of a conjugate of the embodiments and 2) an effective amount of a coagulation factor. In some embodiments, the same coagulation factor used in step 2) is used to create the conjugate used in step 1), so that tolerance is created specifically against the coagulation factor being administered.

In one embodiment, the conjugate is administered to the patient before the coagulation factor protein to induce tolerance and prevent the generation of antibodies against the coagulation factor protein when it is administered.

In some embodiments, the conjugate is administered to the subject following the detection of antibodies against a coagulation factor protein in a subject undergoing replacement therapy. In some embodiments, the coagulation factor protein is subsequently administered after tolerance is achieved using the conjugate.

In some embodiments, the conjugate is administered about 30 days before the coagulation factor protein is administered. In some embodiments, the conjugate is administered about 25 days, 20 days, 15 days, 10 days, 9 days, 8 days, 7 days, 6 days, 5 days, 4 days, 3 days, 2 days, or one day before the coagulation factor protein is administered. In some embodiments, the conjugate is administered on the same day as the coagulation factor protein. In some embodiments, the conjugate is administered about 30 days, 25 days, 20 days, 15 days, 10 days, 9 days, 8 days, 7 days, 6 days, 5 days, 4 days, 3 days, 2 days, or one day after the coagulation factor protein is administered. The term "subject" refers to any animal classified as a mammal, e.g., human and non-human mammals. Examples of non-human animals include dogs, cats, cattle, horses, sheep, pigs, goats, rabbits, and etc. Unless otherwise noted, the terms "patient" or "subject" are used herein interchangeably. In some embodiments, the subject is human.

Subjects in need of treatment or inducing tolerance include those already suffering from the disease or disorder as well as those being at risk of developing the bleeding disorder. In some embodiments, the subject has demonstrated a positive antibody response against the coagulation factor protein biotherapeutic.

The conjugates described herein can be administered alone or as a component of pharmaceutical compositions. Pharmaceutical compositions of the embodiments comprise an effective amount of the conjugates formulated with at least one pharmaceutically acceptable carrier. Pharmaceutical compositions of the embodiments can be prepared and administered to a subject by any methods well known in the art of pharmacy. *See, e.g.,* Goodman & Gilman's The Pharmacological Bases of Therapeutics, Hardman et al., eds., McGraw-Hill Professional (10th ed., 2001); Remington: The Science and Practice of Pharmacy, Gennaro, ed., Lippincott Williams & Wilkins (20th ed., 2003); and Pharmaceutical Dosage Forms and Drug Delivery Systems, Ansel et al. (eds.), Lippincott Williams & Wilkins (7th ed., 1999). In addition, the pharmaceutical compositions of the embodiments may also be formulated to include other medically useful drugs or biological agents.

In some embodiments, the conjugates are used for *in vivo* applications. In these applications, the conjugates as set forth herein can be administered to a subject in need of treatment according to protocols already well-established in the art. The conjugates can be administered alone or in combination with a carrier in an appropriate pharmaceutical composition. Typically, a therapeutically effective amount of the conjugate is combined with a pharmaceutically acceptable carrier. The pharmaceutically acceptable carrier is any carrier known or established in the art. Exemplary pharmaceutically acceptable carriers include sterile pyrogen-free water and sterile pyrogen-free saline solution. Other forms of pharmaceutically acceptable carriers that can be utilized for the present embodiments include binders, disintegrants, surfactants, absorption accelerators, moisture retention agents, absorbers, lubricants, fillers, extenders, moisture imparting agents, preservatives, stabilizers, emulsifiers, solubilizing agents, salts which control osmotic pressure, diluting agents such as buffers and excipients usually used depending on the use form of the formulation. These are optionally selected and used depending on the

### unit dosage of the resulting formulation.

An effective amount of the conjugate varies depending upon the bleeding disorder that a subject is afflicted with, other known factors of the subject such as age, weight, etc., and thus must be determined empirically in each case. This empirical determination can be made by routine experimentation. In some embodiments, the liposome components may be used at a ratio of about 200:1 w/w, e.g., 100-300:1 w/w, compared to the antigen delivered. In some embodiments, a typical therapeutic dose of the liposome composition is about 5-100 mg per dose, e.g., 10 mg per dose.

For in vivo applications, the conjugates can be administered to the patient by any customary administration route, e.g., orally, parenterally or by inhalation. As shown in the Example below, a liposome co-displaying an antigen and a Siglec ligand can be administered to a subject by intravenous injection. In some other embodiments, the liposome complex can be administered to a subject intravascularly. A liposome useful for intravascular administration can be a small unilamellar liposome, or may be a liposome comprising PEG-2000. When the composition is parenterally administered, the form of the drug includes injectable agents (liquid agents, suspensions) used for intravenous injection, subcutaneous injection, intraperitoneal injection, intramuscular injection and intraperitoneal injection, liquid agents, suspensions, emulsions and dripping agents.

In some other embodiments, the conjugate is administered orally to a subject. In these embodiments, a form of the drug includes solid formulations such as tablets, coated tablets, powdered agents, granules, capsules and pills, liquid formulations such as liquid agents (e.g., eye drops, nose drops), suspension, emulsion and syrup, inhales such as aerosol agents, atomizers and nebulizers, and liposome inclusion agents. In still some other embodiments, the conjugate is administered by inhalation to the respiratory tract of a patient to target the trachea and/or the lung of a subject. In these embodiments, a commercially available nebulizer may be used to deliver a therapeutic dose of the liposome complex in the form of an aerosol.

The embodiments further provide for a pharmaceutical combination (e.g., a kit) for carrying out the methods of the present embodiments. In some embodiments, the kit comprises one or more conjugates of the present embodiments, including conjugates comprising FVII, FVIII, FVIX, FX, and FXI. In some embodiments, the kit further comprises reagents for the detection of subject antibodies against one or more of FVII, FVIII, FVIX, FX, and FXI, including control antibodies, antibody detection reagents, and purified antigens. In some embodiments, the kit comprises one or more biotherapeutics which can be administered to the subject, including FVII, FVIII, FVIX, FX, and FXI. In some embodiments, the conjugates are present in a pharmaceutical composition. In some embodiments, the kit further comprises instructions for administration of the agents and/or testing for the detection of antibodies in the subject. In some embodiments, the instructions in the kits generally contain information as to dosage, dosing schedule, and route of administration for the intended method of use. The containers of kits may be unit doses, bulk packages (e.g., multi-dose packages) or sub-unit doses. Instructions supplied in the kits of the embodiments are typically written instructions on a label or package insert (e.g., a paper sheet included in the kit), but machine-readable instructions (e.g., instructions carried on a magnetic or optical storage disk) are also acceptable.

In some embodiments, kits comprise materials for production of a conjugate comprising a specific coagulation factor polypeptide and a Siglec ligand. Generally, these kits contain separate containers of one or more antigens and one or more Siglec ligands from which a liposomal composition or immune conjugate can be made. Additional regents for making the compounds can also be provided in the kits, e.g., reagents for making liposome. The Siglec ligands and the antigens are in some embodiments supplied in a form which allows formation of complexes upon mixing of the other reagents with the supplied Siglec ligand and antigen.

While the embodiments have been described with reference to certain particular examples and embodiments herein, those skilled in the art will appreciate that various examples and embodiments can be combined for the purpose of complying with all relevant patent laws (e.g., methods described in specific examples can be used to describe particular aspects of the embodiments and its operation even though such are not explicitly set forth in reference thereto).

### EXAMPLE 1

### Toleragenic liposomes with Siglec ligands.

To investigate the possibility of recruiting CD22 to the immunological synapse on B cells to induce tolerance to T-dependent antigens, a versatile platform was needed. Liposomal nanoparticles were selected because of their validated in vivo use and the robust methods that exist for covalently linking proteins and glycan ligands to lipids for incorporation into the membrane (Chen, W. C. et al. Blood 115, 4778-4786 (2010); Loughrey et al. J Immunol Methods 132, 25-35 (1990); Shek et al. Immunology 50, 101-106 (1983)). Accordingly, liposomes were constructed that displayed either antigen alone (immunogen) or antigen and CD22 ligand (tolerogen; Fig. 1a). For initial studies high affinity siglec ligand was used, ^{BPA}NeuGc (^{BPA}NeuGca2-6Galβ1-4GlcNAc; Fig. 1b), which binds to murine CD22 with 200-fold higher affinity than its natural ligand, (NeuGcα2-6Galβ1-4GlcNAc; Fig. 1b), and has only a small degree of cross-reactivity with Siglec-G 15,19.

This platform was validated using the T-independent antigen nitrophenol (NP) in experiments analogous to earlier studies with the same antigen tethered to a polyacrylamide polymer. Mice injected with tolerogenic liposomes had a dramatic reduction in anti-NP response (both IgM and IgG isotypes) and failed to response to two subsequent challenges with immunogenic liposomes (Fig. 1c). In contrast, CD22KO mice treated with tolerogenic liposomes displayed no tolerization to NP upon a subsequent challenge; thus, tolerance to NP was induced in WT mice in a CD22-dependent manner.

Tolerogenic and immunogenic liposomes were next formulated displaying hen egg lysozyme (HEL) to investigate the potential to induce tolerance to a T-dependent antigen. Using the same experimental design, tolerogenic liposomes induced robust tolerance of C57BL/6J mice to HEL in a CD22-dependent manner (Fig. 2d). Tolerization experiments to HEL were repeated with liposomes formulated with varying amounts of either ^{BPA}NeuGc or NeuGc. At the end of the 44-day experiment, which involved two challenges with immunogenic liposomes on days 15 and 30, a dose-dependent effect on antibody production was apparent for both ligands (Fig. 2e). The two orders of magnitude difference in EC50 between the two ligands is consistent with their known affinities for CD2219. Full tolerization to HEL required two weeks to develop and was slowly lost over 4 months (Fig. 2f). The kinetics of loss in tolerance suggests that newly emerging B cells re-establish the anti-HEL response.

### EXAMPLE 2

### Tolerogenic liposomes induce apoptosis.

The mechanism of tolerance induction was next investigated using transgenic HEL-reactive (IgM^{HEL}) B cells from MD4 mice20. Liposomes displaying HEL and ^{BPA}NeuGc completely abrogated in vitro activation of IgM^{HEL} B cells compared to liposomes displaying HEL alone, as judged by calcium flux, CD86 upregulation, and proliferation (Fig. 2a-c). The use of IgM^{HEL} B cells on a CD22KO background revealed that in all three readouts of B cell activation, inhibition was fully CD22-dependent (Fig. 2a). Inhibition required presentation of both ligand and antigen on the same liposome since a mixture of liposomes displaying either ligand or antigen alone resulted in no inhibition (Fig. 2a). In proliferation assays (Fig. 2c), it was noticed that cells treated with the tolerogenic liposomes were decreasing in number relative to unstimulated cells. The percentage of live cells (AnnexinV-PI-) was analyzed, revealing that tolerogenic liposomes caused a significant decrease in the number of live cells in a time-dependent manner (Fig. 2d). Culturing cells with anti-CD40, to mimic T cell help, slowed down but did not prevent cell death. It is noteworthy that liposomes displaying the CD22 ligand alone did not reproduce the effects of the tolerogenic liposomes.

Next, similar experiments were conducted in vivo to examine the fate of IgMHEL B cells adoptively transferred into host mice following immunization with liposomes. Four days after immunization, IgMHEL B cells from mice immunized with tolerogenic liposomes had proliferated far less and were decreasing in number relative to the control (Fig. 2e). After 12 days, IgMHEL cells (Ly5a⁺IgMa⁺) were depleted by greater than 95% relative to mice that received naked liposomes (Fig. 2f). These in vivo effects were also CD22-dependent.

### EXAMPLE 3

### Impact of tolerogenic liposomes on BCR signaling

BCR signaling in IgMHEL B cells was analyzed by Western blotting at several time points after stimulation with liposomes (Fig. 3a). Tolerogenic liposomes gave rise to strong CD22 phosphorylation on all four ITIMs analyzed, which is consistent with physical tethering of CD22 and the BCR within the immunological synapse. Conversely, phosphorylation of numerous proximal (Syk and CD19) and distal (p38, Erk, JNK, Akt, GSK3β, FoxO1, FoxO3a, BIM) BCR signaling components were strongly inhibited by the tolerogenic liposomes compared to the immunogenic liposomes at both 3 and 30-minute time points. In striking contrast, equivalently strong phosphorylation of signaling components was observed with both the immunogenic and tolerogenic liposomes in IgMHEL cells lacking CD22.

Among the affected signaling components, it is particularly striking that tolerogenic liposomes induced hypo-phosphorylation of components in the Akt survival pathway compared to unstimulated (resting) B cells. Akt was hypo-phosphorylated at both the Thr308 and Ser473 sites while downstream targets of Akt, such as GSK3β and FoxO1/FoxO3a, were also hypo-phosphorylated. Given that Akt-mediated phosphorylation of the forkhead family of transcription controls their cellular location21, confocal microscopy was used to analyze cellular staining of both FoxO1 and FoxO3a (Fig. 3b). While nuclear staining of FoxO1 and FoxO3a was notably absent in resting IgMHEL B cells or cells activated with immunogenic liposomes, there was strong nuclear staining of cells treated with tolerogenic liposomes. As FoxO1 and FoxO3a regulate the transcription of genes involved in cell cycle inhibition and apoptosis in B cells21, these results are consistent with the induction of apoptosis by the tolerogenic liposomes.

### EXAMPLE 4

### Tolerance to strong T-dependent antigens

To investigate if tolerogenic liposomes can be used to induce tolerance to strong T-dependent antigens, several combinations of proteins were investigated and mouse strains known to provide strong T cell help. For tolerance studies in a more highly immunogenic system, the liposomal formulation was optimized to maximize CD22-mediated tolerance. This involved varying the amount of HEL on the liposome and titrating the amount of liposomes injected. Using optimized conditions, amounting to the use of 1000-fold less antigen in the initial tolerization step, robust tolerization to subsequent challenge with liposomal HEL was achieved in Balb/c mice (Fig. 4a). Notably, tolerization was also intact when soluble antigen was used in place of immunogenic liposomes during the challenge step (Fig. 4b). With optimized conditions in hand, tolerization to OVA, myelin oligodendrocyte glycoprotein (MOG), and FVIII was also achieved (Fig 4c-e). To assess the specificity of tolerization toward the intended antigen, the response of tolerized mice to a different antigen was investigated. Mice tolerized to HEL or OVA were found to have an unaltered response to the other antigen (Fig. 4f). Tolerization does not appear to involve induction of suppressor cells, since adoptively transferred splenocytes from a tolerized mouse do not suppress an antibody response to that antigen in host mice.

### EXAMPLE 5

### Bleeding protection in hemophelia mice.

Having established conditions to tolerize mice to human FVIII, this tolerizing approach in FVIII KO mice was applied, which served as a model of hemophelia A. FVIII KO mice that received immunogenic liposomes on day 0 and day 15 were unsuccessfully reconstituted with rhFVIII on day 30 since they bled to a similar extent in a tail cut experiment as FVIII KO mice that had not been reconstituted (Fig. 5a). On the other hand, mice that received tolerogenic liposomes followed by a challenge with immunogenic liposomes were protected from bleeding in the tail cut experiment to a level that was statistically indistinguishable from control mice that were reconstituted. The levels of anti-FVIII antibodies in the mice from this study correlated with the results from the bleeding assay; mice that were first treated with tolerogenic liposomes prior to a challenge with immunogenic liposomes did not produce a statistically significant increase in anti-FVIII antibodies relative to control mice (Fig. 5b). In contrast, mice that received the immunogenic liposomes twice had high levels of anti-FVIII antibodies. Thus, engaging CD22 to inhibit an antibody response is an effective means of suppressing inhibitory antibody formation against the biotherapeutic FVIII, which maintains the effectiveness of the reconstitution therapy.

### EXAMPLE 6

### A CD22-mediated tolerogenic circuit is operational in human naive and memory B cells.

To investigate if CD22 is capable of inducing a tolerogenic circuit in human B cells using our liposomal platform, a method was needed to stimulate B cells having different antigen-specificities. To accomplish this, anti-IgM or anti-IgG Fab fragments were linked to liposomes to act as surrogate antigens in order to stimulate naive or memory B cells, respectively. Furthermore, a different CD22 ligand was required since murine and human CD22 have different ligand preferences. Fortunately, a high affinity ligand of human CD22 has been developed, which is termed ^{BPC}NeuAc (^{BPA}NeuGcα2-6Galβ1-4GlcNAc; Fig. 6a). The anti-IgM and anti-IgG liposomes induced robust B cell activation of purified B cells isolated from peripheral human blood, as judged by calcium flux, in the naive (CD27⁻CD38^{low}) and memory (IgM⁻IgD⁻) B cell compartments, respectively (Fig. 6b). In contrast, the presence of human CD22 ligands on these liposomes strongly inhibited B cell activation. Similarly strong inhibition of BCR signaling was also observed in Western blot analyses (Fig. 6c) and CD86 upregulation (Fig. 6d). To determine tolerogenic also decrease the viability of primary human B cells, liposomes were incubated with cells for 24 hr and cell viability was analyzed by AnnexinV and PI staining. The number of live cells (AnnexinV-PI-) decreased in both naive and memory B cells incubated with anti-IgM and anti-IgG liposomes displaying ^{BPC}NeuAc, respectively, even in the presence of anti-CD40 (Fig. 6e). The more profound effect observed in memory B cells is in line with the stronger inhibition observed in the other readouts of B cell activation (Fig. 6b-d) and is particularly intriguing since the memory cells express moderately lower levels of CD22 than naive B cells (Fig. 6f).

### EXAMPLE 7

### The following materials and methods were used in conducting the experiments described in Examples 1-6.

Animal Studies: The Institutional Animal Care and Use Committee of The Scripps Research Institute (TSRI) approved all experimental procedures involving mice. CD22KO and Siglec-GKO mice, on a C57BL/6J background, were obtained from L. Nitchke (University of Erlangen) and Y. Liu (University of Michigan), respectively. Double knockout (CD22KO/Siglec-GKO; DKO) mice were previous bred in our laboratory. WT MD4 transgenic mice20 that express IgMHEL (C57BL/6J background) were obtained from Jackson laboratories. MD4 mice were crossed to the siglec KO strains (CD22KO, Siglec-GKO, and CD22KO/Siglec-GKO) and, subsequently, with C57BL/6J Ly5a mice. Mice expressing mHEL (KLK4)43 on a C57BL/6J background were obtained from C. Xiao (The Scripps Research Institute) and crossed to ST6Gall-deficient mice44. Mice expressing mOVA45 on a C56BL/6J background were obtained from Jackson laboratories. FVIII-deficient mice on a BalbC background were a generous gift of David Lillicrap (Queens University). WT C57BL/6J and Balb/c mice were obtained from the TSRI rodent breeding colony.

Isolation of Human B cells: The procedures involving human subjects were reviewed and approved by TSRI Institutional Review Board. Normal blood was obtained from TSRI's Normal Blood Donor Service. To isolate peripheral blood mononuclear cells (PBMCs) from heperanized blood, it was first diluted 2-fold with HBSS. The diluted blood (35 mL) was layered on top of 15 mL of ficoll-paque plus (GE healthcare) and centrifuged for 40 min. at 400 rcf. The buffy coat was isolated and diluted 4-fold with HBSS and spun (10 min, 300 rcf). B cells were purified by negative selection (Miltenyi) and were typically 99% pure (CD19⁺). For Western blot analysis of BCR signaling components, the purified B cells were additionally sorted for either naive (CD3⁻CD27⁻) or isotype-switch memory (CD3⁻IgM⁻IgD⁻) B cells.
Immunization and Blood Collection: Whole blood (50 µL) was collected from mice via a retro-orbital bleed using heparinized capillary tubes (Fisher). Blood was centrifuged (17,000 rcf, 1 min) to collect the serum. Serum was either used immediately for ELISAs or stored at -20 °C. One freeze thaw cycle was found to have a minimal affect on antibody titer determination. Liposomes and cells were delivered via the lateral tail vein in a volume of 200 µL. For studies involving a challenge with soluble (non-liposomal) antigen, mice were injected with 200 µg of HEL dissolved in HBSS and delivered intraperitoneally or 1 µg of FVIII delivered intravenously.

Bleeding assays in FVIII-deficient mice: Mice were reconstituted with 200 µL of recombinant human FVIII (rhFVIII; Kogenate, Bayer Healtcare) or saline one hour prior to tail cut. rhFVIII was dissolved according to manufacturer's instructions, diluted in sterile saline solution, and dosed at 50 U/Kg using a retroorbital intravenous injection. Following one hour, mice were anesthetized and the distal portion of the tail was cut at 1.5 mm diameter and immersed in a predefined volume of saline for 20 min. During this step, the solution of saline was maintained at 37 °C. Hemoglobin concentration in the saline solution was determined after red cell lysis with 2% acetic acid and quantified by absorbance at 405 nm. Hemoglobin concentration against a known standard was used to calculate blood loss per gram mouse weight and expressed in µL/g, assuming a hematocrit of 46% for a normal mouse. Blood loss in WT Balb/c mice injected with 200 µL saline served as a control. Mice were considered protected if blood loss was below the mean blood loss plus three standard deviations observed in WT Balb/c mice.

Flow Cytometry: Two color flow cytometry was carried out on a FACS Calibur flow cytometer (BD). When three or more colors were used, an LSRII flow cytometer (BD) was used. Labeled antibodies for flow cytometery were obtained from Biolegend. In all cases, dead cells were gated out with 1 µg/mL of propidium iodide.

B cell Purification: B cells were purified by negative selection using magnetic beads according to the manufacture's protocol (Miltenyi). The purity of isolated cells was generally ≥ 99%.

Fluorescent Labeling of B cells: Purified IgMHEL B cells (10x10⁶ cells/ml) were fluorescently-labeled with either CFSE (6 µM) or CTV (1.5 µM) (Invitrogen) in HBSS for 7 minutes at RT with mixing every 2 minutes. Reactions were quenched by the addition of HBSS containing 3% FBS and centrifuged (270 rcf, 7 min). Cells were resuspended in the appropriate buffer and centrifuged again, after which the cells were resuspended at the appropriate concentration in the assay buffer.

In Vitro B Cell Assays: Purified IgMHEL B cells were incubated for 1hr in media (RPMI, 3% FCS, Pen/Srep) prior to beginning the assay. Cells (0.2x106) were plated in U-bottom 96-well culture plates (Falcon). Liposomes (5 µM lipid final concentration) were added and cells were incubated at 37 °C for various lengths of time. To analyze the cells by flow cytometry, cells were first centrifuged (270 rcf, 7 min) followed by incubation with the appropriate antibodies in 50 µL of FACS buffer (HBSS containing 0.1% BSA and 2 mM EDTA). After 30-60 min of staining on ice, cells were washed once with 220 µL of FACS buffer and finally resuspended in FACS buffer containing 1 µg/mL propidium iodide prior to analyzing by flow cytometry. One exception to this protocol was AnnexinV staining, which was carried out in buffer supplied by the manufacturer (Biolegend).

In Vivo B cell Proliferation Assays: CFSE-labeled IgMHEL cells were resuspended at a concentration of 10x10⁶ cells/mL in HBSS and 200 µL (2x106 cells) were injected into host mice via the tail vein. The following day (24 hr), liposomes were injected via the tail vein. Four days later, the spleens of the host mice were harvested to analyze the CFSE staining of Ly5a+IgMa+ B cells. To analyze the number of IgMHEL B cells left in the host mouse 12 days after immunization with liposomes, IgMHEL B cells were not CFSE-labeled.

Calcium Flux: Purified B cells were resuspended at 15x10⁶ cells/mL in RPMI media containing 1% FCS, 10 mM HEPES, 1 mM MgCl₂, 1 mM EGTA, and 1 µM Indo-1 (Invitrogen). Cells were incubated in a 37 °C water incubator for 30 minutes. Following this incubation period, a five-fold volume of the same buffer (without Indo-1) was added and the cells were centrifuged (270 rcf, 7 min). For experiments involving human B cells, cells were stained for 20 min on ice in HBSS containing 3% FCS. To investigate human naive B cells, the cells were stained with CD3 and CD27 to gate out any contaminating T cells as well as the memory B cells. To investigate human memory B cells, cells were stained with CD3, IgM, and IgD to gate out any contaminating T cells as well as the naive B cells. Cells were washed, spun, and resuspended at a concentration of 2x10⁶ cells/mL in HBSS containing 1% FCS, 1 mM MgCl₂, and 1 mM CaCl₂. Cells were stored on ice and an aliquot (0.5 mL; 1x10⁶ cells) was warmed to 37 °C for 5 minutes prior to measuring calcium flux. Cells were stimulated with liposomes (ranging from 5-50 µM) and Indo-1 fluorescence was monitored by flow cytometry (500-1000 events/sec) for 3-6 minutes. Stimulation always took place after acquiring 10 sec. to establish the background. During the assay, a water jacket was used to keep the tube at 37 °C. Data was analyzed in FlowJo using the kinetics functions and data is plotted as the mean intensity with Gaussian smoothing.

ELISAs: Maxisorp plates (96-well; Thermo Fisher) were coated with 50 µL/well of the relevant protein at a concentration of between 10-100 µg/mL in PBS and left overnight at 4 °C. To look at anti-NP antibodies, NP4-7-BSA in PBS (Biosearch Technologies) was used. The following day, the plates were washed twice in TBS-T (Tris-buffered saline blocked containing 0.1% Tween 20) and blocked for 1 hr at RT with 100 µL of assay diluent (TBS-T with 1% BSA). Washing of the plates was accomplished by submerging the entire plate into a basin of wash buffer the appropriate number of times. Serum was initially diluted between 20-10,000-fold and diluted in 2-3 fold serial dilutions eight times on the ELISA plate. Plates were incubated with serum (50 µL/well) for 1 hr at 37°C after which the plates were washed four times in TBS-T. HRP-conjugated secondary antibodies (Santa Cruz Biotechnologies) were diluted 2000-fold in assay diluent and 50 µL was added to each well. After incubation for 1 hr at 37°C, the plates were washed five times in TBS-T. To develop the plate, 75 µL/well of TMB substrate (Thermo Fisher) was added. The plate was incubated at RT for 15 minutes and then 75 µL/well of 2N H₂SO₄ was added to quench the reaction. Plates were read at 450 nm using a spectrophotometer (Molecular Devices). The titer was defined as the endpoint titer, which was the dilution of serum that produced an absorbance 2-fold above background.

Western Blotting: Purified IgMHEL B cells (30x106/condition) were incubated in media (RPMI, 3% FCS, Pen/Strep) at 37 °C for 1 hr prior to stimulating the cells. Liposomes (5 µM lipid final concentration) were added to cells and after a 3 or 30 minute incubation at 37 °C, cells were briefly centrifuged (13,000 rcf, 8 sec), washed in 1 mL of cold PBS, centrifuged a second time, and lysed in 280 µL of lysis buffer (20 mM Tris, 150 NaCl, 1 mM EDTA, 1% Triton-X 100, 10 mM NaF, 2 mM Sodium orthovanadate, protease inhibitor cocktail (Roche), pH 7.5) on ice for 30 min. Cell debris was removed by centrifugation (13,000 rcf, 5 min, 4 °C) and the protein concentration of cell lysates were standardized by BCA assay (Pierce). SDS-PAGE loading buffer containing 100 mM DTT was added to lysates and samples were heated at 75 °C for 15 min. Samples were run on 4-12% gradient SDS-PAGE gels (Invitrogen) at 150 V for 60-90 min and transferred to nitrocellulose (30 V, 2 hr). Membranes were blocked at RT for 1 hr in 5% nonfat milk powder dissolved in TBS-T (0.1% Tween-20) and probed with primary antibody overnight at 4 °C in TBS-T containing 1% BSA. Primary antibodies were obtained from Cellular Signaling Technologies and used at dilution of 1:1000. Phosphospecific CD22 antibodies were a gift from M. Fujimoto (University of Tokyo). The following day, membranes were washed 4x5min followed by 30 min blocking with TBS-T containing 1% BSA. Membranes were incubated for 1 hr at RT with secondary HRP-conjugated antibodies (1:10,000 dilution; Santa Cruz Biotechnologies) dissolved in TBS-T + 1% BSA. Following four washes, the blots were incubated with developing solution (GE Healthcare) for 2 minutes and exposed to film. Microscopy: Purified IgMHEL B cells were incubated in media (RPMI, 3% FCS, Pen/Strep) at 37 °C for 1 hr prior to stimulating the cells. Cells were stimulated in the same manner as the Western blot analysis except that stimulation took place for 2 hr. Following stimulation with liposomes, cells were gently pelleted (0.5 rcf, 3 min), washed in 1 mL of cold PBS, and again gently centrifuged. To fix the cells, the pellet was resuspended in 1 mL of cold 4% paraformaldehyde (PFA) and rotated at 4 °C for 10 min. Cells were gently centrifuged and the pellet was resuspended in 200 µL of PBS and 50 µL of the resuspended cells (approximately 3x106 cells) were dispersed onto polylysine slides (Fisher). After drying of the solution, the slides were washed a further three times with PBS to remove excess PFA. Cells were permeabilized with 5% Triton-X 100 for 5 min at RT followed by blocking with 5% normal goat serum (NGS) for 30 min at RT. Slides were probed with anti-FoxO1 or anti-FoxO3a (Cellular Signaling Technologies) at a concentration of 1:80 in solution of 1% NGS containing 0.01% TX-100 overnight at 4 °C. The following day, the slides were wash three times with PBS and probed with Alexa488-conjugated goat anti-rabbit (1:1000 dilution; Invitrogen) along with Alex555-conjugated phalloidin (1:40 dilution; Invitrogen) in 1% NGS. Following three washes with PBS, slides were briefly incubated with a solution of DAPI and mounted in Prolong anti-fade medium (Invitrogen). Imaging of the cells was carried out on a Zeiss confocal microscope.

Protein-Lipid Conjugation: Proteins were conjugated to pegylated distearoylphosethanolamine (PEG-DSPE) using maleimide chemistry in a similar procedure as described by others (Loughrey, H. C., Choi, L. S., Cullis, P. R. & Bally, M. B. Optimized Procedures for the Coupling of Proteins to Liposomes. J Immunol Methods 132, 25-35 (1990)). First, a thiol group was introduced onto the protein using the heterobifunctional crosslinker N-succinimidyl 3-(2-pyridyldithio)-propionate (SPDP; Pierce), which modifies lysine residues. Approximately 5 molar equivalents of SPDP (freshly dissolved in DMSO) were added to a protein solution (in PBS) in the range of 1-20 mg/mL. The reaction was gently rocked at RT for 1 hr and then centrifuged to remove any precipitate. To remove unreacted SPDP, the protein was desalted on a sephadex G-50 column. The desalted protein was treated with 25 mM DTT (10 min, RT) to deprotect the 2-pyridyl disulphide group and thereby generate a free thiol. The amount of thiol 2-pyridyl leaving group released during the reaction was determined by measuring the absorbance at 343 nm (7550 M-1cm-1 extinction coefficient), which could be used to calculate the extent of modification of the protein with the linker by comparing it to the concentration of the protein. The protein was again desalted on a sephadex G-50 to remove excess DTT. The thiol-derivatized protein (in the range of 10-50 µM) was immediately reacted with Maleimide-PEG2000-DSPE (200 µM; NOF America) under nitrogen at RT overnight. Lipid-modified proteins were micelles and could be easily purified from unmodified protein on a sephadex G-100 column. The desired lipid-modified protein eluted in the void volume and the protein concentration was determined by an A280 measurement and then stored at 4 °C. To validate that the proteins were modified by lipid, SDS-PAGE was used. Lipid-modification of the proteins was readily apparent by an increase in their apparent MW on the gel (Figure S2c). Using these reaction conditions, proteins were modified with between one to three lipids.

Sugar-Lipid Conjugation: The high affinity murine CD22 ligand (^{BPA}NeuGc) and human CD22 ligand (^{BPC}NeuAc) were attached to PEG-DSPE by coupling 9-N-biphenylacetyl-NeuGcα2-6Galβ1-4GlcNAc-β-ethylamine or 9-N-biphenylcarboxyl-NeuAcα2-6Galβ1-4GlcNAc-β-ethylamine to NHS-PEG₂₀₀₀-DSPE (NOF), respectively, as described previously (Chen et al., Blood 115:4778-4786 (2010)).

Synthesis of NP-PEG₂₀₀₀-DSPE: 4-Hydroxy-3-nitrophenylacetyl-O-succinimide (1.5 mg, 0.0050 mmol, 2.8 eq) and amine-PEG₂₀₀₀-DSPE (5.0 mg, 0.0018 mmol; NOF) were dissolved in 0.5 ml of dry dichloromethane containing 10 molar equivalents N,N-Diisopropylethylamine (3.1 µL, 0.018 mmol, 10 eq). After three hours at RT, the solvent was evaporated *in vacuo* and the remaining solid residue was resuspended in ddH₂O (2 mL) with the help of sonication. The suspension was dialyzed three consecutive times against ddH2O using dialysis cassettes with a molecular weight cutoff of 10 kDa (Pierce). The dialyzed sample was then lyophilized in a tarred vial to give a fluffy light yellow powder. 1H NMR spectroscopy in DMSO confirmed the expected ratio (1:2) of aromatic protons from the nitrophenol group and the terminating methyl groups of the stearoyl lipids, respectively.

Liposomes: All liposomes were composed of a 60:35:5 molar ratio of distearoyl phosphatidylcholine (DSPC; Avanti Polar Lipids), cholesterol (Sigma), and pegylated lipids. The total mol% of pegylated lipids was always kept at 5%; this 5% was made up of the appropriate combination of polyethyleneglycol(PEG₂₀₀₀)-distearoyl phosphoethanolamine (PEG-DSPE; Avanti Polar Lipids), ^{BPA}NeuGc-PEG₂₀₀₀-DSPE, ^{BPC}NeuAc-PEG₂₀₀₀-DSPE, NP-PEG₂₀₀₀-DSPE or Protein-PEG₂₀₀₀-DSPE. To assemble the liposomes, the appropriate amount of freshly dissolved DSPC and cholesterol were evaporated under a stream of nitrogen gas. An aliquot of ^{BPA}NeuGc-PEG₂₀₀₀-DSPE, ^{BPC}NeuAc-PEG₂₀₀₀-DSPE, NP-PEG₂₀₀₀-DSPE, from DMSO stocks, were added to the dried lipid and this mixture was lyophilized. The dried lipids were hydrated in PBS and sonicated vigorously for a minimum of five times 30 s with several minutes delay between rounds of sonication. Protein-PEG₂₀₀₀-DSPE was added at the time of hydration. The mol% of the protein on the liposome was varied during our studies from 0.0033-0.33%. The total concentration of the liposomes is defined by the molarity of the lipids and liposomes were typically hydrated in the range of 1-10 mM. Liposomes were passed a minimum of 20 times through 800 nm, 100 nm, and finally 100 nm filters using a hand-held mini-extrusion device (Avanti Polar Lipids). Extrusion was carried out between 40-45 C. The diameter of the liposomes were measured on a zetasizer (Malvern) and generally found to be in the range of 100-130 ± 30 nm. Incorporation of Protein-PEG₂₀₀₀-DSPE into the liposomes did not influence their size.

Cloning, Expression, and Purification of MOG: Residues 1-120 of rat myelin oligodendrocyte glycoprotein were cloned from a rat brain cDNA library (Zyagen) using the following primers: 5'-GCAGCACATATGGGACAGTTCATAGTGATAGGG-3' (SEQ ID NO:11) and 5'-GCAGACCTCGAGGTAGAAGGGATCTTCTACTTTC-3' (SEQ ID NO:12), where the underlined letters represent the NdeI and XhoI restriction sites, respectively. The PCR product was ligated into pET23a to express a protein with a C-terminal His6-tag. Protein expression and purification was carried out as described previously (Chan, J. W. et al. Monitoring dynamic protein expression in living E-coli. Bacterial Celts by laser tweezers raman spectroscopy. Cytom Part A 71A, 468-474 (2007)).

Statistical Analyses: Statistical significance was determined using an unpaired two-tailed Student's t-test.

### EXAMPLE 8

### Generation of Factor VIII-PEG conjugates

STRUCTURE ACTIVITY RELATIONSHIP ANALYSIS OF FVIII. FVIII and BDD FVIII are very large complex molecules with many different sites involved in biological reactions. Previous attempts to covalently modify them to improve pharmacokinetic properties had mixed results. That the molecules could be specifically mutated and then a polymer added in a site-specific manner was surprising. Furthermore, the results of improved pharmacokinetic properties and retained activity were surprising also, given the problems with past polymeric conjugates causing nonspecific addition and reduced activity.

In one embodiment, there is provided site-directed mutagenesis using cysteine-specific ligands such as PEG-maleimide. A non-mutated BDD does not have any available cysteines to react with a PEG-maleimide, so only the mutated cysteine position will be the site of PEGylation. More specifically, BDD FVIII has 19 cysteines, 16 of which form disulfides and the other 3 of which are free cysteines (McMullen et al., 1995, Protein Sci. 4, pp. 740-746). The structural model of BDD suggests that all 3 free cysteines are buried (Stoliova-McPhie et al., 2002, Blood 99, pp. 1215-1223). Because oxidized cysteines cannot be PEGylated by PEG-maleimides, the 16 cysteines that form disulfides in BDD cannot be PEGylated without being first reduced. Based on the structural models of BDD, the 3 free cysteines in BDD may not be PEGylated without first denaturing the protein to expose these cysteines to the PEG reagent. Thus, it does not appear feasible to achieve specific PEGylation of BDD by PEGylation at native cysteine residues without dramatically altering the BDD structure, which will most likely destroy its function.

The redox state of the 4 cysteines in the B domain of full-length FVIII is unknown. PEGylation of the 4 cysteines in the B domain may be possible if they do not form disulfides and are surface exposed. However, because full-length FVIII and BDD have a similar pharmacokinetic (PK) profile and similar half-lives in vivo (Gruppo et al., 2003, Haemophilia 9, pp. 251-260), B domain PEGylation is unlikely to result in improved plasma half-life unless the PEG happens to also protect non-B domain regions.

To determine the predefined site on a polypeptide having FVIII activity for polymer attachment that will retain factor VIII activity and improve pharmacokinetics, the following guidelines are presented based on BDD FVIII. Modifications should be targeted toward clearance, inactivation, and immunogenic mechanisms such as LRP, HSPG, APC, and inhibitory antibody binding sites. Stoilova-McPhie, S. et al., 2002, Blood 99(4), pp. 1215-23 shows the structure of BDD. For example, to prolong half-life, a single PEG can be introduced at a specific site at or near LRP binding sites in A2 residues 484-509 and A3 residues 1811-1818. Introduction of the bulky PEG at these sites should disrupt FVIII's ability to bind LRP and reduce the clearance of FVIII from circulation. It is also believed that to prolong half-life without significantly affecting activity that a PEG can be introduced at residue 1648, which is at the junction of the B domain and the A3 domain in the full-length molecule and in the 14-amino acid liker I the BDD between the A2 and A3 domains.

Specificity of PEGylation can be achieved by engineering single cysteine residues into the A2 or A3 domains using recombinant DNA mutagenesis techniques followed by site-specific PEGylation of the introduced cysteine with a cysteine-specific PEG reagent such as PEG-maleimide. Another advantage of PEGylating at 484-509 and 1811-1818 is that these two epitopes represent two of the three major classes of inhibitory antigenic sites in patients. To achieve maximal effect of improved circulating half-life and reduction of immunogenic response, both A2 and A3 LRP binding sites can be PEGylated to yield a diPEGylated product. It should be noted that PEGylation within the 1811-1818 region may lead to significant loss of activity since this region is also involved in FIX binding. Site-directed PEGylation within 558-565 should abolish HSPG binding, but may also reduce activity as this region also binds to FIX.

Additional surface sites can be PEGylated to identify novel clearance mechanism of FVIII. PEGylation of the A2 domain may offer additional advantage in that the A2 domain dissociates from FVIII upon activation and is presumably removed from circulation faster than the rest of FVIII molecule because of its smaller size. PEGylated A2, on the other hand, may be big enough to escape kidney clearance and have a comparable plasma half-life to the rest of FVIII and thus can reconstitute the activated FVIII in vivo.

IDENTIFICATION OF PEGylation SITES IN A2 AND A3 REGIONS. Five positions (Y487, L491, K496, L504 and Q468 corresponding to PEG1-5 positions) at or near the putative A2 LRP binding region were selected as examples for site-directed PEGylation based on the high surface exposure and outward direction of their Cα to Cβ trajectory. Furthermore, these residues are roughly equidistant from each other in the three-dimensional structure of the molecule, so that together they can represent this entire region. Eight positions (1808, 1810, 1812, 1813, 1815, 1795, 1796, 1803, 1804 corresponding to PEG6-14) at or near the putative A3 LRP binding region were selected as examples for site-directed PEGylation. PEG6 (K1808) is adjacent to 1811-1818 and the natural N-linked glycosylation site at 1810. PEGylation at position 1810 (PEG7) will replace the sugar with a PEG. Mutation at the PEG8 position T1812 will also abolish the glycosylation site. Although the PEG9 position (K1813) was predicted to be pointing inward, it was selected in case the structure model is not correct. PEG10 (Y1815) is a bulky hydrophobic amino acid within the LRP binding loop, and may be a critical interacting residue since hydrophobic amino acids are typically found at the center of protein-protein interactions. Because the 1811-1818 region has been reported to be involved in both LRP and FIX binding, PEGylation within this loop was thought possibly to result in reduced activity. Thus, PEG11-PEG14 (1795, 1796, 1803, 1804) were designed to be near the 1811-1818 loop but not within the loop so that one can dissociate LRP and FIX binding with different PEG sizes.

To block both LRP binding sites simultaneously, double PEGylation at, for example, the PEG2 and PEG6 position, can be generated.

Since the 558-565 region has been shown to bind to both HSPG and FIX, no sites were designed within this region. Instead, PEG15-PEG17 (377, 378, and 556) were designed in between the A2 LRP and HSPG binding regions so that an attached PEG may interfere both interactions and disrupt possible interactions between them. Additional sites that are surface exposed and outwardly pointing could also be selected within or near the LRP and HPSG binding regions. To identify novel clearance mechanisms, FVIII can be systematically PEGylated. In addition to PEG1-17, the three other natural glycosylation sites, namely, N41, N239, and N2118 corresponding to PEG18-20 can be used as tethering points for PEGylation since they should be surface exposed. Surface areas within a 20 angstrom radius from the Cβ atoms of PEG2, PEG6, and the four glycosylation sites were mapped onto the BDD model in addition to functional interaction sites for vWF, FIX, FX, phospholipid, and thrombin.

PEG21-29 corresponding to Y81, F129, K422, K523, K570, N1864, T1911, Q2091, and Q2284 were then selected based on their ability to cover nearly the entire remaining BDD surface with a 20 angstrom radius from each of their Cβ atoms. These positions were also selected because they are fully exposed, outwardly pointing, and far away from natural cysteines to minimize possible incorrect disulfide formation. The 20 angstrom radius is chosen because a large PEG, such as a 64 kD branched PEG, is expected to have the potential to cover a sphere with about a 20 angstrom radius. PEGylation of PEG21-29 together with PEG2 and PEG6 and glycosylation sites PEG18, 19, and 20 is likely to protect nearly the entire non-functional surface of FVIII.

PEGylation positions that lead to enhanced properties such as improved PK profile, greater stability, or reduced immunogenicity can be combined to generate multi-PEGylated product with maximally enhanced properties. PEG30 and PEG31 were designed by removing the exposed disulfides in A2 and A3 domain, respectively. PEG30, or C630A, should free up its disulfide partner C711 for PEGylation. Likewise, PEG31, C1899A should allow C1903 to be PEGylated.

MUTAGENESIS. Substrates for site-directed PEGylation of FVIII may be generated by introducing a cysteine codon at the site chosen for PEGylation. The Stratagene cQuickChange II site-directed mutagenesis kit was used to make all of the PEG mutants (Stratagene kit 200523 from Stratagene Corporation, La Jolla, Calif.). The cQuikChange.TM. site-directed mutagenesis method is performed using Pfu Turbo.RTM. DNA polymerase and a temperature cycler. Two complimentary oligonucleotide primers, containing the desired mutation, are elongated using Pfu Turbo, which will not displace the primers. dsDNA containing the wildtype FVIII gene is used as a template. Following multiple elongation cycles, the product is digested with DpnI endonuclease, which is specific for methylated DNA. The newly synthesized DNA, containing the mutation, is not methylated, whereas the parental wild-type DNA is methylated. The digested DNA is then used to transform XL-1 Blue super-competent cells.

The mutagenesis efficiency is almost 80%. The mutagenesis reactions were performed in either pSK207+BDD C2.6 or pSK207+BDD. Successful mutagenesis was confirmed by DNA sequencing and appropriate fragments, containing the mutation, were transferred into the FVIII backbone in the mammalian expression vector pSS207+BDD. After transfer, all of the mutations were again sequence-confirmed. For A3 muteins PEG 6, 7, 8, 9, and 10, mutagenesis was done in the vector pSK207+BDD C2.6. After being confirmed by sequencing, the mutant fragment, Kpnl/Pme was subcloned into pSK207+BDD. The BDD mutein was then subcloned into the pSS207+BDD expression vector. For A3 muteins PEG 11, 12, 13, 14, the mutagenesis was done directly in the vector pSK207+BDD and sequence-confirmed mutant BDD were then subcloned into pSS207+BDD. For A2 muteins PEG 1, 2, 3, 4, 5, the mutagenesis was done in the pSK207+BDD C2.6vector. The sequence confirmed mutant was subcloned into pSK207+BDD and then to pSS207+BDD.

The Primers (Sense Stand Only) Used for Mutagenesis are Listed for Each Reaction
PEG1, Y487C: GATGTCCGTCCTTTGTGCTCAAGGAGATTACCA (SEQ ID NO:13)
PEG2, L491C: TTGTATTCAAGGAGATGCCCAAAAGGTGTAAAAC (SEQ ID NO:14)
PEG3, K496C: TTACCAAAAGGTGTATGCCATTTGAAGGATTTTC (SEQ ID NO:15)
PEG4, L504C: AAGGATTTTCCAATTTGCCCAGGAGAAATATTC (SEQ ID NO:16)
PEG5, Q468C: GATTATATTTAAGAATTGCGCAAGCAGACCATAT (SEQ ID NO:17)
PEG6, K1808C: TAGAAAAAACTTTGTCTGCCCTAATGAAACCAAAAC (SEQ ID NO:18)
PEG7, N1810C: AACTTTGTCAAGCCTTGCGAAACCAAAACTTAC (SEQ ID NO:19)
PEG8, T1812C: GTCAAGCCTAATGAATGCAAAACTTACTTTTGGA (SEQ ID NO:20)
PEG9, K1813C: CAAGCCTAATGAAACCTGCACTTACTTTTGGAAAG (SEQ ID NO:21)
PEG10, Y1815C: CTAATGAAACCAAAACTTGCTTTTGGAAAGTGCAAC (SEQ ID NO:22)
PEG11, D1795C: ATTTCTTATGAGGAATGCCAGAGGCAAGGAGCA (SEQ ID NO:23)
PEG12, Q1796C: TCTTATGAGGAAGATTGCAGGCAAGGAGCAGAA (SEQ ID NO:24)
PEG13, R1803C: CAAGGAGCAGAACCTTGCAAAAACTTTGTCAAGCCT (SEQ ID NO:25)
PEG14, K1804C: GGAGCAGAACCTAGATGCAACTTTGTCAAGCCT (SEQ ID NO:26)
PEG15, K377C: CGCTCAGTTGCCAAGTGTCATCCTAAAACTTGG (SEQ ID NO:27)
PEG16, H378C: TCAGTTGCCAAGAAGTGTCCTAAAACTTGGGTA (SEQ ID NO:28)
PEG17, K556C: CTCCTCATCTGCTACTGCGAATCTGTAGATCAA (SEQ ID NO:29)
PEG18, N41C: CAAAATCTTTTCCATTCTGCACCTCAGTCGTGTAC (SEQ ID NO:30)
PEG19, N239C: GTCAATGGTTATGTATGCAGGTCTCTGCCAGGT (SEQ ID NO:31)
PEG20, N2118C: CAGACTTATCGAGGATGTTCCACTGGAACCTTA (SEQ ID NO:32)
PEG21, Y81C: ATCCAGGCTGAGGTTTGTGATACAGTGGTCATT (SEQ ID NO:33)
PEG22, F129C: GAAGATGATAAAGTCTGTCCTGGTGGAAGCCAT (SEQ ID NO:34)
PEG23, K422C: CAGCGGATTGGTAGGTGTTACAAAAAAGTCCGA (SEQ ID NO:35)
PEG24, K523C: GAAGATGGGCCAACTTGCTCAGATCCTCGGTGC (SEQ ID NO:36)
PEG25, K570C: CAGATAATGTCAGACTGCAGGAATGTCATCCTG (SEQ ID NO:37)
PEG26, N1864C: CACACTAACACACTGTGTCCTGCTCATGGGAGA (SEQ ID NO:38)
PEG27, T1911C, CAGATGGAAGATCCCTGCTTTAAAGAGAATTAT (SEQ ID NO:39)
PEG28, Q2091C: ACCCAGGGTGCCCGTTGCAAGTTCTCCAGCCTC (SEQ ID NO:40)
PEG29, Q2284C: AAAGTAAAGGTTTTTTGCGGAAATCAAGACTCC (SEQ ID NO:41)
PEG30, C630A: TTGCAGTTGTCAGTTGCTTTGCATGAGGTGGCA (SEQ ID NO:42)
PEG31, C1899A: AATATGGAAAGAAACGCTAGGGCTCCCTGCAAT (SEQ ID NO:43)

MUTEIN EXPRESSION. After insertion in a vector that confers resistance to Hygromycin B, the PEG muteins were transfected into HKB11 cells (U.S. Pat. No. 6,136,599) complexed with 293 Fectin Transfection Reagent (Invitrogen Corp. Cat#12347-019) per the manufacturer's instructions. FVIII expression at three days post-transfection was assessed by Coatest chromogenic assay (Chromogenix Corp. Cat#821033, see Example 12 Chromogenic Assay). The transfected cells were then placed under selective pressure with 50 .quadrature.g/ml of Hyg B in a growth medium supplemented with 5% FBS. When Hyg B-resistant colonies appeared, they were manually picked and screened for FVIII expression by Coatest chromogenic assay. The FVIII expressing stable cells were then adapted to a medium containing HPPS supplement. The cells were expanded and seeded at IX 10⁶ cells/ml in shaking flasks with fresh media. Tissue culture fluid (TCF), harvested after 3 days, was used for purification of FVIII BDD muteins. The FVIII activity of the TCF was assayed by Coatest.

MUTEIN PURIFICATION. Upon collecting the cell culture supernatant containing the secreted mutein FVIII protein, the supernatant is filtered through a 0.2 micron membrane filter to remove any remaining cells. The supernatant is then concentrated by either ultrafiltration or anion exchange. It is then applied to an immunoaffinity column where the cell culture media components and the majority of the host cell protein impurities are removed. The immunoaffinity column eluate is then buffer exchanged by diafiltration into a formulation buffer containing sucrose and frozen. Yield and recovery of protein across a monoclonal FVIII antibody column was assessed by chromogenic assay. Samples of load, flow through, various eluate fractions, strip, and the diafiltered eluate of a chromatography run were assayed for FVIII activity.

PEGYLATION. Native full-length FVIII or BDD cannot be PEGylated by cysteine-specific PEGs without reduction and denaturation at over 100-fold excess PEG: protein ratio (data not shown), confirming the hypothesis based on the BDD structure model that all native cysteines form disulfides or are buried within FVIII. FVIII cysteine muteins expressed and purified using the standard protocols listed above could not be PEGylated with a cysteine-specific PEG maleimide reagent, presumably because the introduced FVIII cysteine is "capped" by reacting with sulfhydryl groups such as cysteine and β-mecaptoethanol present in the cell growth media. This issue can potentially be resolved by eliminating cysteines and β-mecaptoethanol from the culture media, but this may lead to lower FVIII production and would not prevent sulfhydryls released by the cells from blocking the introduced FVIII cysteine.

In another aspect, a three-step method was developed to allow site-specific PEGylation of FVIII. In step 1, the purified FVIII cysteine mutein at about 1 µM is mildly reduced with reductants such as about 0.7 mM Tris(2-carboxyethyl)phosphine (TCEP) or 0.07 mM dithiothreitol (DTT) for 30 minutes at 4°C. to release the "cap." In step 2, the reductant is removed along with the "cap" by a size-exclusion chromatography (SEC) method such as running the sample through a spin column (BioRad) to allow FVIII disulfides to reform while leaving the introduced cysteine free and reduced. In step 3, at least 30 minutes after the removal of the reductant, the freed FVIII cysteine mutein is treated with at least 10-fold molar excess of PEG-maleimide with sizes ranging from 5 to 64 kD (Nektar Therapeutics and N.O.F. Corporation) for at least 1 hour at 4°C. This method yields highly consistent product profile with reproducible data for dozens of reactions repeated by different individuals.

Because the spin column method for removal of TCEP is not scaleable, gel filtration desalting chromatography was selected. However, upon testing this method using a TCEP spike sample, it was shown that the TCEP eluted at measurable levels in the column void and not just in the salt fraction as would be expected from a molecule with its low molecular weight. Western Blot assays showed significant background PEGylation probably due to incomplete removal of TCEP. In the meantime separate experiments showed that C7F7 purified material could be significantly purified further from other protein impurities using an anion exchange chromatography media combined with a salt gradient. It was then decided to reduce the C7F7 material with TCEP as described above and then process the material over the anion exchange column. Because of charge difference the FVIII protein would be retained while the TCEP would flow through the column and not be retained. At the same time during the gradient salt elution the FVIII protein would be purified away from the majority of remaining protein impurities. This meant that the later occurring PEGylation would be theoretically more homogeneous with purer starting material. However, upon testing with a spike sample of TCEP, it was shown that measurable levels of TCEP were found eluting in the gradient with the FVIII. Therefore it was decided to implement gel filtration desalting chromatography after anion exchange chromatography so these two steps when used in sequence would result in complete removal of TCEP and elimination of non-specific PEGylation.

PEGYLATION ANALYSIS BY SDS PAGE AND WESTERN BLOT. The PEGylated product can be analyzed by electrophoresis on a reducing 6% TrisGlycine SDS polyacrylamide gel (Invitrogen). Following electrophoresis, the gel can be stained with Coomassie Blue to identify all the proteins or subjected to a standard Western Blot protocol to identify PEGylation pattern on different regions of FVIII. Staining of the blot with a mouse monoclonal R8B12 or C7F7 antibody raised against the C-terminal region of the FVIII heavy chain or the N-terminal region of the VIII light chain, respectively, should identify PEGylation of the respective chains. Staining with the 413 antibody against the 484-509 region of FVIII will determine whether PEGylation is indeed site-specific or not for muteins such as PEG 1-4. Likewise, staining with the CLB-CAg A antibody that recognizes the 1801-1823 region of FVIII will determine if PEGylation is site-specific or not for muteins such as PEG6-10.

PEG2 (L491C) PEGylation was shown to be selective for the heavy chain over light chain and particularly selective for the 484-509 region while PEG6 (K1808C) was shown to be selective for the light chain over the heavy chain.

PEGYLATION ANALYSIS BY THROMBIN CLEAVAGE AND WESTERN BLOT. The PEGylated product can be treated with thrombin (40 IU/ug FVIII) at 37°C. for 30 minutes. The thrombin used also contains APC as a contaminant. Thrombin cleavage will generate the 50 kD A1 and 43 kD A2 domains from the heavy chain while the APC cleavage will split the A2 domain further into the 21 and 22 kD fragments. Staining with the R8B12 antibody, which recognizes the C-terminus of the heavy chain, will identify only the intact A2 domain and the 21 kD C-terminal fragment (FVIII 562-740). Thus, if PEG2 PEGylation was specific for position 491, the 43 kD A2 domain should be PEGylated but not the 21 kD C-terminal fragment. This was indeed confirmed by the Western blot for the 22 kD PEGylated PEG2. Thus, by elimination, PEG2 PEGylation has been localized to the N-terminal 22 kD fragment (FVIII 373-561) of A2 domain. Since PEG-maleimide is completely selective for cysteines at pH 6.8 and the only native FVIII cysteines within 373-561 come from a buried disulfide between 528 and 554, PEG2 is very likely PEGylated on the introduced cysteine at position 491. Western staining of thrombin-treated PEGylated PEG2 with a FVIII heavy chain N-terminal antibody showed no PEGylation of the A1 domain (data not shown). Selective PEGylation of PEG2 using thrombin cleavage method has also been confirmed for PEGs of 5, 12, 33, and 43 kDs (data not shown). Thrombin cleavage of PEGylated wildtype full-length FVIII shows that only B domain is PEGylated.

PEGYLATION ANALYSIS BY IODINE STAINING. To confirm that the newly created bands on Coomassie Blue and Western staining were indeed PEGylated bands, barium-iodine staining, which is specific for PEG, was used. PEGylated PEG2 was run on a 6% TrisGlycine gel (Invitrogen) and stained with the R8B12 heavy chain antibody or a barium-iodine solution (Lee et al, Pharm Dev Technol. 1999 4:269-275). The PEGylated bands matched between the two stains using the molecular weight marker to line them up, thus confirming FVIII heavy chain PEGylation.

PEGYLATION ANALYSIS BY MALDI-MASS SPEC. To confirm the PEGylation of the A2 domain in the heavy chain, the rFVIII sample, before and after PEGylation was analyzed by matrix-assisted laser desorption/ionization (MALDI) mass spectrometry. The samples were mixed and crystallized on the MALDI target plate with a sinapinic acid matrix in 30% acetonitrile, 0.1% TFA. They were then analyzed in a Voyager DE-PRO spectrometer in positive, linear mode. The results showed the light chain of PEG2 centered at 83 kD and the heavy chain (HC) at 89 kD. The spectrum acquired for the PEGylated sample showed a drop in the HC peak and a new peak, centered at 111 kD, to form. This confirms PEGylation of the heavy chain. No PEGylated light chain (at 105 kD) was observed above detection limit.

The samples were then both subjected to thrombin digestion at 20 units of thrombin/mg FVIII at 37°C for 30 minutes, following FVIII concentration determination by amino acid analysis (Commonwealth Biotechnologies, Inc). The heavy chain was cleaved into a 46 kD (A1) N-terminal fraction and a 43 kD (A2) fraction. The MALDI spectrum acquired for the PEGylated sample shows the loss of the 43 kD peak and the development of a new 65 kD peak, due to the PEGylated A2 domain. PEGylation of the LC is again not observed above the detection limit. These results again confirm PEGylation of the A2 domain of FVIII. The same analysis was applied to PEGylated PEG6, confirming PEGylation of the light chain A3C1C2 fragment.

### Activity Measurement

COAGULATION ASSAY. The clotting FVIII:C test method is a one-stage assay based upon the activated partial thromboplastin time (aPTT). FVIII acts as a cofactor in the presence of Factor IXa, calcium, and phospholipid in the enzymatic conversion of Factor X to Xa. In this assay, the diluted test samples are incubated at 37°C. with a mixture of FVIII deficient plasma substrate and aPTT reagent. Calcium chloride is added to the incubated mixture and clotting is initiated. An inverse relationship exists between the time (seconds) it takes for a clot to form and logarithm of the concentration of FVIII:C. Activity levels for unknown samples are interpolated by comparing the clotting times of various dilutions of test material with a curve constructed from a series of dilutions of standard material of known activity and are reported in International Units per mL (IU/mL).

CHROMOGENIC ASSAY. The chromogenic assay method consists of two consecutive steps where the intensity of color is proportional to the FVIII activity. In the first step, Factor X is activated to FXa by FIXa with its cofactor, FVIIIa, in the presence of optimal amounts of calcium ions and phospholipids. Excess amounts of Factor X are present such that the rate of activation of Factor X is solely dependent on the amount of FVIII. In the second step, Factor Xa hydrolyzes the chromogenic substrate to yield a chromophore and the color intensity is read photometrically at 405 nm. Potency of an unknown is calculated and the validity of the assay is checked with the slope-ratio statistical method. Activity is reported in International Units per mL (IU/mL).

The 1811-1818 loop is involved in binding to FIX, but the importance of individual positions within this loop has not been determined. PEG7-10 muteins display nearly identical specific chromogenic activity relative to native FVIII.

TOTAL ANTIGEN ELISA (TAE). FVIII is captured on a microtiter plate that has been coated with a polyclonal FVIII antibody. The FVIII bound is detected with a biotinylated polyclonal rFVIII antibody and streptavidin horseradish peroxidase (HRP) conjugate. The peroxidase-streptavidin complex produces a color reaction upon addition of the tetramethylbenzidine (TMB) substrate. Sample concentrations are interpolated from a standard curve using four parameter fit models.

vWF BINDING ELISA. FVIII is allowed to bind to vWf in Severe Hemophilic Plasma in solution. The FVIII-vWf complex is then captured on a microtiter plate that has been coated with a vWf-specific monoclonal antibody. The FVIII bound to the vWf is detected with a FVIII polyclonal antibody and a horseradish peroxidase-anti-rabbit conjugate. The peroxidase-conjugated antibody complex produces a color reaction upon addition of the substrate. Sample concentrations are interpolated from a standard curve using four parameter fit model. FVIII binding results are reported in µg/mL. There was no significant impact on any of the activities upon PEGylation, which would be consistent with PEGylation at the B domain.

PURIFICATION OF PEGylated FVIII BY ION-EXCHANGE CHROMATOGRAPHY. PEGylated FVIII is applied to an anion exchange column or cation exchange column where the protein binds to the column while any excess free PEG reagent does not bind and is removed in the flow through. The PEG mutein is then eluted from the column with a sodium chloride gradient. A barium-iodine stained 4-12% Bis-Tris gel of load, flow through, and gradient fractions was used to confirm that the column elution fractions have PEGylated mutein.

PURIFICATION OF PEGylated FVIII BY SIZE-EXCLUSION CHROMATOGRAPHY. The anion exchange fractions containing the majority of PEG2 mutein are pooled and concentrated by ultrafiltration then applied to a size exclusion column. The column is then eluted using the formulation buffer. Because of the difference in the size and shape of the protein depends on whether PEG is bound to the protein, this column separates the PEGylated PEG2 mutein from that of any remaining PEG2, which is not PEGylated. The PEGylated mutein FVIII fractions are pooled based on having the most FVIII activity then frozen for subsequent animal studies and molecular characterization.

With muteins such as PEG6 that show lower efficiencies of PEGylation, i.e. less than 50%, the most effective purification scheme to yield highly pure mono-PEGylated product is to use a combination of cation exchange chromatography followed by size exclusion chromatography. For example, with PEG6, the cation exchange chromatography purifies the PEGylated PEG6 (earlier eluting fraction) away from the majority of un-PEGylated PEG6 (later eluting fraction). The size exclusion chromatography then polishes the PEGylated protein (earlier eluting fraction) from the remainder of un-PEGylated protein (later eluting fraction).

EFFECT OF PEG SIZE ON ACTIVITY. To test whether PEG sizes have an effect on both coagulation and chromogenic activities of FVIII upon PEGylation, purified full-length FVIII, PEG2, PEG6, and PEG14 were reduced by TCEP followed by reductant removal and reaction with a buffer control or PEGs ranging from 6 kD to 64 kD. The resulting PEGylated FVIII was directly assayed without removal of excess PEG or unPEGylated FVIII. Control experiments showed that the excess PEG has no effect on FVIII activity.

PEGylation within the A2 or A3 domain at PEG2, PEG6, or PEG14 position of BDD led to dramatic losses of coagulation activity when PEG size increases beyond 6 kD. However, PEGylation within the B domain at a native B-domain cysteine of the full-length FVIII had no effect on the coagulation activity. Interestingly, the chromogenic activity is not affected for all PEGylated constructs. This may be due to assay differences. It is possible that the small chromogenic peptide substrate has an easier access to a PEGylated FVIII/FIX/FX complex than the larger protein substrate used in the coagulation assay. Alternatively, PEG may affect activation of the mutein. This would be more readily detected by the one-stage coagulation assay than the two-stage chromogenic assay.

To confirm the observation of PEG effects on the coagulation activity of PEG2, 6, and 14, several PEGylated contructs were purified away from excess PEG and unPEGylated. Since PEG does not have any effect on the chromogenic activity, the chromogenic to coagulation activity ratio is a good estimate on the relative effect of PEG on coagulation activity. Larger PEGs at a given position such as PEG2 and a higher number of PEGs as in the case with the PEG2+6 construct induce a greater loss of coagulation activity.

RABBIT PK STUDY. To understand the effects of PEGylation on the pharmacokinetics (PK) of FVIII, PK studies were performed in a number of species. NZW SPF rabbits were used for the study: 10 females, 5 rabbits per group, 2 groups (PEG2 FVIII and 22 kD PEGylated PEG2). Samples were diluted into sterile PBS with a final concentration of 100 IU/mL (chromogenic units). Each rabbit received a dose of 1 ml/kg (100 IU/kg) of the diluted test or control substance via marginal ear vein. At various times post-injection, blood samples (1 mL) were drawn into a 1 mL syringe (charged with 100 µL of 3.8% Na-Citrate) from the central ear artery at defined time points after dosing. Plasma samples were incubated with R8B12 heavy chain antibody coated on a 96-well plate to specifically capture the dosed human FVIII. The activity of the captured FVIII was determined by the chromogenic assay. PEGylated PEG2 and PEGylated PEG6 were also compared with BDD, with PEGylated muteins showing an improvement in plasma recovery compared to BDD. PEGylated wildtype full-length FVIII did not appear to show much improvement.

MOUSE PK STUDY. As a second species, ICR normal or hemophilic, FVIII deficient, mice (Taconic, Hudson, N.Y.) were used in PK studies. Normal mice were used for the study, 5 mice per group per time point. Test materials were diluted into formulation buffer to a nominal final concentration of 25 IU/mL. Each mouse can be administered 4 mL/kg (about 0.1 mL total volume) of the dilute test material via tail vein. Blood samples (0.45 or 0.3 mL for normal or hemophilic mouse study, respectively) are drawn into a 1 mL syringe (charged with 50 or 30 .mu.L of 3.8% Na-Citrate for normal or hemophilic mouse study, respectively) from the inferior vena cava at the indicated time point (one animal per sample). Plasma samples are assayed for FVIII concentration using the chromogenic assay method described above. PEGylated PEG6 shows greater plasma recovery compared to BDD or PEG6. PEGylated PEG2 shows greater plasma recovery compared to BDD.

HEMOPHILIC MOUSE (BDD) FACTOR VIII RECOVERY. The Hemophilic Mouse (BDD) Factor VIII recovery histogram depicts a pharmacokinetic (PK) assessment of the half-life of two species of BDD Factor VIII in a hemophilic mouse assay. This assay was designed to measure plasma concentrations of both BDD Factor VIII and the PEG 2+6 double PEGylated variant of BDD Factor VIII (and identified elsewhere herein as the L491C, K1808C double variant of BDD Factor VIII) at three time points post intravenous administration in a mouse model. While the PK assessments at both the 0.8 and 4 hour time points were comparable, the 16 hour assessment is particularly noteworthy. At 16 hours, approximately four times (400%) as much of the doubly PEGylated BDD Factor VIII variant (PEG 2+6) remained in the mouse plasma 16 hours after administration as compared to the un-PEGylated molecule.

KIDNEY LACERATION MODEL. To determine if PEGylated FVIII muteins were efficacious at stopping a bleed in a hemophilic mouse, the kidney laceration model was employed. Hemophilic mice (C57/BL6 with a disrupted FVIII gene) are anesthetized under isofluorane and weighed. The inferior vena cava was exposed and 100 ul of either saline or FVIII were injected using a 31 gauge needle. The needle was carefully removed and pressure applied at the sight of injection for 30-45 seconds to prevent bleeding. After two minutes, the right kidney was exposed and held between the forceps along the vertical axis. Using a #15 scalpel, the kidney was cut horizontally to a depth of 3 mm. To insure a uniform depth of the lesion, kidney was lightly held in the middle to expose equal tissue on either side of the forceps. The exposed surface of the kidney was cut to the depth of the forceps. Blood loss was quantified as described above. Different doses of FVIII were tested on mice to characterize the dose response relationship of FVIII on kidney bleeding. PEGylated PEG2 shows comparable potency to BDD in reducing blood loss after mouse kidney injury. Thus, although the coagulation activity of PEGylated PEG2 is lower than that of BDD, this kidney laceration model shows that the in vivo efficacy of PEGylated PEG2 was not measurably reduced compared to BDD, consistent with the chromogenic assay data.

ANTIBODY INHIBITION ASSAY. Adding a high molecular weight polymer such as polyethylene glycol (PEG) specifically at position 491 (i.e. PEG2) should reduce binding and sensitivity to mAB 413, and by extension to a large proportion of patient inhibitory antibodies since many patients develop inhibitor antibodies against the same mAB 413 epitope. To test this, increasing amounts of mAB 413 was incubated with non-saturating amounts (0.003 IU/mL) of BDD or 43 kD PEGylated PEG2 and tested for functional activity in a chromogenic assay. R8B12, a non-inhibitory antibody, and ESH4, an inhibitory antibody that targets the C2 domain were used as controls. PEGylated PEG2 is indeed more resistant to mAB 413 inhibition than BDD and shows a similar inhibition pattern in the presence of the control antibodies that do not bind near the 491 position. Furthermore, the protection effect of PEG against mAB 413 inhibition is dependent on PEG size, with larger PEGs having a greater effect. To test whether PEGylated FVIII is more resistant to inhibitor antibodies from patients, chromogenic activity was measured in the presence of a panel of plasma derived from hemophilia A patients who have developed inhibitors to FVIII. Of the 8 patient plasma tested, 43 kD PEGylated PEG2 was more resistant to patient plasma inhibition than BDD in 4 patient plasma samples. For example, PEGylated PEG2, PEG6, or PEG2+6 showed greater residual activity than BDD in one patient plasma but not in another plasma. The diPEGylated PEG2+6 appears to be more resistant than monoPEGylated PEG2 or PEG6. These results suggest that PEGylated PEG muteins can be more effective in treating patients that develop inhibitors to FVIII.

HIGH THROUGHPUT PEGYLATION SCREENING. PEGylation efficiency of a particular PEG mutein is unpredictable, especially since there is no direct structural information of BDD. For example, based on the structure model of BDD, one would predict the PEGylation efficiency of PEG4 and PEG5 should be very high, similar to that of PEG2 and PEG15 since all three positions are surface exposed and point outwardly according to the structure. Thus, to use PEG to search for novel clearance mechanism via systematic PEGylation will require a large number of muteins to be screened.

To rapidly screen a large number of PEG muteins, a novel high throughput method has been developed that can test PEGylation efficiency and functional activity of PEGylated products from transiently transfected muteins. As little as 5-10 mL of transiently expressed PEG muteins with an FVIII chromogenic value of as low as 0.1-0.2 IU/mL is concentrated by about 50-fold using Amicon-centra Ultra device MWCO 30K so that the concentration of FVIII reaches above 1 nM, near the affinity range of antibody to FVIII interaction. The concentrated PEG mutein (about 300 uL) is incubated with .about.30 uL of C7F7 FVIII antibody resin overnight at 4°C., washed, eluted, dialyzed, and reduced. The reductant is removed and the reduced PEG muteins is PEGylated and run on a Western analysis as described above. Relative PEGylation efficiency of transiently expressed PEG muteins matches exactly to that of purified PEG muteins.

Dozens of PEG muteins can be screened by this method in one to two months. For example, PEG14 (K1804C BDD) had at least about 80% PEGylation of light chain with a 12 kD PEG and no PEGylation of heavy chain (data not shown), consistent with the K1804C mutation located on the light chain. The C.quadrature. to C.quadrature. distance between K1804 and K1808 (PEG6 position) is only 8.4 angstrom based on the BDD structure, suggesting that the introduction of a 43 kD PEG at this position will have similar improvement in PK as the 33 kD PEGylated PEG6, with the advantage of much higher PEGylation yield. PEGylation was highly selective for the particular FVIII chain where the cysteine mutation was introduced, in that every mutein with the cysteine in the heavy chain only gets PEGylated on the heavy chain while every mutein with the cysteine in the light chain gets PEGylated on the light chain. Mutein numbers 2 to 31 represent cysteine mutations of BDD replacing the native amino acid at the position listed with a cysteine. PEG2+6 is a double mutein of BDD where position 491 and 1808 were substituted with cysteines. A1 and A2, (and B domain for KG-2, the full-length FVIII) belong to the heavy chain while A3, C1, and C2 belong to the light chain. PEGylation efficiency was estimated from running the PEGylated products on a SDS PAGE comparing the intensities of the PEGylated band with unPEGylated band: +++ about >80% PEGylation yield, ++ about 30-70% yield, + about 10-30% yield, and about <10% yield.

MASS SPECTROMETRY ANALYSIS OF REDUCED PEG MUTEINS. To determine the identity of the "cap" that prevents direct PEGylation of PEG muteins or full-length FVIII, PEG2+14 was reduced with TCEP at concentrations ranging from 67 uM to 670 uM. PEGylation yield increased in proportion to increasing amounts of TCEP. The same samples were also analyzed by mass spectrometry prior to PEGylation. In order to have a protein domain that could be directly studied, the samples were digested with thrombin at a ratio of 20 units/mg FVIII for 30 minutes at 37°C. Thrombin cleavage produces an A2 fragment that includes residues 372 to 740 and no occupied glycosylation sites. The digested sample was injected onto a C4 reversed phase liquid chromatography system and the eluent from the column was introduced directly into the quadrupole time-of-flight mass spectrometer via an electrospray interface. The mass spectrum from under the chromatographic peak corresponding to the A2 domain was deconvoluted to provide a protein intact mass value. Prior to reduction, the A2 domain of PEG2+14 yields a mass that is 118 daltons larger than theoretically predicted. As the TCEP concentration is increased, a new peak that has the precise predicted mass of A2 domain appears. The proportion of this new peak increases as the TCEP concentration is increased. The 118 dalton difference can be accounted for by cysteinylation at residue Cys 491 via disulfide formulation with a cystine (119 Da) and instrumental accuracy. Thus this shows that the PEG muteins are capped by a cysteine, which prevents direct PEGylation.

### EXAMPLE 9

### Protocol to quantify the number of ligands bound to each coagulation factor molecule. Quantification of sialic acid content using TAKARA DMB labeling kit

Sialic Acid Fluorescence Labeling Kit (Cat#4400) is for quantitative and highly sensitive analysis of sialoglycoconjugates. This HPLC-based sialic acid fluorescence labeling technique using 1,2-diamino-4,5-methyleneoxybenzene (DMB) is a simple and highly sensitive quantitative method. In this method, free sialic acids are analyzed by reverse phase HPLC (GlycosepR, from Glyko, # 1-4727) after labeling by DMB.

### Experiment procedure:

### 1. DMB labeling:

Pipette out 5∼50µg sample into an Eppendorf tube, speed vacuum dry down, then add 500ul 2M Acetic Acid to the tube, 80*C heat blocker for 2hr. After reaction over, use speed vacuum to dry down the acid treated sample.

Make DMB reagents; each reaction tube needs 200ul DMB

| | |
|---|---|
| 1 part reagent B | 80ul |
| 5 part reagent A | 400ul |
| 4 part water | 320ul |

Reactions set up as followed:

| | | Acetic acid | DMB reagent |
|---|---|---|---|
| Sample | | 10ul | 190ul |
| Blank | | 10ul | 190ul |
| Standard (100uM) | 10ul | 10ul | 180ul |

| | | | |
|---|---|---|---|
| (Note: Make 2M acetic acid: 114 ul in HPLC water to final 1 ml) Mix well and 50*C heat block for 2.5 hour | | | |

Stop the reaction with adding equal volume ice-cold HPLC water (i.e. 200ul H₂O), terminate the reaction by place the Eppendorf tube on ice. Run the HPLC at same day.

### 2. HPLC analysis: isocratic

Column: GlycoSepR from Glyko, Cat# 1-4727
Solvent: Acentoitrile/methanol/water=9/7/84
Flow rate: 1ml/minute
Detection: FLD: Ex 310nm, Em 448nm

The peak of DMB tagged sialic acid usually appear @6∼7minutes. To quantify the sialic acid, the peak area of sample was compared with the peak area of the standard sialic acid.

The section headings used herein are for organizational purposes.

## Claims

1. A conjugate for inducing tolerance of a coagulation factor protein, wherein the conjugate comprises a coagulation factor protein or an antigenic fragment or variant thereof and a B cell Siglec ligand,
wherein the Siglec ligand is a glycan selected from 9-N-biphenylcarboxyl-NeuAca2-6Gal∼1-4GlcNAc (6'-BPCNeuAc), NeuAca2-6Gal∼1-4GlcNAc and NeuAca2-6Gal∼1-4(6-sulfo)GlcNAc and combinations thereof.

2. The conjugate of claim 1, wherein the coagulation factor protein is conjugated directly to a Siglec ligand, or
wherein the coagulation factor protein is conjugated indirectly to a Siglec ligand.

3. The conjugate of any of claims 1-2, wherein the conjugate comprises a liposome.

4. The conjugate of any of claims 1-3, wherein the distance separating the coagulation factor protein and the Siglec ligand of the conjugate enables efficient presentation to a B cell resulting in enforced ligation and juxtaposition of the Siglec and B cell receptor in an immunological synapse.

5. The conjugate of any of claims 1-4, wherein the coagulation factor protein is selected from the group consisting of Factor VII, Factor VIII, Factor IX, Factor X, and Factor XI and combinations thereof.

6. A pharmaceutical composition comprising an effective amount of the conjugate according to any of claims 1-5.

7. An effective amount of a conjugate according to any of claims 1-5 for use in the treatment of a bleeding disorder.

8. The effective amount of a conjugate for use according to claim 7, wherein said use comprises inducing tolerance to a coagulation factor protein.

9. The effective amount of a conjugate for use according to claims 7 or 8, wherein said use is in the presence of antibodies against the coagulation factor protein.

10. A kit comprising the conjugate of claims 1-5.

11. The conjugate of claim 1, wherein the coagulation factor protein is FVIII and a biocompatible polymer is covalently attached to one or more FVIII amino acid positions 81, 129, 377, 378, 468, 487, 491, 504, 556, 570, 711, 1648, 1795, 1796, 1803, 1804, 1808, 1810, 1864, 1903, 1911, 2091, 2118 and 2284.

12. The conjugate of claim 1, wherein the coagulation factor protein is FVIII and a biocompatible polymer is covalently attached to one or more FVIII amino acid positions 377, 378, 468, 491, 504, 556, 1795, 1796, 1803, 1804, 1808, 1810, 1864, 1903, 1911 and 2284.

13. The conjugate of claim 1, wherein the coagulation factor protein is FVIII and a biocompatible polymer is covalently attached to one or more FVIII amino acid positions 377, 378, 468, 491, 504, 556 and 711.

14. The conjugate of claim 1, wherein the coagulation factor protein is FVIII and a biocompatible polymer is covalently attached to one or more FVIII amino acid positions 81, 129, 377, 378, 468, 487, 491, 504, 556, 570, 711, 1648, 1795, 1796, 1803, 1804, 1808, 1810, 1864, 1903, 1911, 2091, 2118 and 2284.

15. The conjugate of any of claim 1, wherein the coagulation factor protein is B-domain deleted factor VIII,
wherein preferably a biocompatible polymer is covalently attached to B-domain deleted FVIII at amino acid position 129, 491, 1804, and/or 1808.

16. The conjugate of claim 1, wherein the coagulation factor protein is full length FVIII or B-domain deleted FVIII and a biocompatible polymer is attached to FVIII amino acid position 1804 and comprises polyethylene glycol.

17. An effective amount of a conjugate of any of claims 1-6 or 13-19 and an effective amount of a coagulation factor for use in the treatment of a bleeding disorder.

18. The conjugate according to any of claims 11-16, wherein the amino acid position is mutated to cysteine.

## Patentansprüche

1. Konjugat zum Induzieren von Toleranz eines Koagulationsfaktorproteins, wobei das Konjugat ein Koagulationsfaktorprotein oder ein antigenes Fragment oder eine antigene Variante davon sowie einen B-Zell-Siglec-Liganden umfasst,
wobei es sich bei dem Siglec-Liganden um ein Glycan, ausgewählt aus 9-N-Biphenylcarboxyl-NeuAca2-6Gal∼1-4GlcNAc (6'-BPCNeuAc), NeuAca2-6Gal∼1-4GlcNAc und NeuAca2-6Gal∼1-4(6-sulfo)GlcNAc und Kombinationen davon, handelt.

2. Konjugat nach Anspruch 1, wobei das Koagulationsfaktorprotein direkt an einen Siglec-Liganden konjugiert ist, oder
wobei das Koagulationsfaktorprotein indirekt an einen Siglec-Liganden konjugiert ist.

3. Konjugat nach einem der Ansprüche 1-2, wobei das Konjugat ein Liposom umfasst.

4. Konjugat nach einem der Ansprüche 1-3, wobei der Abstand, der das Koagulationsfaktorprotein und den Siglec-Liganden des Konjugats trennt, eine wirksame Präsentation gegenüber einer B-Zelle ermöglicht, die zu einer zwangsmäßigen Ligation und Nebeneinanderstellung des Siglec und des B-Zell-Rezeptors in einer immunologischen Synapse führt.

5. Konjugat nach einem der Ansprüche 1-4, wobei das Koagulationsfaktorprotein aus der Gruppe bestehend aus Faktor VII, Faktor VIII, Faktor IX, Faktor X und Faktor XI und Kombinationen davon ausgewählt ist.

6. Pharmazeutische Zusammensetzung, umfassend eine wirksame Menge des Konjugats nach einem der Ansprüche 1-5.

7. Wirksame Menge eines Konjugats nach einem der Ansprüche 1-5 zur Verwendung in der Behandlung einer Blutungsstörung.

8. Wirksame Menge eines Konjugats zur Verwendung nach Anspruch 7, wobei die Verwendung das Induzieren von Toleranz gegenüber einem Koagulationsfaktorprotein umfasst.

9. Wirksame Menge eines Konjugats zur Verwendung nach Anspruch 7 oder 8, wobei die Verwendung in Gegenwart von Antikörpern gegen das Koagulationsfaktorprotein erfolgt.

10. Kit, umfassend das Konjugat nach den Ansprüchen 1-5.

11. Konjugat nach Anspruch 1, wobei es sich bei dem Koagulationsfaktorprotein um FVIII handelt und ein biologisch kompatibles Polymer kovalent an eine oder mehrere FVIII-Aminosäurepositionen 81, 129, 377, 378, 468, 487, 491, 504, 556, 570, 711, 1648, 1795, 1796, 1803, 1804, 1808, 1810, 1864, 1903, 1911, 2091, 2118 und 2284 gebunden ist.

12. Konjugat nach Anspruch 1, wobei es sich bei dem Koagulationsfaktorprotein um FVIII handelt und ein biologisch kompatibles Polymer kovalent an eine oder mehrere FVIII-Aminosäurepositionen 377, 378, 468, 491, 504, 556, 1795, 1796, 1803, 1804, 1808, 1810, 1864, 1903, 1911 und 2284 gebunden ist.

13. Konjugat nach Anspruch 1, wobei es sich bei dem Koagulationsfaktorprotein um FVIII handelt und ein biologisch kompatibles Polymer kovalent an eine oder mehrere FVIII-Aminosäurepositionen 377, 378, 468, 491, 504, 556 und 711 gebunden ist.

14. Konjugat nach Anspruch 1, wobei es sich bei dem Koagulationsfaktorprotein um FVIII handelt und ein biologisch kompatibles Polymer kovalent an eine oder mehrere FVIII-Aminosäurepositionen 81, 129, 377, 378, 468, 487, 491, 504, 556, 570, 711, 1648, 1795, 1796, 1803, 1804, 1808, 1810, 1864, 1903, 1911, 2091, 2118 und 2284 gebunden ist.

15. Konjugat nach einem des Anspruchs 1, wobei es sich bei dem Koagulationsfaktorprotein um B-Domäne-deletierten Faktor VIII handelt,
wobei vorzugsweise ein biologisch kompatibles Polymer kovalent an B-Domäne-deletiertem FVIII an Aminosäureposition 129, 491, 1804 und/oder 1808 gebunden ist.

16. Konjugat nach Anspruch 1, wobei es sich bei dem Koagulationsfaktorprotein um Volllängen-FVIII oder B-Domäne-deletierten FVIII handelt und ein biologisch kompatibles Polymer an die FVIII-Aminosäureposition 1804 gebunden ist und Polyethylenglykol umfasst.

17. Wirksame Menge eines Konjugats nach einem der Ansprüche 1-6 oder 13-19 und wirksame Menge eines Koagulationsfaktors zur Verwendung in der Behandlung einer Blutungsstörung.

18. Konjugat nach einem der Ansprüche 11-16, wobei die Aminosäureposition zu Cystein mutiert ist.

## Revendications

1. Conjugué pour induction d'une tolérance vis-à-vis d'une protéine de facteur de coagulation, où le conjugué comprend une protéine de facteur de coagulation et un fragment antigène ou une variante de celui-ci ainsi qu'un ligand Siglec de lymphocytes B,
où le ligand Siglec est un glycane choisi parmi 9-N-biphénylcarboxyl-NeuAca2-6Gal∼1-4GlcNAc (6'-BPCNeuAc), NeuAca2-6Gal∼1-4GlcNAc et NeuAca2-6Gal∼1-4(6-sulfo)GlcNAc et leurs combinaisons.

2. Conjugué selon la revendication 1, où la protéine de facteur de coagulation est directement conjuguée à un ligand Siglec, ou
où la protéine de facteur de coagulation est conjuguée indirectement à un ligand Siglec.

3. Conjugué selon l'une quelconque des revendications 1 à 2, où le conjugué comprend un liposome.

4. Conjugué selon l'une quelconque des revendications 1 à 3, où la distance séparant la protéine de facteur de coagulation du ligand Siglec du conjugué permet une présentation efficace à un lymphocyte B, ce qui entraîne une ligature forcée et une juxtaposition du Siglec et du récepteur de lymphocyte B dans une synapse immunologique.

5. Conjugué selon l'une quelconque des revendications 1 à 4, où la protéine de facteur de coagulation est choisie dans le groupe constitué par Facteur VII, Facteur VIII, Facteur IX, Facteur X et Facteur XI, ainsi que leurs combinaisons.

6. Composition pharmaceutique comprenant une quantité active du conjugué selon l'une quelconque des revendications 1 à 5.

7. Quantité active d'un conjugué selon l'une quelconque des revendications 1 à 5 pour utilisation dans le traitement d'un trouble hémorragique.

8. Quantité active d'un conjugué pour utilisation selon la revendication 7, où ladite utilisation comprend l'induction d'une tolérance vis-à-vis d'une protéine de facteur de coagulation.

9. Quantité active d'un conjugué pour utilisation selon les revendications 7 ou 8, où ladite utilisation s'effectue en présence d'anticorps anti-protéine de facteur de coagulation.

10. Kit comprenant le conjugué selon les revendications 1 à 5.

11. Conjugué selon la revendication 1, où la protéine de facteur de coagulation est FVIII et un polymère biocompatible est lié de façon covalente en une ou plusieurs des positions d'acides aminés de FVIII 81, 129, 377, 378, 468, 487, 491, 504, 556, 570, 711, 1648, 1795, 1796, 1803, 1804, 1808, 1810, 1864, 1903, 1911, 2091, 2118 et 2284.

12. Conjugué selon la revendication 1, où la protéine de facteur de coagulation est FVIII et un polymère biocompatible est lié de façon covalente en une ou plusieurs des positions d'acides aminés de FVIII 377, 378, 468, 491, 504, 556, 1795, 1796, 1803, 1804, 1808, 1810, 1864, 1903, 1911 et 2284.

13. Conjugué selon la revendication 1, où la protéine de facteur de coagulation est FVIII et un polymère biocompatible est lié de façon covalente en une ou plusieurs des positions d'acides aminés de FVIII 377, 378, 468, 491, 504, 556 et 711.

14. Conjugué selon la revendication 1, où la protéine de facteur de coagulation est FVIII et un polymère biocompatible est lié de façon covalente en une ou plusieurs des positions d'acides aminés de FVIII 81, 129, 377, 378, 468, 487, 491, 504, 556, 570, 711, 1648, 1795, 1796, 1803, 1804, 1808, 1810, 1864, 1903, 1911, 2091, 2118 et 2284.

15. Conjugué selon l'une de la revendication 1, où la protéine de facteur de coagulation est le facteur VIII délété du domaine B,
où préférentiellement, un polymère biocompatible est lié de façon covalente au FVIII délété du domaine B aux positions d'acides aminés 129, 491, 1804 et/ou 1808.

16. Conjugué selon la revendication 1, où la protéine de facteur de coagulation est FVIII de longueur complète ou FVIII délété du domaine B et un polymère biocompatible est lié en la position d'acide aminé de FVIII 1804 et comprend du polyéthylène glycol.

17. Quantité active d'un conjugué selon l'une quelconque des revendications 1 à 6 ou 13 à 19 et quantité active d'un facteur de coagulation pour utilisation dans le traitement d'un trouble hémorragique.

18. Conjugué selon l'une quelconque des revendications 11 à 16, où la position d'acide aminé est mutée en cystéine.


### SEQUENCE LISTING

<110> Bayer HealthCare LLC
<120> COMPOSITIONS AND METHODS FOR INDUCING IMMUNE TOLERANCE TO
   COAGULATION FACTOR PROTEINS
<130> BAY-006PCT1
<150> 61/816,790
   <151> 2013-04-28
<160> 43
<170> PatentIn version 3.5
<210> 1
   <211> 2351
   <212> PRT
   <213> Homo sapiens
<400> 1
<210> 2
   <211> 2332
   <212> PRT
   <213> Homo sapiens
<400> 2
   Ala Thr Arg Arg Tyr Tyr Leu Gly Ala Val Glu Leu Ser Trp Asp Tyr
   1 5 10 15
   Met Gln Ser Asp Leu Gly Glu Leu Pro Val Asp Ala Arg Phe Pro Pro
   20 25 30
   Arg Val Pro Lys Ser Phe Pro Phe Asn Thr Ser Val Val Tyr Lys Lys
   35 40 45
   Thr Leu Phe Val Glu Phe Thr Val His Leu Phe Asn Ile Ala Lys Pro
   50 55 60
   Arg Pro Pro Trp Met Gly Leu Leu Gly Pro Thr Ile Gln Ala Glu Val
   65 70 75 80
   Tyr Asp Thr Val Val Ile Thr Leu Lys Asn Met Ala Ser His Pro Val
   85 90 95
   Ser Leu His Ala Val Gly Val Ser Tyr Trp Lys Ala Ser Glu Gly Ala
   100 105 110
   Glu Tyr Asp Asp Gln Thr Ser Gln Arg Glu Lys Glu Asp Asp Lys Val
   115 120 125
   Phe Pro Gly Gly Ser His Thr Tyr Val Trp Gln Val Leu Lys Glu Asn
   130 135 140
   Gly Pro Met Ala Ser Asp Pro Leu Cys Leu Thr Tyr Ser Tyr Leu Ser
   145 150 155 160
   His Val Asp Leu Val Lys Asp Leu Asn Ser Gly Leu Ile Gly Ala Leu
   165 170 175
   Leu Val Cys Arg Glu Gly Ser Leu Ala Lys Glu Lys Thr Gln Thr Leu
   180 185 190
   His Lys Phe Ile Leu Leu Phe Ala Val Phe Asp Glu Gly Lys Ser Trp
   195 200 205
   His Ser Glu Thr Lys Asn Ser Leu Met Gln Asp Arg Asp Ala Ala Ser
       210                 215                 220
   Ala Arg Ala Trp Pro Lys Met His Thr Val Asn Gly Tyr Val Asn Arg
   225                 230                 235                 240
   Ser Leu Pro Gly Leu Ile Gly Cys His Arg Lys Ser Val Tyr Trp His
                   245                 250                 255
   Val Ile Gly Met Gly Thr Thr Pro Glu Val His Ser Ile Phe Leu Glu
               260                 265                 270
   Gly His Thr Phe Leu Val Arg Asn His Arg Gln Ala Ser Leu Glu Ile
           275                 280                 285
   Ser Pro Ile Thr Phe Leu Thr Ala Gln Thr Leu Leu Met Asp Leu Gly
       290                 295                 300
   Gln Phe Leu Leu Phe Cys His Ile Ser Ser His Gln His Asp Gly Met
   305                 310                 315                 320
   Glu Ala Tyr Val Lys Val Asp Ser Cys Pro Glu Glu Pro Gln Leu Arg
                   325                 330                 335
   Met Lys Asn Asn Glu Glu Ala Glu Asp Tyr Asp Asp Asp Leu Thr Asp
               340                 345                 350
   Ser Glu Met Asp Val Val Arg Phe Asp Asp Asp Asn Ser Pro Ser Phe
           355                 360                 365
   Ile Gln Ile Arg Ser Val Ala Lys Lys His Pro Lys Thr Trp Val His
       370                 375                 380
   Tyr Ile Ala Ala Glu Glu Glu Asp Trp Asp Tyr Ala Pro Leu Val Leu
   385                 390                 395                 400
   Ala Pro Asp Asp Arg Ser Tyr Lys Ser Gln Tyr Leu Asn Asn Gly Pro
                   405                 410                 415
   Gln Arg Ile Gly Arg Lys Tyr Lys Lys Val Arg Phe Met Ala Tyr Thr
               420                 425                 430
   Asp Glu Thr Phe Lys Thr Arg Glu Ala Ile Gln His Glu Ser Gly Ile
           435                 440                 445
   Leu Gly Pro Leu Leu Tyr Gly Glu Val Gly Asp Thr Leu Leu Ile Ile
       450                 455                 460
   Phe Lys Asn Gln Ala Ser Arg Pro Tyr Asn Ile Tyr Pro His Gly Ile
   465                 470                 475                 480
   Thr Asp Val Arg Pro Leu Tyr Ser Arg Arg Leu Pro Lys Gly Val Lys
                   485                 490                 495
   His Leu Lys Asp Phe Pro Ile Leu Pro Gly Glu Ile Phe Lys Tyr Lys
               500                 505                 510
   Trp Thr Val Thr Val Glu Asp Gly Pro Thr Lys Ser Asp Pro Arg Cys
           515                 520                 525
   Leu Thr Arg Tyr Tyr Ser Ser Phe Val Asn Met Glu Arg Asp Leu Ala
       530                 535                 540
   Ser Gly Leu Ile Gly Pro Leu Leu Ile Cys Tyr Lys Glu Ser Val Asp
   545                 550                 555                 560
   Gln Arg Gly Asn Gln Ile Met Ser Asp Lys Arg Asn Val Ile Leu Phe
                   565                 570                 575
   Ser Val Phe Asp Glu Asn Arg Ser Trp Tyr Leu Thr Glu Asn Ile Gln
               580                 585                 590
   Arg Phe Leu Pro Asn Pro Ala Gly Val Gln Leu Glu Asp Pro Glu Phe
           595                 600                 605
   Gln Ala Ser Asn Ile Met His Ser Ile Asn Gly Tyr Val Phe Asp Ser
       610                 615                 620
   Leu Gln Leu Ser Val Cys Leu His Glu Val Ala Tyr Trp Tyr Ile Leu
   625                 630                 635                 640
   Ser Ile Gly Ala Gln Thr Asp Phe Leu Ser Val Phe Phe Ser Gly Tyr
                   645                 650                 655
   Thr Phe Lys His Lys Met Val Tyr Glu Asp Thr Leu Thr Leu Phe Pro
               660                 665                 670
   Phe Ser Gly Glu Thr Val Phe Met Ser Met Glu Asn Pro Gly Leu Trp
           675                 680                 685
   Ile Leu Gly Cys His Asn Ser Asp Phe Arg Asn Arg Gly Met Thr Ala
       690                 695                 700
   Leu Leu Lys Val Ser Ser Cys Asp Lys Asn Thr Gly Asp Tyr Tyr Glu
   705                 710                 715                 720
   Asp Ser Tyr Glu Asp Ile Ser Ala Tyr Leu Leu Ser Lys Asn Asn Ala
                   725                 730                 735
   Ile Glu Pro Arg Ser Phe Ser Gln Asn Ser Arg His Pro Ser Thr Arg
               740                 745                 750
   Gln Lys Gln Phe Asn Ala Thr Thr Ile Pro Glu Asn Asp Ile Glu Lys
           755                 760                 765
   Thr Asp Pro Trp Phe Ala His Arg Thr Pro Met Pro Lys Ile Gln Asn
       770                 775                 780
   Val Ser Ser Ser Asp Leu Leu Met Leu Leu Arg Gln Ser Pro Thr Pro
   785                 790                 795                 800
   His Gly Leu Ser Leu Ser Asp Leu Gln Glu Ala Lys Tyr Glu Thr Phe
                   805                 810                 815
   Ser Asp Asp Pro Ser Pro Gly Ala Ile Asp Ser Asn Asn Ser Leu Ser
               820                 825                 830
   Glu Met Thr His Phe Arg Pro Gln Leu His His Ser Gly Asp Met Val
           835                 840                 845
   Phe Thr Pro Glu Ser Gly Leu Gln Leu Arg Leu Asn Glu Lys Leu Gly
       850                 855                 860
   Thr Thr Ala Ala Thr Glu Leu Lys Lys Leu Asp Phe Lys Val Ser Ser
   865                 870                 875                 880
   Thr Ser Asn Asn Leu Ile Ser Thr Ile Pro Ser Asp Asn Leu Ala Ala
                   885                 890                 895
   Gly Thr Asp Asn Thr Ser Ser Leu Gly Pro Pro Ser Met Pro Val His
               900                 905                 910
   Tyr Asp Ser Gln Leu Asp Thr Thr Leu Phe Gly Lys Lys Ser Ser Pro
           915                 920                 925
   Leu Thr Glu Ser Gly Gly Pro Leu Ser Leu Ser Glu Glu Asn Asn Asp
       930                 935                 940
   Ser Lys Leu Leu Glu Ser Gly Leu Met Asn Ser Gln Glu Ser Ser Trp
   945                 950                 955                 960
   Gly Lys Asn Val Ser Ser Thr Glu Ser Gly Arg Leu Phe Lys Gly Lys
                   965                 970                 975
   Arg Ala His Gly Pro Ala Leu Leu Thr Lys Asp Asn Ala Leu Phe Lys
               980                 985                 990
   Val Ser Ile Ser Leu Leu Lys Thr  Asn Lys Thr Ser Asn  Asn Ser Ala
           995                 1000                 1005
   Thr Asn  Arg Lys Thr His Ile  Asp Gly Pro Ser Leu  Leu Ile Glu
       1010                 1015                 1020
   Asn Ser  Pro Ser Val Trp Gln  Asn Ile Leu Glu Ser  Asp Thr Glu
       1025                 1030                 1035
   Phe Lys  Lys Val Thr Pro Leu  Ile His Asp Arg Met  Leu Met Asp
       1040                 1045                 1050
   Lys Asn  Ala Thr Ala Leu Arg  Leu Asn His Met Ser  Asn Lys Thr
       1055                 1060                 1065
   Thr Ser  Ser Lys Asn Met Glu  Met Val Gln Gln Lys  Lys Glu Gly
       1070                 1075                 1080
   Pro Ile  Pro Pro Asp Ala Gln  Asn Pro Asp Met Ser  Phe Phe Lys
       1085                 1090                 1095
   Met Leu  Phe Leu Pro Glu Ser  Ala Arg Trp Ile Gln  Arg Thr His
       1100                 1105                 1110
   Gly Lys  Asn Ser Leu Asn Ser  Gly Gln Gly Pro Ser  Pro Lys Gln
       1115                 1120                 1125
   Leu Val  Ser Leu Gly Pro Glu  Lys Ser Val Glu Gly  Gln Asn Phe
       1130                 1135                 1140
   Leu Ser  Glu Lys Asn Lys Val  Val Val Gly Lys Gly  Glu Phe Thr
       1145                 1150                 1155
   Lys Asp  Val Gly Leu Lys Glu  Met Val Phe Pro Ser  Ser Arg Asn
       1160                 1165                 1170
   Leu Phe  Leu Thr Asn Leu Asp  Asn Leu His Glu Asn  Asn Thr His
       1175                 1180                 1185
   Asn Gln  Glu Lys Lys Ile Gln  Glu Glu Ile Glu Lys  Lys Glu Thr
       1190                 1195                 1200
   Leu Ile  Gln Glu Asn Val Val  Leu Pro Gln Ile His  Thr Val Thr
       1205                 1210                 1215
   Gly Thr  Lys Asn Phe Met Lys  Asn Leu Phe Leu Leu  Ser Thr Arg
       1220                 1225                 1230
   Gln Asn  Val Glu Gly Ser Tyr  Glu Gly Ala Tyr Ala  Pro Val Leu
       1235                 1240                 1245
   Gln Asp  Phe Arg Ser Leu Asn  Asp Ser Thr Asn Arg  Thr Lys Lys
       1250                 1255                 1260
   His Thr  Ala His Phe Ser Lys  Lys Gly Glu Glu Glu  Asn Leu Glu
       1265                 1270                 1275
   Gly Leu  Gly Asn Gln Thr Lys  Gln Ile Val Glu Lys  Tyr Ala Cys
       1280                 1285                 1290
   Thr Thr  Arg Ile Ser Pro Asn  Thr Ser Gln Gln Asn  Phe Val Thr
       1295                 1300                 1305
   Gln Arg  Ser Lys Arg Ala Leu  Lys Gln Phe Arg Leu  Pro Leu Glu
       1310                 1315                 1320
   Glu Thr  Glu Leu Glu Lys Arg  Ile Ile Val Asp Asp  Thr Ser Thr
       1325                 1330                 1335
   Gln Trp  Ser Lys Asn Met Lys  His Leu Thr Pro Ser  Thr Leu Thr
       1340                 1345                 1350
   Gln Ile  Asp Tyr Asn Glu Lys  Glu Lys Gly Ala Ile  Thr Gln Ser
       1355                 1360                 1365
   Pro Leu  Ser Asp Cys Leu Thr  Arg Ser His Ser Ile  Pro Gln Ala
       1370                 1375                 1380
   Asn Arg  Ser Pro Leu Pro Ile  Ala Lys Val Ser Ser  Phe Pro Ser
       1385                 1390                 1395
   Ile Arg  Pro Ile Tyr Leu Thr  Arg Val Leu Phe Gln  Asp Asn Ser
       1400                 1405                 1410
   Ser His  Leu Pro Ala Ala Ser  Tyr Arg Lys Lys Asp  Ser Gly Val
       1415                 1420                 1425
   Gln Glu  Ser Ser His Phe Leu  Gln Gly Ala Lys Lys  Asn Asn Leu
       1430                 1435                 1440
   Ser Leu Ala Ile Leu Thr Leu Glu Met Thr Gly Asp Gln Arg Glu
       1445                1450                1455
   Val Gly Ser Leu Gly Thr Ser Ala Thr Asn Ser Val Thr Tyr Lys
       1460                1465                1470
   Lys Val Glu Asn Thr Val Leu Pro Lys Pro Asp Leu Pro Lys Thr
       1475                1480                1485
   Ser Gly Lys Val Glu Leu Leu Pro Lys Val His Ile Tyr Gln Lys
       1490                1495                1500
   Asp Leu Phe Pro Thr Glu Thr Ser Asn Gly Ser Pro Gly His Leu
       1505                1510                1515
   Asp Leu Val Glu Gly Ser Leu Leu Gln Gly Thr Glu Gly Ala Ile
       1520                1525                1530
   Lys Trp Asn Glu Ala Asn Arg Pro Gly Lys Val Pro Phe Leu Arg
       1535                1540                1545
   Val Ala Thr Glu Ser Ser Ala Lys Thr Pro Ser Lys Leu Leu Asp
       1550                1555                1560
   Pro Leu Ala Trp Asp Asn His Tyr Gly Thr Gln Ile Pro Lys Glu
       1565                1570                1575
   Glu Trp Lys Ser Gln Glu Lys Ser Pro Glu Lys Thr Ala Phe Lys
       1580                1585                1590
   Lys Lys Asp Thr Ile Leu Ser Leu Asn Ala Cys Glu Ser Asn His
       1595                1600                1605
   Ala Ile Ala Ala Ile Asn Glu Gly Gln Asn Lys Pro Glu Ile Glu
       1610                1615                1620
   Val Thr Trp Ala Lys Gln Gly Arg Thr Glu Arg Leu Cys Ser Gln
       1625                1630                1635
   Asn Pro Pro Val Leu Lys Arg His Gln Arg Glu Ile Thr Arg Thr
       1640                1645                1650
   Thr Leu Gln Ser Asp Gln Glu Glu Ile Asp Tyr Asp Asp Thr Ile
       1655                1660                1665
   Ser Val Glu Met Lys Lys Glu Asp Phe Asp Ile Tyr Asp Glu Asp
   1670 1675 1680
   Glu Asn Gln Ser Pro Arg Ser Phe Gln Lys Lys Thr Arg His Tyr
   1685 1690 1695
   Phe Ile Ala Ala Val Glu Arg Leu Trp Asp Tyr Gly Met Ser Ser
   1700 1705 1710
   Ser Pro His Val Leu Arg Asn Arg Ala Gln Ser Gly Ser Val Pro
   1715 1720 1725
   Gln Phe Lys Lys Val Val Phe Gln Glu Phe Thr Asp Gly Ser Phe
   1730 1735 1740
   Thr Gln Pro Leu Tyr Arg Gly Glu Leu Asn Glu His Leu Gly Leu
   1745 1750 1755
   Leu Gly Pro Tyr Ile Arg Ala Glu Val Glu Asp Asn Ile Met Val
   1760 1765 1770
   Thr Phe Arg Asn Gln Ala Ser Arg Pro Tyr Ser Phe Tyr Ser Ser
   1775 1780 1785
   Leu Ile Ser Tyr Glu Glu Asp Gln Arg Gln Gly Ala Glu Pro Arg
   1790 1795 1800
   Lys Asn Phe Val Lys Pro Asn Glu Thr Lys Thr Tyr Phe Trp Lys
   1805 1810 1815
   Val Gln His His Met Ala Pro Thr Lys Asp Glu Phe Asp Cys Lys
   1820 1825 1830
   Ala Trp Ala Tyr Phe Ser Asp Val Asp Leu Glu Lys Asp Val His
   1835 1840 1845
   Ser Gly Leu Ile Gly Pro Leu Leu Val Cys His Thr Asn Thr Leu
   1850 1855 1860
   Asn Pro Ala His Gly Arg Gln Val Thr Val Gln Glu Phe Ala Leu
   1865 1870 1875
   Phe Phe Thr Ile Phe Asp Glu Thr Lys Ser Trp Tyr Phe Thr Glu
   1880 1885 1890
   Asn Met Glu Arg Asn Cys Arg Ala Pro Cys Asn Ile Gln Met Glu
   1895 1900 1905
   Asp Pro  Thr Phe Lys Glu Asn  Tyr Arg Phe His Ala  Ile Asn Gly
       1910                 1915                 1920
   Tyr Ile  Met Asp Thr Leu Pro  Gly Leu Val Met Ala  Gln Asp Gln
       1925                 1930                 1935
   Arg Ile  Arg Trp Tyr Leu Leu  Ser Met Gly Ser Asn  Glu Asn Ile
       1940                 1945                 1950
   His Ser  Ile His Phe Ser Gly  His Val Phe Thr Val  Arg Lys Lys
       1955                 1960                 1965
   Glu Glu  Tyr Lys Met Ala Leu  Tyr Asn Leu Tyr Pro  Gly Val Phe
       1970                 1975                 1980
   Glu Thr  Val Glu Met Leu Pro  Ser Lys Ala Gly Ile  Trp Arg Val
       1985                 1990                 1995
   Glu Cys  Leu Ile Gly Glu His  Leu His Ala Gly Met  Ser Thr Leu
       2000                 2005                 2010
   Phe Leu  Val Tyr Ser Asn Lys  Cys Gln Thr Pro Leu  Gly Met Ala
       2015                 2020                 2025
   Ser Gly  His Ile Arg Asp Phe  Gln Ile Thr Ala Ser  Gly Gln Tyr
       2030                 2035                 2040
   Gly Gln  Trp Ala Pro Lys Leu  Ala Arg Leu His Tyr  Ser Gly Ser
       2045                 2050                 2055
   Ile Asn  Ala Trp Ser Thr Lys  Glu Pro Phe Ser Trp  Ile Lys Val
       2060                 2065                 2070
   Asp Leu  Leu Ala Pro Met Ile  Ile His Gly Ile Lys  Thr Gln Gly
       2075                 2080                 2085
   Ala Arg  Gln Lys Phe Ser Ser  Leu Tyr Ile Ser Gln  Phe Ile Ile
       2090                 2095                 2100
   Met Tyr  Ser Leu Asp Gly Lys  Lys Trp Gln Thr Tyr  Arg Gly Asn
       2105                 2110                 2115
   Ser Thr  Gly Thr Leu Met Val  Phe Phe Gly Asn Val  Asp Ser Ser
       2120                 2125                 2130
   Gly Ile  Lys His Asn Ile Phe  Asn Pro Pro Ile Ile  Ala Arg Tyr
       2135                 2140                 2145
   Ile Arg Leu His Pro Thr His Tyr Ser Ile Arg Ser Thr Leu Arg
   2150 2155 2160
   Met Glu Leu Met Gly Cys Asp Leu Asn Ser Cys Ser Met Pro Leu
   2165 2170 2175
   Gly Met Glu Ser Lys Ala Ile Ser Asp Ala Gln Ile Thr Ala Ser
   2180 2185 2190
   Ser Tyr Phe Thr Asn Met Phe Ala Thr Trp Ser Pro Ser Lys Ala
   2195 2200 2205
   Arg Leu His Leu Gln Gly Arg Ser Asn Ala Trp Arg Pro Gln Val
   2210 2215 2220
   Asn Asn Pro Lys Glu Trp Leu Gln Val Asp Phe Gln Lys Thr Met
   2225 2230 2235
   Lys Val Thr Gly Val Thr Thr Gln Gly Val Lys Ser Leu Leu Thr
   2240 2245 2250
   Ser Met Tyr Val Lys Glu Phe Leu Ile Ser Ser Ser Gln Asp Gly
   2255 2260 2265
   His Gln Trp Thr Leu Phe Phe Gln Asn Gly Lys Val Lys Val Phe
   2270 2275 2280
   Gln Gly Asn Gln Asp Ser Phe Thr Pro Val Val Asn Ser Leu Asp
   2285 2290 2295
   Pro Pro Leu Leu Thr Arg Tyr Leu Arg Ile His Pro Gln Ser Trp
   2300 2305 2310
   Val His Gln Ile Ala Leu Arg Met Glu Val Leu Gly Cys Glu Ala
   2315 2320 2325
   Gln Asp Leu Tyr
   2330
<210> 3
   <211> 4
   <212> PRT
   <213> Artificial sequence
<220>
   <223> peptide
<400> 3
   Ser Phe Ser Gln
   1
<210> 4
   <211> 10
   <212> PRT
   <213> Artificial sequence
<220>
   <223> peptide
<400> 4
   Asn Pro Pro Val Leu Lys Arg His Gln Arg
   1 5 10
<210> 5
   <211> 1457
   <212> PRT
   <213> Artificial sequence
<220>
   <223> B-domain deleted recombinant FVIII
<400> 5
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Val His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
               180                 185                 190
   Gly Ala Leu Leu Val Cys Arg Glu Gly Ser Leu Ala Lys Glu Lys Thr
           195                 200                 205
   Gln Thr Leu His Lys Phe Ile Leu Leu Phe Ala Val Phe Asp Glu Gly
       210                 215                 220
   Lys Ser Trp His Ser Glu Thr Lys Asn Ser Leu Met Gln Asp Arg Asp
   225                 230                 235                 240
   Ala Ala Ser Ala Arg Ala Trp Pro Lys Met His Thr Val Asn Gly Tyr
                   245                 250                 255
   Val Asn Arg Ser Leu Pro Gly Leu Ile Gly Cys His Arg Lys Ser Val
               260                 265                 270
   Tyr Trp His Val Ile Gly Met Gly Thr Thr Pro Glu Val His Ser Ile
           275                 280                 285
   Phe Leu Glu Gly His Thr Phe Leu Val Arg Asn His Arg Gln Ala Ser
       290                 295                 300
   Leu Glu Ile Ser Pro Ile Thr Phe Leu Thr Ala Gln Thr Leu Leu Met
   305                 310                 315                 320
   Asp Leu Gly Gln Phe Leu Leu Phe Cys His Ile Ser Ser His Gln His
                   325                 330                 335
   Asp Gly Met Glu Ala Tyr Val Lys Val Asp Ser Cys Pro Glu Glu Pro
               340                 345                 350
   Gln Leu Arg Met Lys Asn Asn Glu Glu Ala Glu Asp Tyr Asp Asp Asp
           355                 360                 365
   Leu Thr Asp Ser Glu Met Asp Val Val Arg Phe Asp Asp Asp Asn Ser
       370                 375                 380
   Pro Ser Phe Ile Gln Ile Arg Ser Val Ala Lys Lys His Pro Lys Thr
   385                 390                 395                 400
   Trp Val His Tyr Ile Ala Ala Glu Glu Glu Asp Trp Asp Tyr Ala Pro
                   405                 410                 415
   Leu Val Leu Ala Pro Asp Asp Arg Ser Tyr Lys Ser Gln Tyr Leu Asn
               420                 425                 430
   Asn Gly Pro Gln Arg Ile Gly Arg Lys Tyr Lys Lys Val Arg Phe Met
           435                 440                 445
   Ala Tyr Thr Asp Glu Thr Phe Lys Thr Arg Glu Ala Ile Gln His Glu
       450                 455                 460
   Ser Gly Ile Leu Gly Pro Leu Leu Tyr Gly Glu Val Gly Asp Thr Leu
   465                 470                 475                 480
   Leu Ile Ile Phe Lys Asn Gln Ala Ser Arg Pro Tyr Asn Ile Tyr Pro
                   485                 490                 495
   His Gly Ile Thr Asp Val Arg Pro Leu Tyr Ser Arg Arg Leu Pro Lys
               500                 505                 510
   Gly Val Lys His Leu Lys Asp Phe Pro Ile Leu Pro Gly Glu Ile Phe
           515                 520                 525
   Lys Tyr Lys Trp Thr Val Thr Val Glu Asp Gly Pro Thr Lys Ser Asp
       530                 535                 540
   Pro Arg Cys Leu Thr Arg Tyr Tyr Ser Ser Phe Val Asn Met Glu Arg
   545                 550                 555                 560
   Asp Leu Ala Ser Gly Leu Ile Gly Pro Leu Leu Ile Cys Tyr Lys Glu
                   565                 570                 575
   Ser Val Asp Gln Arg Gly Asn Gln Ile Met Ser Asp Lys Arg Asn Val
               580                 585                 590
   Ile Leu Phe Ser Val Phe Asp Glu Asn Arg Ser Trp Tyr Leu Thr Glu
           595                 600                 605
   Asn Ile Gln Arg Phe Leu Pro Asn Pro Ala Gly Val Gln Leu Glu Asp
       610                 615                 620
   Pro Glu Phe Gln Ala Ser Asn Ile Met His Ser Ile Asn Gly Tyr Val
   625                 630                 635                 640
   Phe Asp Ser Leu Gln Leu Ser Val Cys Leu His Glu Val Ala Tyr Trp
                   645                 650                 655
   Tyr Ile Leu Ser Ile Gly Ala Gln Thr Asp Phe Leu Ser Val Phe Phe
               660                 665                 670
   Ser Gly Tyr Thr Phe Lys His Lys Met Val Tyr Glu Asp Thr Leu Thr
           675                 680                 685
   Leu Phe Pro Phe Ser Gly Glu Thr Val Phe Met Ser Met Glu Asn Pro
       690                 695                 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Asn Pro Pro Val Leu
           755                 760                 765
   Lys Arg His Gln Arg Glu Ile Thr Arg Thr Thr Leu Gln Ser Asp Gln
       770                 775                 780
   Glu Glu Ile Asp Tyr Asp Asp Thr Ile Ser Val Glu Met Lys Lys Glu
   785                 790                 795                 800
   Asp Phe Asp Ile Tyr Asp Glu Asp Glu Asn Gln Ser Pro Arg Ser Phe
                   805                 810                 815
   Gln Lys Lys Thr Arg His Tyr Phe Ile Ala Ala Val Glu Arg Leu Trp
               820                 825                 830
   Asp Tyr Gly Met Ser Ser Ser Pro His Val Leu Arg Asn Arg Ala Gln
           835                 840                 845
   Ser Gly Ser Val Pro Gln Phe Lys Lys Val Val Phe Gln Glu Phe Thr
       850                 855                 860
   Asp Gly Ser Phe Thr Gln Pro Leu Tyr Arg Gly Glu Leu Asn Glu His
   865                 870                 875                 880
   Leu Gly Leu Leu Gly Pro Tyr Ile Arg Ala Glu Val Glu Asp Asn Ile
                   885                 890                 895
   Met Val Thr Phe Arg Asn Gln Ala Ser Arg Pro Tyr Ser Phe Tyr Ser
   900 905 910
   Ser Leu Ile Ser Tyr Glu Glu Asp Gln Arg Gln Gly Ala Glu Pro Arg
   915 920 925
   Lys Asn Phe Val Lys Pro Asn Glu Thr Lys Thr Tyr Phe Trp Lys Val
   930 935 940
   Gln His His Met Ala Pro Thr Lys Asp Glu Phe Asp Cys Lys Ala Trp
   945 950 955 960
   Ala Tyr Phe Ser Asp Val Asp Leu Glu Lys Asp Val His Ser Gly Leu
   965 970 975
   Ile Gly Pro Leu Leu Val Cys His Thr Asn Thr Leu Asn Pro Ala His
   980 985 990
   Gly Arg Gln Val Thr Val Gln Glu Phe Ala Leu Phe Phe Thr Ile Phe
   995 1000 1005
   Asp Glu Thr Lys Ser Trp Tyr Phe Thr Glu Asn Met Glu Arg Asn
   1010 1015 1020
   Cys Arg Ala Pro Cys Asn Ile Gln Met Glu Asp Pro Thr Phe Lys
   1025 1030 1035
   Glu Asn Tyr Arg Phe His Ala Ile Asn Gly Tyr Ile Met Asp Thr
   1040 1045 1050
   Leu Pro Gly Leu Val Met Ala Gln Asp Gln Arg Ile Arg Trp Tyr
   1055 1060 1065
   Leu Leu Ser Met Gly Ser Asn Glu Asn Ile His Ser Ile His Phe
   1070 1075 1080
   Ser Gly His Val Phe Thr Val Arg Lys Lys Glu Glu Tyr Lys Met
   1085 1090 1095
   Ala Leu Tyr Asn Leu Tyr Pro Gly Val Phe Glu Thr Val Glu Met
   1100 1105 1110
   Leu Pro Ser Lys Ala Gly Ile Trp Arg Val Glu Cys Leu Ile Gly
   1115 1120 1125
   Glu His Leu His Ala Gly Met Ser Thr Leu Phe Leu Val Tyr Ser
   1130 1135 1140
   Asn Lys Cys Gln Thr Pro Leu Gly Met Ala Ser Gly His Ile Arg
   1145 1150 1155
   Asp Phe Gln Ile Thr Ala Ser Gly Gln Tyr Gly Gln Trp Ala Pro
   1160 1165 1170
   Lys Leu Ala Arg Leu His Tyr Ser Gly Ser Ile Asn Ala Trp Ser
   1175 1180 1185
   Thr Lys Glu Pro Phe Ser Trp Ile Lys Val Asp Leu Leu Ala Pro
   1190 1195 1200
   Met Ile Ile His Gly Ile Lys Thr Gln Gly Ala Arg Gln Lys Phe
   1205 1210 1215
   Ser Ser Leu Tyr Ile Ser Gln Phe Ile Ile Met Tyr Ser Leu Asp
   1220 1225 1230
   Gly Lys Lys Trp Gln Thr Tyr Arg Gly Asn Ser Thr Gly Thr Leu
   1235 1240 1245
   Met Val Phe Phe Gly Asn Val Asp Ser Ser Gly Ile Lys His Asn
   1250 1255 1260
   Ile Phe Asn Pro Pro Ile Ile Ala Arg Tyr Ile Arg Leu His Pro
   1265 1270 1275
   Thr His Tyr Ser Ile Arg Ser Thr Leu Arg Met Glu Leu Met Gly
   1280 1285 1290
   Cys Asp Leu Asn Ser Cys Ser Met Pro Leu Gly Met Glu Ser Lys
   1295 1300 1305
   Ala Ile Ser Asp Ala Gln Ile Thr Ala Ser Ser Tyr Phe Thr Asn
   1310 1315 1320
   Met Phe Ala Thr Trp Ser Pro Ser Lys Ala Arg Leu His Leu Gln
   1325 1330 1335
   Gly Arg Ser Asn Ala Trp Arg Pro Gln Val Asn Asn Pro Lys Glu
   1340 1345 1350
   Trp Leu Gln Val Asp Phe Gln Lys Thr Met Lys Val Thr Gly Val
   1355 1360 1365
   Thr Thr Gln Gly Val Lys Ser Leu Leu Thr Ser Met Tyr Val Lys
   1370 1375 1380
   Glu Phe Leu Ile Ser Ser Ser Gln Asp Gly His Gln Trp Thr Leu
   1385 1390 1395
   Phe Phe Gln Asn Gly Lys Val Lys Val Phe Gln Gly Asn Gln Asp
   1400 1405 1410
   Ser Phe Thr Pro Val Val Asn Ser Leu Asp Pro Pro Leu Leu Thr
   1415 1420 1425
   Arg Tyr Leu Arg Ile His Pro Gln Ser Trp Val His Gln Ile Ala
   1430 1435 1440
   Leu Arg Met Glu Val Leu Gly Cys Glu Ala Gln Asp Leu Tyr
   1445 1450 1455
<210> 6
   <211> 1438
   <212> PRT
   <213> Artificial sequence
<220>
   <223> B-domain deleted recombinant FVIII
<400> 6
   Ala Thr Arg Arg Tyr Tyr Leu Gly Ala Val Glu Leu Ser Trp Asp Tyr
   1 5 10 15
   Met Gln Ser Asp Leu Gly Glu Leu Pro Val Asp Ala Arg Phe Pro Pro
   20 25 30
   Arg Val Pro Lys Ser Phe Pro Phe Asn Thr Ser Val Val Tyr Lys Lys
   35 40 45
   Thr Leu Phe Val Glu Phe Thr Asp His Leu Phe Asn Ile Ala Lys Pro
   50 55 60
   Arg Pro Pro Trp Met Gly Leu Leu Gly Pro Thr Ile Gln Ala Glu Val
   65 70 75 80
   Tyr Asp Thr Val Val Ile Thr Leu Lys Asn Met Ala Ser His Pro Val
   85 90 95
   Ser Leu His Ala Val Gly Val Ser Tyr Trp Lys Ala Ser Glu Gly Ala
   100 105 110
   Glu Tyr Asp Asp Gln Thr Ser Gln Arg Glu Lys Glu Asp Asp Lys Val
   115 120 125
   Phe Pro Gly Gly Ser His Thr Tyr Val Trp Gln Val Leu Lys Glu Asn
       130                 135                 140
   Gly Pro Met Ala Ser Asp Pro Leu Cys Leu Thr Tyr Ser Tyr Leu Ser
   145                 150                 155                 160
   His Val Asp Leu Val Lys Asp Leu Asn Ser Gly Leu Ile Gly Ala Leu
                   165                 170                 175
   Leu Val Cys Arg Glu Gly Ser Leu Ala Lys Glu Lys Thr Gln Thr Leu
               180                 185                 190
   His Lys Phe Ile Leu Leu Phe Ala Val Phe Asp Glu Gly Lys Ser Trp
           195                 200                 205
   His Ser Glu Thr Lys Asn Ser Leu Met Gln Asp Arg Asp Ala Ala Ser
       210                 215                 220
   Ala Arg Ala Trp Pro Lys Met His Thr Val Asn Gly Tyr Val Asn Arg
   225                 230                 235                 240
   Ser Leu Pro Gly Leu Ile Gly Cys His Arg Lys Ser Val Tyr Trp His
                   245                 250                 255
   Val Ile Gly Met Gly Thr Thr Pro Glu Val His Ser Ile Phe Leu Glu
               260                 265                 270
   Gly His Thr Phe Leu Val Arg Asn His Arg Gln Ala Ser Leu Glu Ile
           275                 280                 285
   Ser Pro Ile Thr Phe Leu Thr Ala Gln Thr Leu Leu Met Asp Leu Gly
       290                 295                 300
   Gln Phe Leu Leu Phe Cys His Ile Ser Ser His Gln His Asp Gly Met
   305                 310                 315                 320
   Glu Ala Tyr Val Lys Val Asp Ser Cys Pro Glu Glu Pro Gln Leu Arg
                   325                 330                 335
   Met Lys Asn Asn Glu Glu Ala Glu Asp Tyr Asp Asp Asp Leu Thr Asp
               340                 345                 350
   Ser Glu Met Asp Val Val Arg Phe Asp Asp Asp Asn Ser Pro Ser Phe
           355                 360                 365
   Ile Gln Ile Arg Ser Val Ala Lys Lys His Pro Lys Thr Trp Val His
       370                 375                 380
   Tyr Ile Ala Ala Glu Glu Glu Asp Trp Asp Tyr Ala Pro Leu Val Leu
   385                 390                 395                 400
   Ala Pro Asp Asp Arg Ser Tyr Lys Ser Gln Tyr Leu Asn Asn Gly Pro
                   405                 410                 415
   Gln Arg Ile Gly Arg Lys Tyr Lys Lys Val Arg Phe Met Ala Tyr Thr
               420                 425                 430
   Asp Glu Thr Phe Lys Thr Arg Glu Ala Ile Gln His Glu Ser Gly Ile
           435                 440                 445
   Leu Gly Pro Leu Leu Tyr Gly Glu Val Gly Asp Thr Leu Leu Ile Ile
       450                 455                 460
   Phe Lys Asn Gln Ala Ser Arg Pro Tyr Asn Ile Tyr Pro His Gly Ile
   465                 470                 475                 480
   Thr Asp Val Arg Pro Leu Tyr Ser Arg Arg Leu Pro Lys Gly Val Lys
                   485                 490                 495
   His Leu Lys Asp Phe Pro Ile Leu Pro Gly Glu Ile Phe Lys Tyr Lys
               500                 505                 510
   Trp Thr Val Thr Val Glu Asp Gly Pro Thr Lys Ser Asp Pro Arg Cys
           515                 520                 525
   Leu Thr Arg Tyr Tyr Ser Ser Phe Val Asn Met Glu Arg Asp Leu Ala
       530                 535                 540
   Ser Gly Leu Ile Gly Pro Leu Leu Ile Cys Tyr Lys Glu Ser Val Asp
   545                 550                 555                 560
   Gln Arg Gly Asn Gln Ile Met Ser Asp Lys Arg Asn Val Ile Leu Phe
                   565                 570                 575
   Ser Val Phe Asp Glu Asn Arg Ser Trp Tyr Leu Thr Glu Asn Ile Gln
               580                 585                 590
   Arg Phe Leu Pro Asn Pro Ala Gly Val Gln Leu Glu Asp Pro Glu Phe
           595                 600                 605
   Gln Ala Ser Asn Ile Met His Ser Ile Asn Gly Tyr Val Phe Asp Ser
       610                 615                 620
   Leu Gln Leu Ser Val Cys Leu His Glu Val Ala Tyr Trp Tyr Ile Leu
   625 630 635 640
   Ser Ile Gly Ala Gln Thr Asp Phe Leu Ser Val Phe Phe Ser Gly Tyr
   645 650 655
   Thr Phe Lys His Lys Met Val Tyr Glu Asp Thr Leu Thr Leu Phe Pro
   660 665 670
   Phe Ser Gly Glu Thr Val Phe Met Ser Met Glu Asn Pro Gly Leu Trp
   675 680 685
   Ile Leu Gly Cys His Asn Ser Asp Phe Arg Asn Arg Gly Met Thr Ala
   690 695 700
   Leu Leu Lys Val Ser Ser Cys Asp Lys Asn Thr Gly Asp Tyr Tyr Glu
   705 710 715 720
   Asp Ser Tyr Glu Asp Ile Ser Ala Tyr Leu Leu Ser Lys Asn Asn Ala
   725 730 735
   Ile Glu Pro Arg Ser Phe Ser Gln Asn Pro Pro Val Leu Lys Arg His
   740 745 750
   Gln Arg Glu Ile Thr Arg Thr Thr Leu Gln Ser Asp Gln Glu Glu Ile
   755 760 765
   Asp Tyr Asp Asp Thr Ile Ser Val Glu Met Lys Lys Glu Asp Phe Asp
   770 775 780
   Ile Tyr Asp Glu Asp Glu Asn Gln Ser Pro Arg Ser Phe Gln Lys Lys
   785 790 795 800
   Thr Arg His Tyr Phe Ile Ala Ala Val Glu Arg Leu Trp Asp Tyr Gly
   805 810 815
   Met Ser Ser Ser Pro His Val Leu Arg Asn Arg Ala Gln Ser Gly Ser
   820 825 830
   Val Pro Gln Phe Lys Lys Val Val Phe Gln Glu Phe Thr Asp Gly Ser
   835 840 845
   Phe Thr Gln Pro Leu Tyr Arg Gly Glu Leu Asn Glu His Leu Gly Leu
   850 855 860
   Leu Gly Pro Tyr Ile Arg Ala Glu Val Glu Asp Asn Ile Met Val Thr
   865 870 875 880
   Phe Arg Asn Gln Ala Ser Arg Pro Tyr Ser Phe Tyr Ser Ser Leu Ile
   885 890 895
   Ser Tyr Glu Glu Asp Gln Arg Gln Gly Ala Glu Pro Arg Lys Asn Phe
   900 905 910
   Val Lys Pro Asn Glu Thr Lys Thr Tyr Phe Trp Lys Val Gln His His
   915 920 925
   Met Ala Pro Thr Lys Asp Glu Phe Asp Cys Lys Ala Trp Ala Tyr Phe
   930 935 940
   Ser Asp Val Asp Leu Glu Lys Asp Val His Ser Gly Leu Ile Gly Pro
   945 950 955 960
   Leu Leu Val Cys His Thr Asn Thr Leu Asn Pro Ala His Gly Arg Gln
   965 970 975
   Val Thr Val Gln Glu Phe Ala Leu Phe Phe Thr Ile Phe Asp Glu Thr
   980 985 990
   Lys Ser Trp Tyr Phe Thr Glu Asn Met Glu Arg Asn Cys Arg Ala Pro
   995 1000 1005
   Cys Asn Ile Gln Met Glu Asp Pro Thr Phe Lys Glu Asn Tyr Arg
   1010 1015 1020
   Phe His Ala Ile Asn Gly Tyr Ile Met Asp Thr Leu Pro Gly Leu
   1025 1030 1035
   Val Met Ala Gln Asp Gln Arg Ile Arg Trp Tyr Leu Leu Ser Met
   1040 1045 1050
   Gly Ser Asn Glu Asn Ile His Ser Ile His Phe Ser Gly His Val
   1055 1060 1065
   Phe Thr Val Arg Lys Lys Glu Glu Tyr Lys Met Ala Leu Tyr Asn
   1070 1075 1080
   Leu Tyr Pro Gly Val Phe Glu Thr Val Glu Met Leu Pro Ser Lys
   1085 1090 1095
   Ala Gly Ile Trp Arg Val Glu Cys Leu Ile Gly Glu His Leu His
   1100 1105 1110
   Ala Gly Met Ser Thr Leu Phe Leu Val Tyr Ser Asn Lys Cys Gln
   1115 1120 1125
   Thr Pro  Leu Gly Met Ala Ser  Gly His Ile Arg Asp  Phe Gln Ile
       1130                 1135                 1140
   Thr Ala  Ser Gly Gln Tyr Gly  Gln Trp Ala Pro Lys  Leu Ala Arg
       1145                 1150                 1155
   Leu His  Tyr Ser Gly Ser Ile  Asn Ala Trp Ser Thr  Lys Glu Pro
       1160                 1165                 1170
   Phe Ser  Trp Ile Lys Val Asp  Leu Leu Ala Pro Met  Ile Ile His
       1175                 1180                 1185
   Gly Ile  Lys Thr Gln Gly Ala  Arg Gln Lys Phe Ser  Ser Leu Tyr
       1190                 1195                 1200
   Ile Ser  Gln Phe Ile Ile Met  Tyr Ser Leu Asp Gly  Lys Lys Trp
       1205                 1210                 1215
   Gln Thr  Tyr Arg Gly Asn Ser  Thr Gly Thr Leu Met  Val Phe Phe
       1220                 1225                 1230
   Gly Asn  Val Asp Ser Ser Gly  Ile Lys His Asn Ile  Phe Asn Pro
       1235                 1240                 1245
   Pro Ile  Ile Ala Arg Tyr Ile  Arg Leu His Pro Thr  His Tyr Ser
       1250                 1255                 1260
   Ile Arg  Ser Thr Leu Arg Met  Glu Leu Met Gly Cys  Asp Leu Asn
       1265                 1270                 1275
   Ser Cys  Ser Met Pro Leu Gly  Met Glu Ser Lys Ala  Ile Ser Asp
       1280                 1285                 1290
   Ala Gln  Ile Thr Ala Ser Ser  Tyr Phe Thr Asn Met  Phe Ala Thr
       1295                 1300                 1305
   Trp Ser  Pro Ser Lys Ala Arg  Leu His Leu Gln Gly  Arg Ser Asn
       1310                 1315                 1320
   Ala Trp  Arg Pro Gln Val Asn  Asn Pro Lys Glu Trp  Leu Gln Val
       1325                 1330                 1335
   Asp Phe  Gln Lys Thr Met Lys  Val Thr Gly Val Thr  Thr Gln Gly
       1340                 1345                 1350
   Val Lys  Ser Leu Leu Thr Ser  Met Tyr Val Lys Glu  Phe Leu Ile
       1355                 1360                 1365
   Ser Ser Ser Gln Asp Gly His Gln Trp Thr Leu Phe Phe Gln Asn
   1370 1375 1380
   Gly Lys Val Lys Val Phe Gln Gly Asn Gln Asp Ser Phe Thr Pro
   1385 1390 1395
   Val Val Asn Ser Leu Asp Pro Pro Leu Leu Thr Arg Tyr Leu Arg
   1400 1405 1410
   Ile His Pro Gln Ser Trp Val His Gln Ile Ala Leu Arg Met Glu
   1415 1420 1425
   Val Leu Gly Cys Glu Ala Gln Asp Leu Tyr
   1430 1435
<210> 7
   <211> 625
   <212> PRT
   <213> Homo sapiens
<400> 7
<210> 8
   <211> 414
   <212> PRT
   <213> Homo sapiens
<400> 8
   Tyr Asn Ser Gly Lys Leu Glu Glu Phe Val Gln Gly Asn Leu Glu Arg
   1               5                   10                  15
   Glu Cys Met Glu Glu Lys Cys Ser Phe Glu Glu Ala Arg Glu Val Phe
               20                  25                  30
   Glu Asn Thr Glu Arg Thr Thr Glu Phe Trp Lys Gln Tyr Val Asp Gly
           35                  40                  45
   Asp Gln Cys Glu Ser Asn Pro Cys Leu Asn Gly Gly Ser Cys Lys Asp
       50                  55                  60
   Asp Ile Asn Ser Tyr Glu Cys Trp Cys Pro Phe Gly Phe Glu Gly Lys
   65                  70                  75                  80
   Asn Cys Glu Leu Asp Val Thr Cys Asn Ile Lys Asn Gly Arg Cys Glu
                   85                  90                  95
   Gln Phe Cys Lys Asn Ser Ala Asp Asn Lys Val Val Cys Ser Cys Thr
               100                 105                 110
   Glu Gly Tyr Arg Leu Ala Glu Asn Gln Lys Ser Cys Glu Pro Ala Val
           115                 120                 125
   Pro Phe Pro Cys Gly Arg Val Ser Val Ser Gln Thr Ser Lys Leu Thr
       130                 135                 140
   Arg Ala Glu Ala Val Phe Pro Asp Val Asp Tyr Val Asn Ser Thr Glu
   145                 150                 155                 160
   Ala Glu Thr Ile Leu Asp Asn Ile Thr Gln Ser Thr Gln Ser Phe Asn
                   165                 170                 175
   Asp Phe Thr Arg Val Val Gly Gly Glu Asp Ala Lys Pro Gly Gln Phe
               180                 185                 190
   Pro Trp Gln Val Val Leu Asn Gly Lys Val Asp Ala Phe Cys Gly Gly
           195                 200                 205
   Ser Ile Val Asn Glu Lys Trp Val Thr Ala Ala His Cys Val Glu Thr
       210                 215                 220
   Gly Val Lys Ile Thr Val Val Ala Gly Glu His Asn Ile Glu Glu Thr
   225                 230                 235                 240
   Glu His Thr Glu Gln Lys Arg Asn Val Ile Arg Ile Ile Pro His His
                   245                 250                 255
   Asn Tyr Asn Ala Ala Ile Asn Lys Tyr Asn His Asp Ile Ala Leu Leu
               260                 265                 270
   Glu Leu Asp Glu Pro Leu Val Leu Asn Ser Tyr Val Thr Pro Ile Cys
           275                 280                 285
   Ile Ala Asp Lys Glu Tyr Thr Asn Ile Phe Leu Lys Phe Gly Ser Gly
       290                 295                 300
   Tyr Val Ser Gly Trp Gly Arg Val Phe His Lys Gly Arg Ser Ala Leu
   305                 310                 315                 320
   Val Leu Gln Tyr Leu Arg Val Pro Leu Val Asp Arg Ala Thr Cys Leu
                   325                 330                 335
   Arg Ser Thr Lys Phe Thr Ile Tyr Asn Asn Met Phe Cys Ala Gly Phe
               340                 345                 350
   His Glu Gly Gly Arg Asp Ser Cys Gln Gly Asp Ser Gly Gly Pro His
           355                 360                 365
   Val Thr Glu Val Glu Gly Thr Ser Phe Leu Thr Gly Ile Ile Ser Trp
       370                 375                 380
   Gly Glu Glu Cys Ala Met Lys Gly Lys Tyr Gly Ile Tyr Thr Lys Val
   385                 390                 395                 400
   Ser Arg Tyr Val Asn Trp Ile Lys Glu Lys Thr Lys Leu Thr
                   405                 410
<210> 9
   <211> 406
   <212> PRT
   <213> Homo sapiens
<220>
   <221> misc_feature
   <222> (7)..(7)
   <223> Xaa is 4-carboxyglutamic acid
<220>
   <221> misc_feature
   <222> (14)..(14)
   <223> Xaa is 4-carboxyglutamic acid
<220>
   <221> misc_feature
   <222> (16)..(16)
   <223> Xaa is 4-carboxyglutamic acid
<220>
   <221> misc_feature
   <222> (19)..(20)
   <223> Xaa is 4-carboxyglutamic acid
<220>
   <221> misc_feature
   <222> (25)..(26)
   <223> Xaa is 4-carboxyglutamic acid
<220>
   <221> misc_feature
   <222> (29)..(29)
   <223> Xaa is 4-carboxyglutamic acid
<220>
   <221> misc_feature
   <222> (35)..(35)
   <223> Xaa is 4-carboxyglutamic acid
<400> 9
   Ala Asn Ala Phe Leu Gly Xaa Leu Arg Pro Gly Ser Leu Xaa Arg Xaa
   1               5                   10                  15
   Cys Lys Xaa Xaa Gln Cys Ser Phe Xaa Xaa Ala Arg Xaa Ile Phe Lys
               20                  25                  30
   Asp Ala Xaa Arg Thr Lys Leu Phe Trp Ile Ser Tyr Ser Asp Gly Asp
           35                  40                  45
   Gln Cys Ala Ser Ser Pro Cys Gln Asn Gly Gly Ser Cys Lys Asp Gln
       50                  55                  60
   Leu Gln Ser Tyr Ile Cys Phe Cys Leu Pro Ala Phe Glu Gly Arg Asn
   65                  70                  75                  80
   Cys Glu Thr His Lys Asp Asp Gln Leu Ile Cys Val Asn Glu Asn Gly
                   85                  90                  95
   Gly Cys Glu Gln Tyr Cys Ser Asp His Thr Gly Thr Lys Arg Ser Cys
               100                 105                 110
   Arg Cys His Glu Gly Tyr Ser Leu Leu Ala Asp Gly Val Ser Cys Thr
           115                 120                 125
   Pro Thr Val Glu Tyr Pro Cys Gly Lys Ile Pro Ile Leu Glu Lys Arg
       130                 135                 140
   Asn Ala Ser Lys Pro Gln Gly Arg Ile Val Gly Gly Lys Val Cys Pro
   145                 150                 155                 160
   Lys Gly Glu Cys Pro Trp Gln Val Leu Leu Leu Val Asn Gly Ala Gln
                   165                 170                 175
   Leu Cys Gly Gly Thr Leu Ile Asn Thr Ile Trp Val Val Ser Ala Ala
               180                 185                 190
   His Cys Phe Asp Lys Ile Lys Asn Trp Arg Asn Leu Ile Ala Val Leu
           195                 200                 205
   Gly Glu His Asp Leu Ser Glu His Asp Gly Asp Glu Gln Ser Arg Arg
       210                 215                 220
   Val Ala Gln Val Ile Ile Pro Ser Thr Tyr Val Pro Gly Thr Thr Asn
   225                 230                 235                 240
   His Asp Ile Ala Leu Leu Arg Leu His Gln Pro Val Val Leu Thr Asp
                   245                 250                 255
   His Val Val Pro Leu Cys Leu Pro Glu Arg Thr Phe Ser Glu Arg Thr
               260                 265                 270
   Leu Ala Phe Val Arg Phe Ser Leu Val Ser Gly Trp Gly Gln Leu Leu
           275                 280                 285
   Asp Arg Gly Ala Thr Ala Leu Glu Leu Met Val Leu Asn Val Pro Arg
       290                 295                 300
   Leu Met Thr Gln Asp Cys Leu Gln Gln Ser Arg Lys Val Gly Asp Ser
   305                 310                 315                 320
   Pro Asn Ile Thr Glu Tyr Met Phe Cys Ala Gly Tyr Ser Asp Gly Ser
                   325                 330                 335
   Lys Asp Ser Cys Lys Gly Asp Ser Gly Gly Pro His Ala Thr His Tyr
               340                 345                 350
   Arg Gly Thr Trp Tyr Leu Thr Gly Ile Val Ser Trp Gly Gln Gly Cys
           355                 360                 365
   Ala Thr Val Gly His Phe Gly Val Tyr Thr Arg Val Ser Gln Tyr Ile
       370                 375                 380
   Glu Trp Leu Gln Lys Leu Met Arg Ser Glu Pro Arg Pro Gly Val Leu
   385                 390                 395                 400
   Leu Arg Ala Pro Phe Pro
   405
<210> 10
   <211> 488
   <212> PRT
   <213> Homo sapiens
<400> 10
<210> 11
   <211> 33
   <212> DNA
   <213> Artificial sequence
<220>
   <223> primer
<400> 11
   gcagcacata tgggacagtt catagtgata ggg 33
<210> 12
   <211> 34
   <212> DNA
   <213> Artificial sequence
<220>
   <223> primer
<400> 12
   gcagacctcg aggtagaagg gatcttctac tttc 34
<210> 13
   <211> 33
   <212> DNA
   <213> Artificial sequence
<220>
   <223> primer
<400> 13
   gatgtccgtc ctttgtgctc aaggagatta cca 33
<210> 14
   <211> 34
   <212> DNA
   <213> Artificial sequence
<220>
   <223> primer
<400> 14
   ttgtattcaa ggagatgccc aaaaggtgta aaac 34
<210> 15
   <211> 34
   <212> DNA
   <213> Artificial sequence
<220>
   <223> primer
<400> 15
   ttaccaaaag gtgtatgcca tttgaaggat tttc 34
<210> 16
   <211> 33
   <212> DNA
   <213> Artificial sequence
<220>
   <223> primer
<400> 16
   aaggattttc caatttgccc aggagaaata ttc 33
<210> 17
   <211> 34
   <212> DNA
   <213> Artificial sequence
<220>
   <223> primer
<400> 17
   gattatattt aagaattgcg caagcagacc atat 34
<210> 18
   <211> 36
   <212> DNA
   <213> Artificial sequence
<220>
   <223> primer
<400> 18
   tagaaaaaac tttgtctgcc ctaatgaaac caaaac 36
<210> 19
   <211> 33
   <212> DNA
   <213> Artificial sequence
<220>
   <223> primer
<400> 19
   aactttgtca agccttgcga aaccaaaact tac 33
<210> 20
   <211> 34
   <212> DNA
   <213> Artificial sequence
<220>
   <223> Artificial sequence
<400> 20
   gtcaagccta atgaatgcaa aacttacttt tgga 34
<210> 21
   <211> 35
   <212> DNA
   <213> Artificial sequence
<220>
   <223> primer
<400> 21
   caagcctaat gaaacctgca cttacttttg gaaag 35
<210> 22
   <211> 36
   <212> DNA
   <213> Artificial sequence
<220>
   <223> primer
<400> 22
   ctaatgaaac caaaacttgc ttttggaaag tgcaac 36
<210> 23
   <211> 33
   <212> DNA
   <213> Artificial sequence
<220>
   <223> primer
<400> 23
   atttcttatg aggaatgcca gaggcaagga gca 33
<210> 24
   <211> 33
   <212> DNA
   <213> Artificial sequence
<220>
   <223> primer
<400> 24
   tcttatgagg aagattgcag gcaaggagca gaa 33
<210> 25
   <211> 36
   <212> DNA
   <213> Artificial sequence
<220>
   <223> primer
<400> 25
   caaggagcag aaccttgcaa aaactttgtc aagcct 36
<210> 26
   <211> 33
   <212> DNA
   <213> Artificial sequence
<220>
   <223> primer
<400> 26
   ggagcagaac ctagatgcaa ctttgtcaag cct 33
<210> 27
   <211> 33
   <212> DNA
   <213> Artificial sequence
<220>
   <223> primer
<400> 27
   cgctcagttg ccaagtgtca tcctaaaact tgg 33
<210> 28
   <211> 33
   <212> DNA
   <213> Artificial sequence
<220>
   <223> primer
<400> 28
   tcagttgcca agaagtgtcc taaaacttgg gta 33
<210> 29
   <211> 33
   <212> DNA
   <213> Artificial sequence
<220>
   <223> primer
<400> 29
   ctcctcatct gctactgcga atctgtagat caa 33
<210> 30
   <211> 35
   <212> DNA
   <213> Artificial sequence
<220>
   <223> primer
<400> 30
   caaaatcttt tccattctgc acctcagtcg tgtac 35
<210> 31
   <211> 33
   <212> DNA
   <213> Artificial sequence
<220>
   <223> primer
<400> 31
   gtcaatggtt atgtatgcag gtctctgcca ggt 33
<210> 32
   <211> 33
   <212> DNA
   <213> Artificial sequence
<220>
   <223> primer
<400> 32
   cagacttatc gaggatgttc cactggaacc tta 33
<210> 33
   <211> 33
   <212> DNA
   <213> Artificial sequence
<220>
   <223> primer
<400> 33
   atccaggctg aggtttgtga tacagtggtc att 33
<210> 34
   <211> 33
   <212> DNA
   <213> Artificial sequence
<220>
   <223> primer
<400> 34
   gaagatgata aagtctgtcc tggtggaagc cat 33
<210> 35
   <211> 33
   <212> DNA
   <213> Artificial sequence
<220>
   <223> primer
<400> 35
   cagcggattg gtaggtgtta caaaaaagtc cga 33
<210> 36
   <211> 33
   <212> DNA
   <213> Artificial sequence
<220>
   <223> primer
<400> 36
   gaagatgggc caacttgctc agatcctcgg tgc 33
<210> 37
   <211> 33
   <212> DNA
   <213> Artificial sequence
<220>
   <223> primer
<400> 37
   cagataatgt cagactgcag gaatgtcatc ctg 33
<210> 38
   <211> 33
   <212> DNA
   <213> Artificial sequence
<220>
   <223> primer
<400> 38
   cacactaaca cactgtgtcc tgctcatggg aga 33
<210> 39
   <211> 33
   <212> DNA
   <213> Artificial sequence
<220>
   <223> primer
<400> 39
   cagatggaag atccctgctt taaagagaat tat 33
<210> 40
   <211> 33
   <212> DNA
   <213> Artificial sequence
<220>
   <223> primer
<400> 40
   acccagggtg cccgttgcaa gttctccagc ctc 33
<210> 41
   <211> 33
   <212> DNA
   <213> Artificial sequence
<220>
   <223> primer
<400> 41
   aaagtaaagg ttttttgcgg aaatcaagac tcc 33
<210> 42
   <211> 33
   <212> DNA
   <213> Artificial sequence
<220>
   <223> primer
<400> 42
   ttgcagttgt cagttgcttt gcatgaggtg gca 33
<210> 43
   <211> 33
   <212> DNA
   <213> Artificial sequence
<220>
   <223> primer
<400> 43
   aatatggaaa gaaacgctag ggctccctgc aat 33